# EUROPEAN PATENT APPLICATION

(11) **EP 3 000 443 A2**
(43) Date of publication of application: **30.03.2016**
(21) Application number: 15189568.7
(22) Date of filing: 26.08.2010
(51) Int. Cl.: A61F 2/38

(54) **PATIENT-SPECIFIC ORTHOPEDIC IMPLANTS**

(30) Priority: 26.08.2009 US 275174 P; 04.11.2009 US 280493 P; 18.12.2009 US 284458 P; 25.02.2010 WO PCT/US2010/025459; 09.03.2010 US 339766 P; 23.06.2010 WO PCT/US2010/039587
(62) Divisional of application: 10752227.8
(71) Applicant: ConforMIS, Inc., Bedford, MA 01730 (US)
(72) Inventor: SLAMIN, John, Wrentham, MA Massachusetts 02093 (US); LANG, Philipp, Lexington, MA Massachusetts 02421 (US); FITZ, Wolfgang, Sherborn, MA Massachusetts 01770 (US); STEINES, Daniel, Lexington, MA Massachusetts 02421 (US); MINAS, Thomas, Dover, MA Massachusetts 02030 (US)
(74) Representative: Grund, Martin

(57) **Abstract**

A patient-specific knee implant 10 includes a femoral component 20 and a tibial tray component 30, and it is designed based on patient-specific data. An inner, bone-facing surface 40 of the femoral component 20 conforms to the corresponding surface of the femoral condyle. Alternatively, it can conform to one or more optimized bone cuts on the femoral condyle. However, the outer, articular surface 50 of the component 20 is enhanced to incorporate a smooth surface having a nearly constant radius in the coronal plane. The corresponding articular surface 70 of the tibial tray 30 has a surface contour in the coronal plane that is matched to the outer articular surface 50. In certain embodiments, the articular surface 50 of the component 20 incorporates a sagittal curvature that positively-matches the patient's existing or healthy sagittal radius.

## Description

### CROSS REFERENCE TO RELATED APPLICATIONS

This application claims the benefit of: U.S. Ser. No. 61/275,174, filed August 26, 2009, entitled "Patient Specific Orthopedic Implants and Models;" U.S. Ser. No. 61/280,493, filed November 4, 2009, entitled "Patient Adapted and Improved Orthopedic Implants, Designs and Related Tools;" U.S. Ser. No. 61/284,458, filed December 18, 2009, entitled "Patient Adapted and Improved Orthopedic Implants, Designs and Related Tools;" U.S. Ser. No. 61/339,766, filed March 9, 2010, entitled "Patient Adapted and Improved Orthopedic Implants, Designs and Related Tools;" PCT/US2010/025459, filed February 25, 2010, entitled "Patient-Adapted And Improved Orthopedic Implants, Designs And Related Tools;" and PCT/US2010/039587, filed June 23, 2010, entitled "Patient-Adapted And Improved Articular Implants, Designs And Related Guide Tools."

Each of the above-described applications is hereby incorporated by reference in its entirety.

### TECHNICAL FIELD

The invention relates to the creation of patient-specific orthopedic implants and devices, as well as designs for and mathematical models of joints, especially human joints, based on data, such as imaging data, representing an existing joint.

### BACKGROUND

Generally, a diseased, injured or defective joint, such as, for example, a joint exhibiting osteoarthritis, has been repaired using standard off-the-wall implants and other surgical devices. Only recently has the concept of patient-specific implants tailored to an individual patient's joint become available. Such patient-specific implants, such as the iForma®, iUni® and iDuo®, offer advantages over the traditional "several-size-fits-all" approach such as a better fit, more natural movement, reduction in the amount of bone removed during surgery and a less invasive procedure. Such patient-specific implants generally are created from images of the patient's joint. Based on the images, the patient-specific implant can be created both to include surfaces that match existing surfaces in the joint as well as to include surfaces that approximate an ideal and/or healthy surface that does not exist in the patient prior to any procedure.

### SUMMARY

The present invention provides methods and devices to create a desired model of a joint or of portions or surfaces of a joint based on data derived from the existing joint. Data from the existing joint, for example, data generated from an image of the joint such as an MRI or CT scan, is processed to generate a varied or corrected version of the joint or of portions of the joint or of surfaces within the joint. For example, the data can also be used to create a model that can be used to analyze the patient's joint and to devise and evaluate a course of corrective action. The data and/or model also can be used to design an implant component having one or more patient-specific aspects, such as a surface or curvature.

In one aspect, some embodiments provide implant components having an inner, bone-facing surface designed to negatively-match a bone surface. In certain embodiments, an outer joint-facing surface of a first implant component is designed to and/or does, at least in a portion of the component, negatively-match an opposing outer joint-facing surface of a second implant component. By creating negatively-matching component surfaces at a joint interface, the opposing surfaces may not have an anatomic or near-anatomic shape, but instead may be negatively-matching or near-negatively matching to each other. This can have various advantages, such as reducing implant and joint wear and providing more predictable joint movement.

In another aspect, some embodiments provide implant components having one or more patient-specific curvatures or radii in one dimension, and one or more standard or engineered curvatures or radii in a second dimension.

In another aspect, methods of designing, selecting, manufacturing, and implanting the patient-specific implant components are provided.

It is to be understood that the features of the various embodiments described herein are not mutually exclusive and may exist in various combinations and permutations.

### BRIEF DESCRIPTION OF THE DRAWINGS

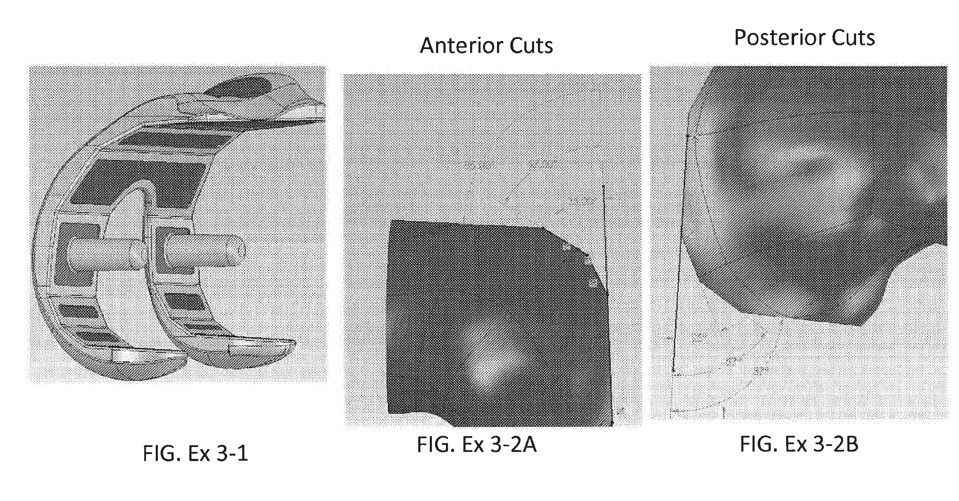

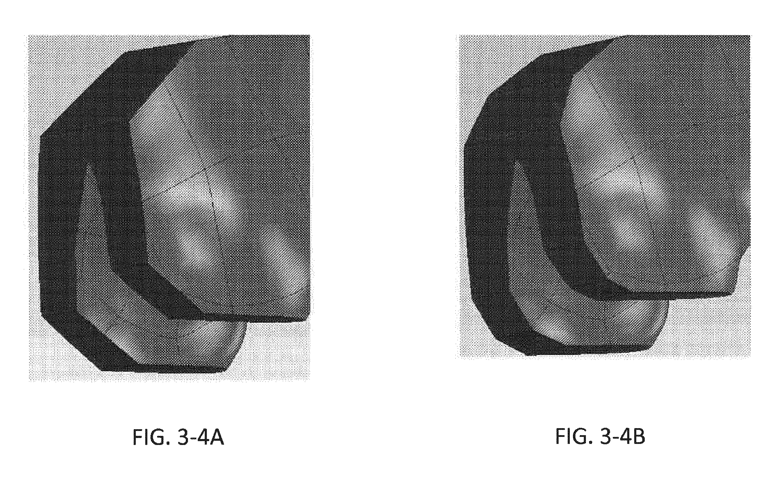

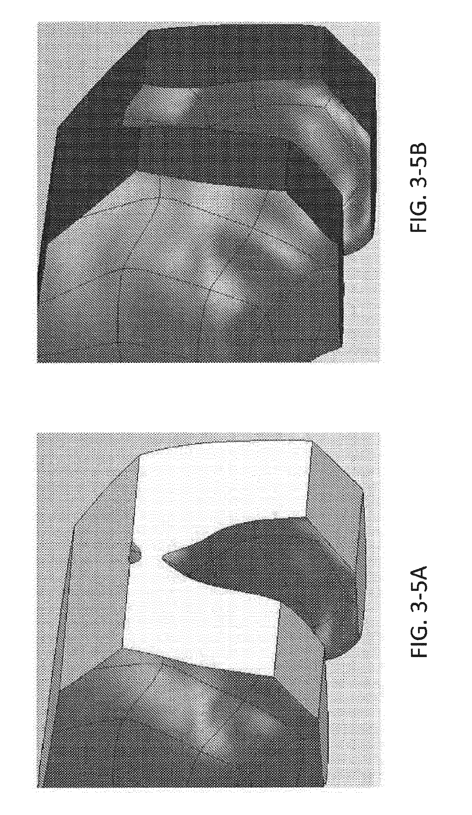

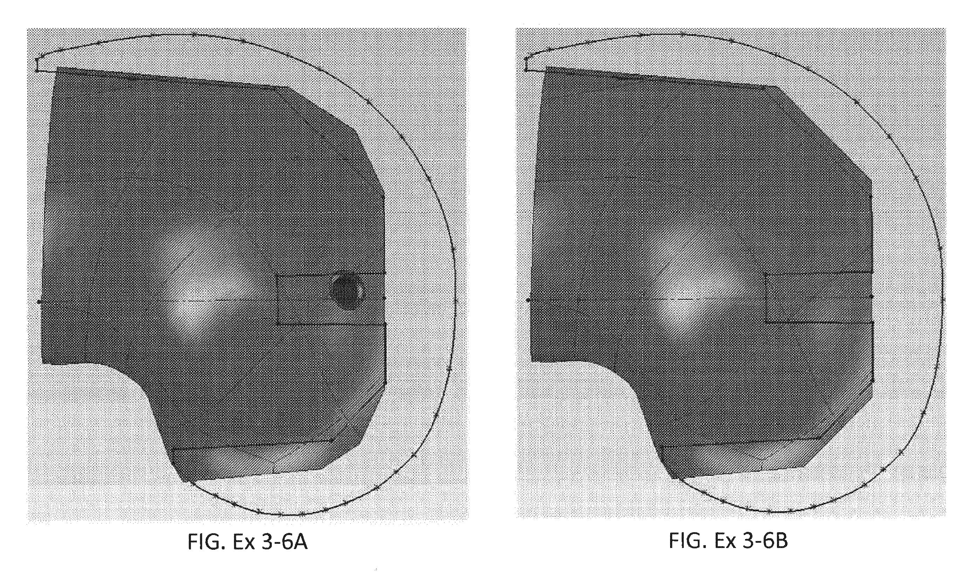

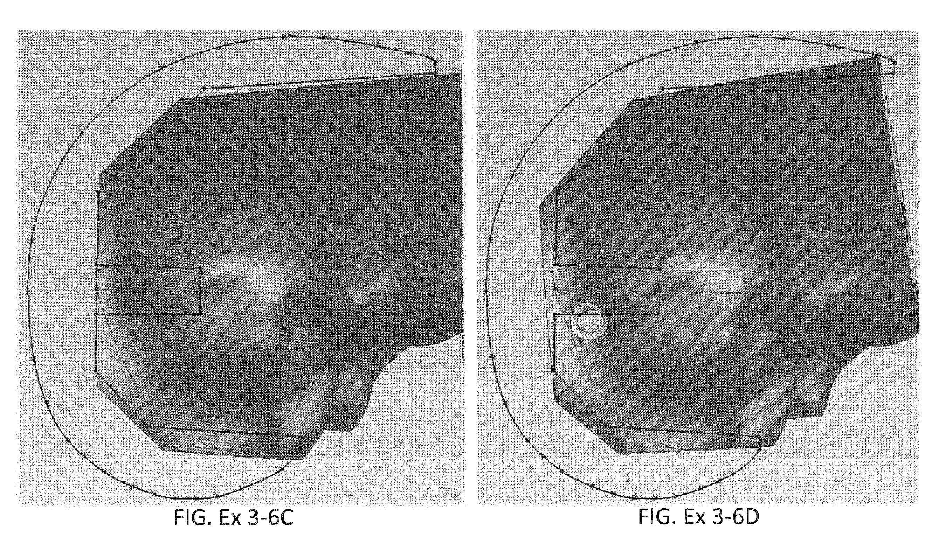

The foregoing and other objects, aspects, features, and advantages of embodiments will become more apparent and may be better understood by referring to the following description, taken in conjunction with the accompanying drawings, in which:
**FIGS. 1A****-1C** each show a schematic diagram of an exemplary embodiment;
**FIGS. 2A- 2C** depict designs of implant components that have six bone cuts **(****FIG. 2A****),** seven bone cuts **(****FIG. 2B****),** and three bone cuts with one being a curvilinear bone cut **(****FIG. 2C****);**
**FIG. 3A** is a photograph showing an exemplary knee replacement using a patient-specific bicompartmental device and a patient-specific unicompartmental device; **FIGS. 3B** and **3C** are x-ray images showing the device of **FIG. 3A** in the coronal plane and in the sagittal plane, respectively;
**FIGS. 4A****-4E** show an exemplary design of a two-piece implant component;
**FIG. 5A** is a drawing of a cross-sectional view of an end of a femur with an osteophyte; **FIG. 5B** is a drawing of the end of the femur of **FIG. 5A** with the osteophyte virtually removed; **FIG. 5C** is a drawing of the end of the femur of **FIG. 5B** with the osteophyte virtually removed and showing a cross-sectional view of an implant designed to the shape of the femur with the osteophyte removed; **FIG.5D** is a drawing of the end of the femur of **FIG. 5A** and shows a cross-sectional view of an implant designed to the shape of the femur with the osteophyte intact;
**FIG. 6A** is a drawing of a cross-sectional view of an end of a femur with a subchondral void in the bone; **FIG. 6B** is a drawing of the end of the femur of **FIG. 6A** with the void virtually removed; **FIG. 6C** is a drawing of the end of the femur of **FIG. 6B** with the void virtually removed and showing a cross-sectional view of an implant designed to the shape of the femur with the void removed; **FIG. 6D** is a drawing of the end of the femur of **FIG. 6A** and showing a cross-sectional view of an implant designed to the shape of the femur with the void intact;

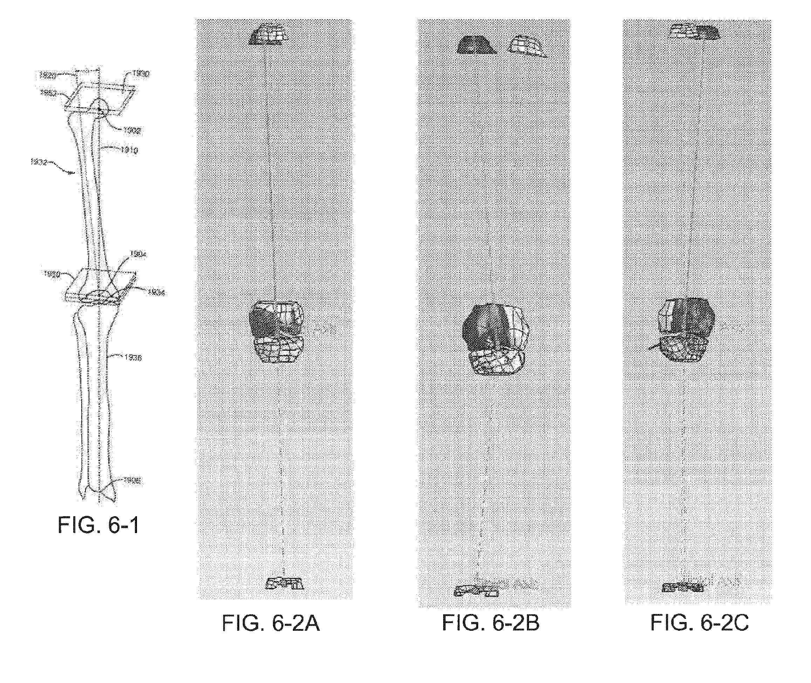

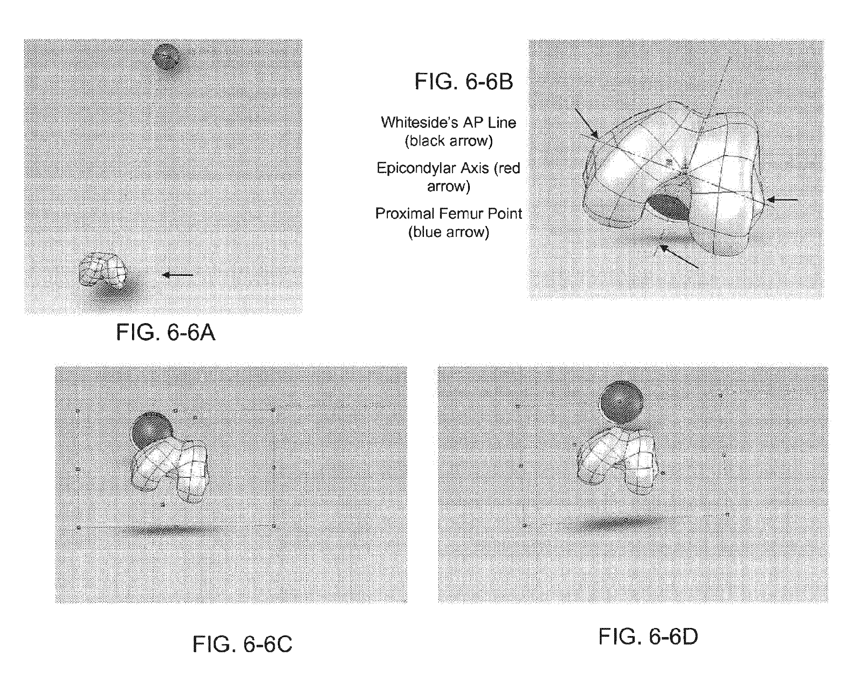

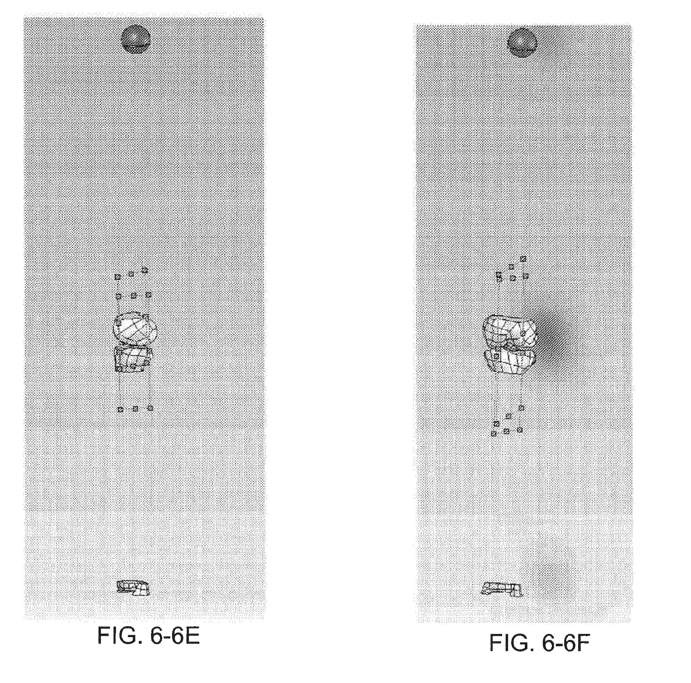

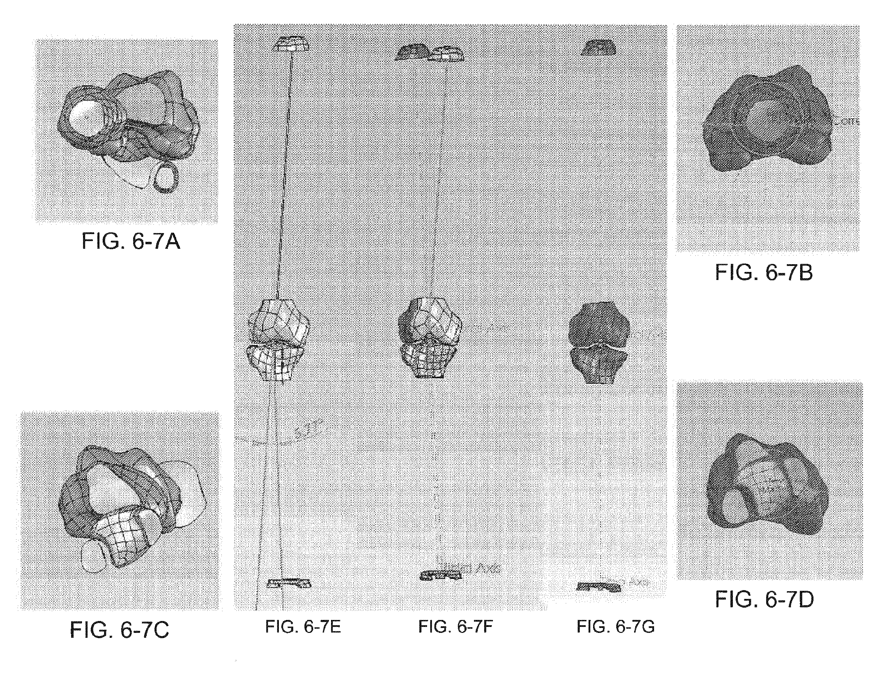

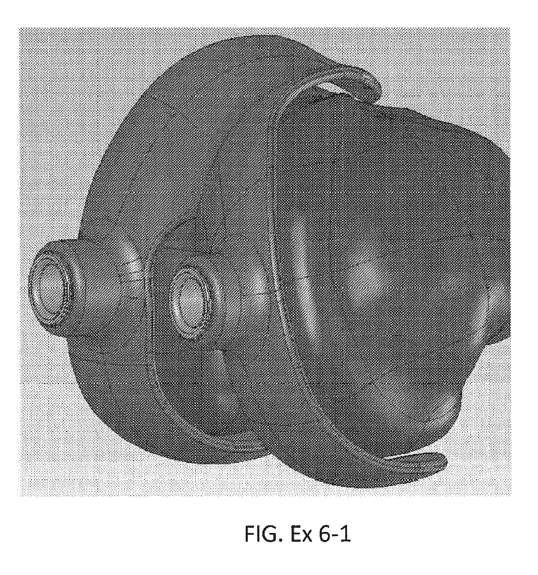

**FIGS. 6-1 - 6-7G** illustrate steps in a virtual limb alignment;

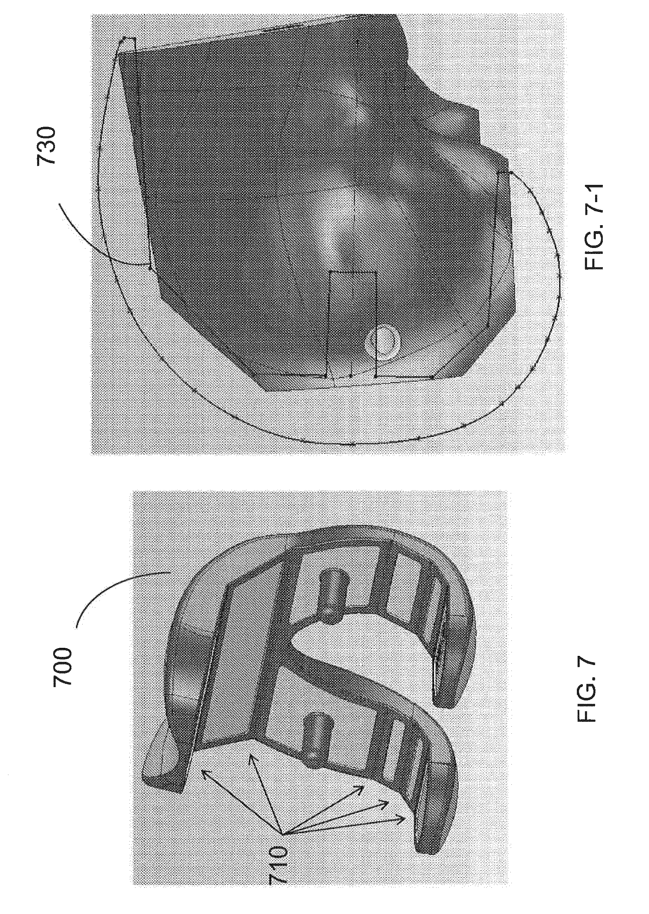

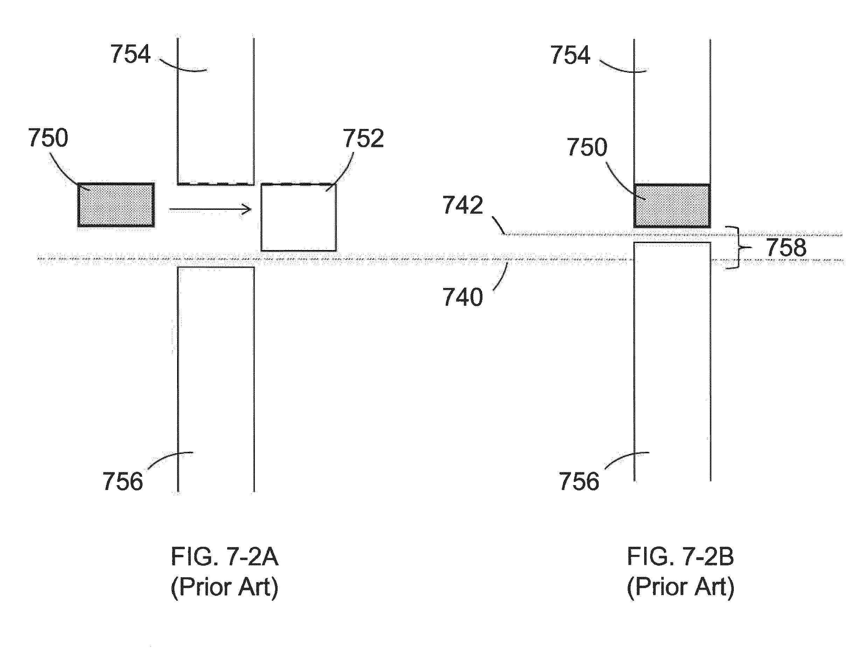

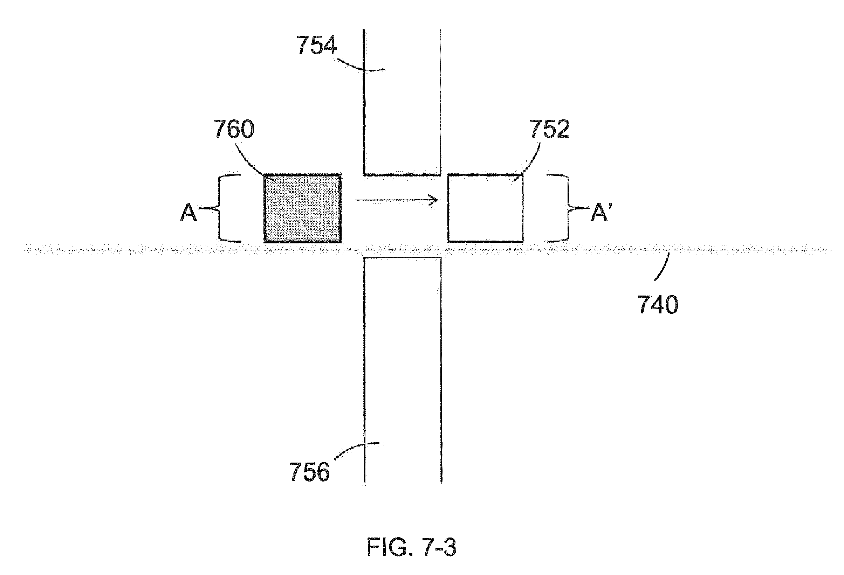

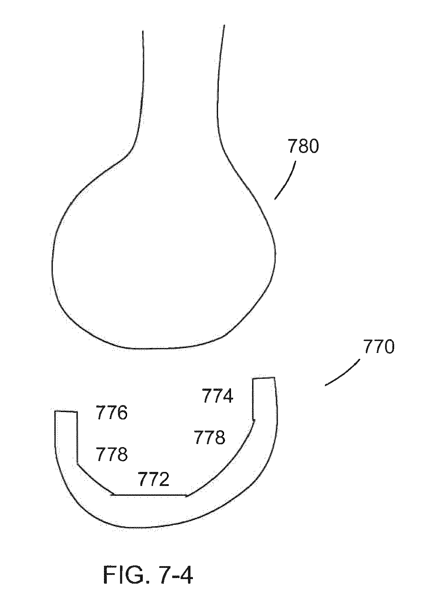

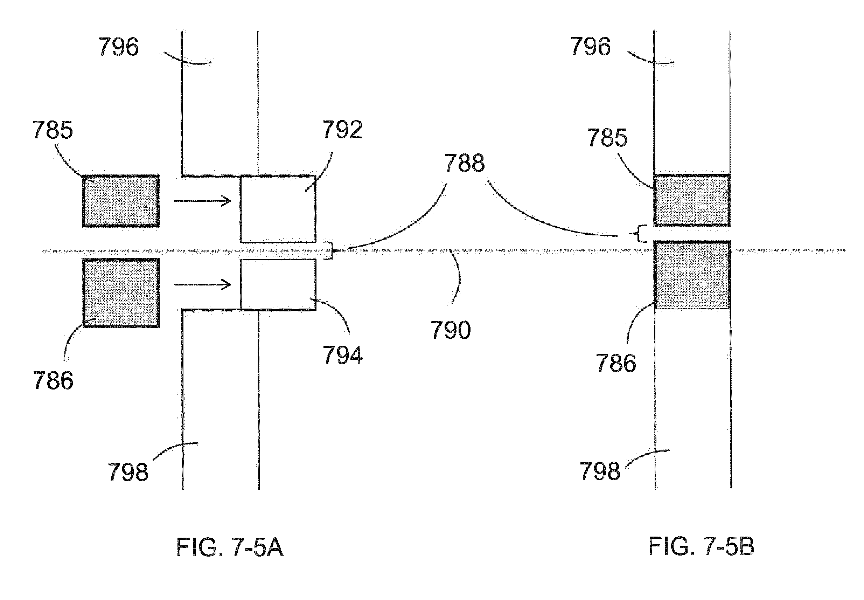

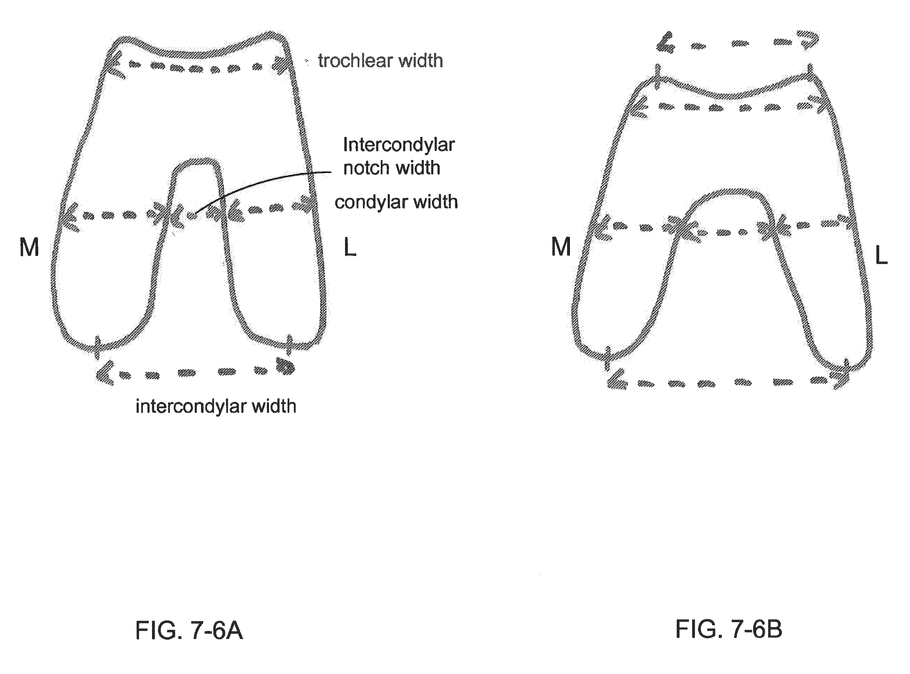

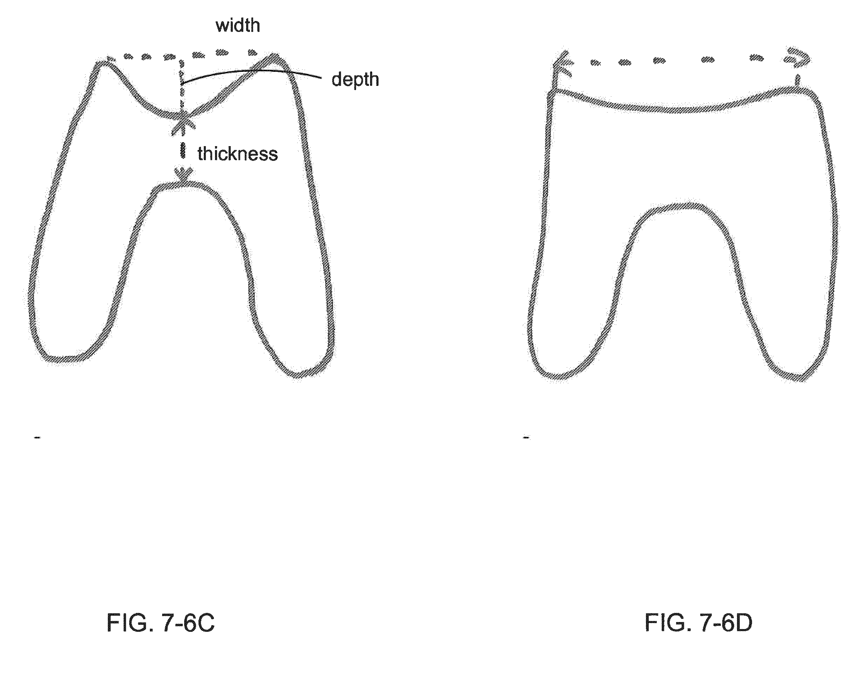

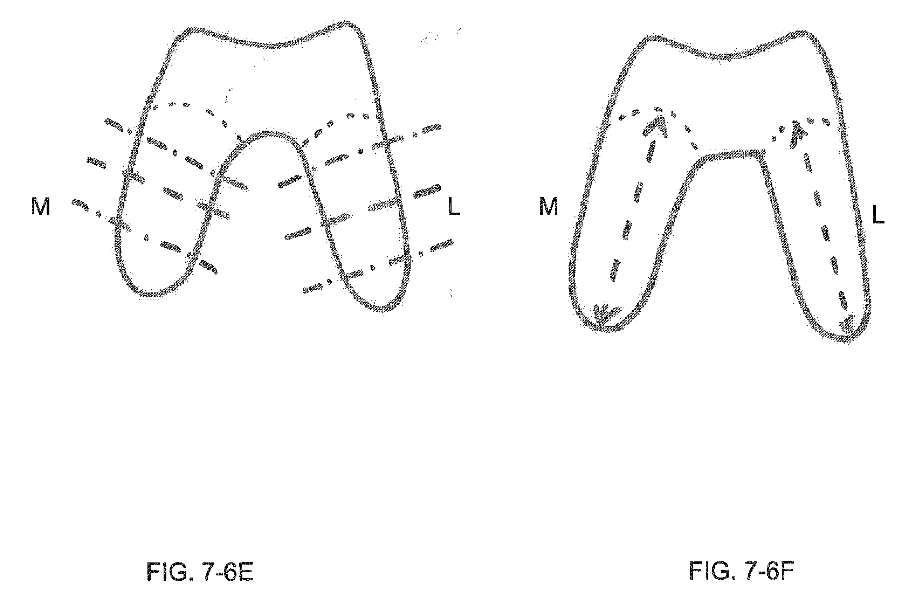

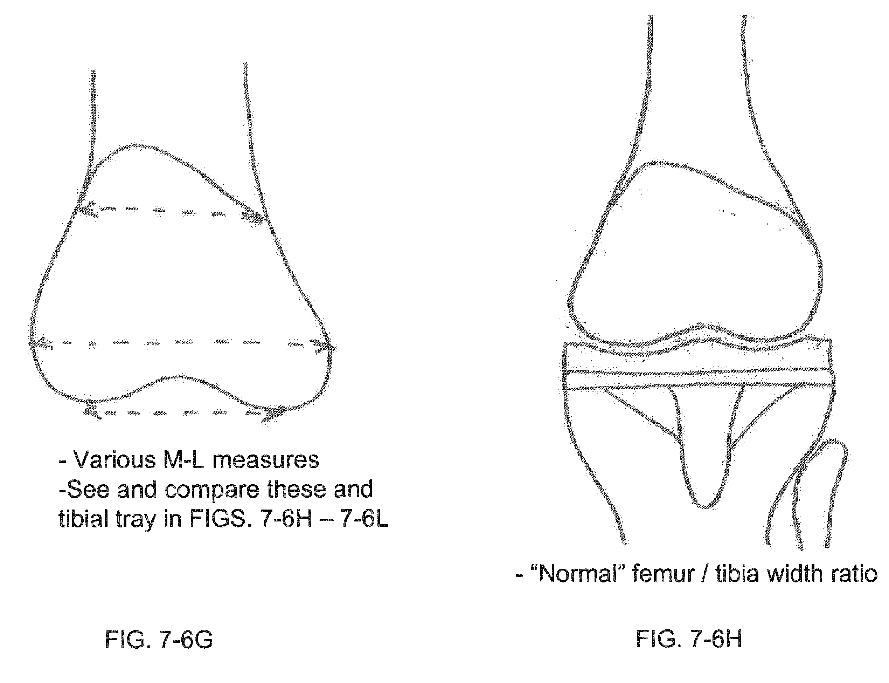

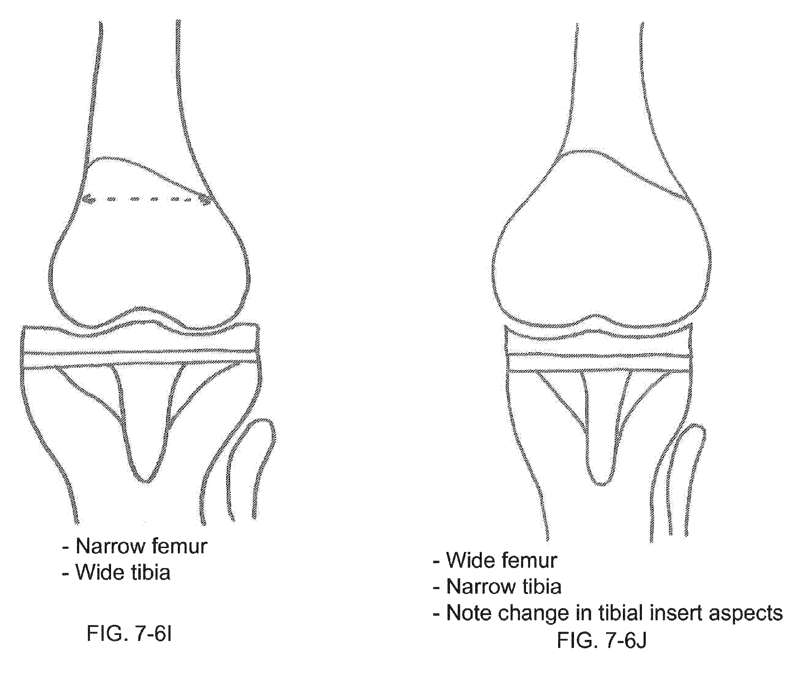

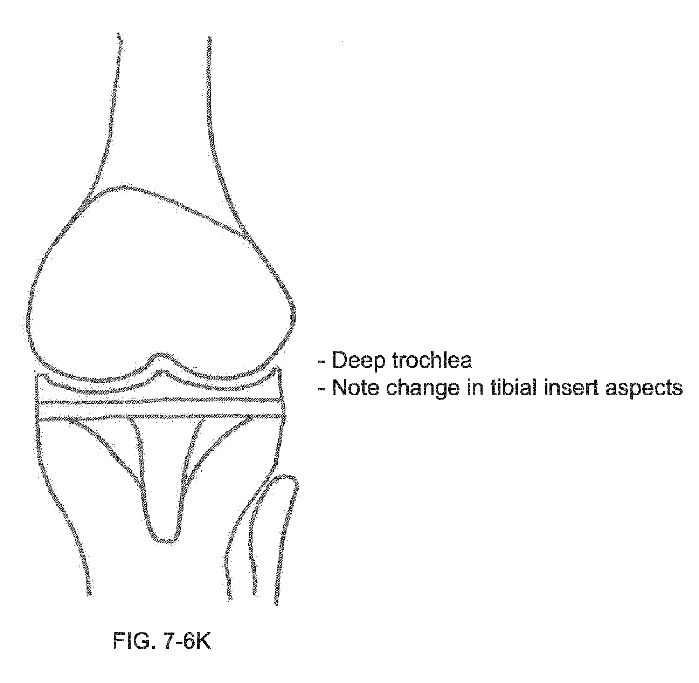

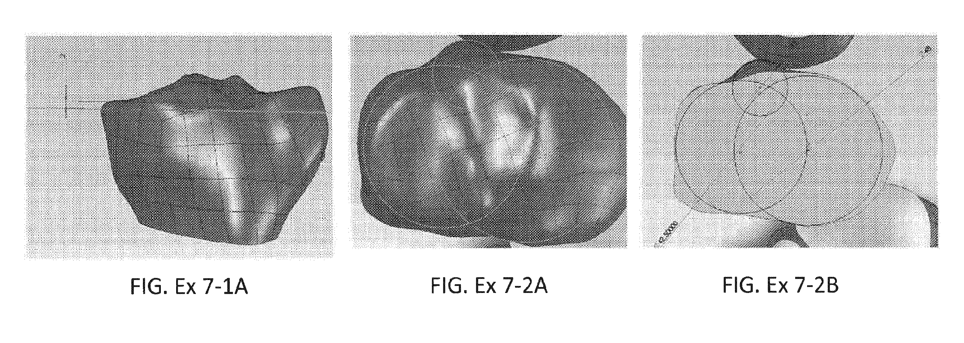

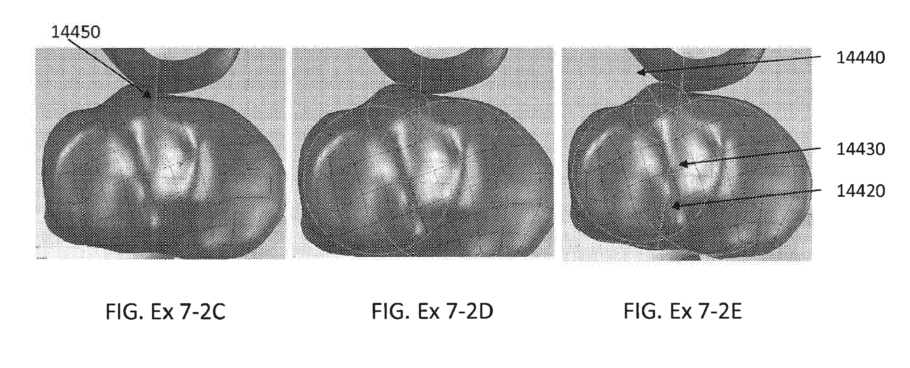

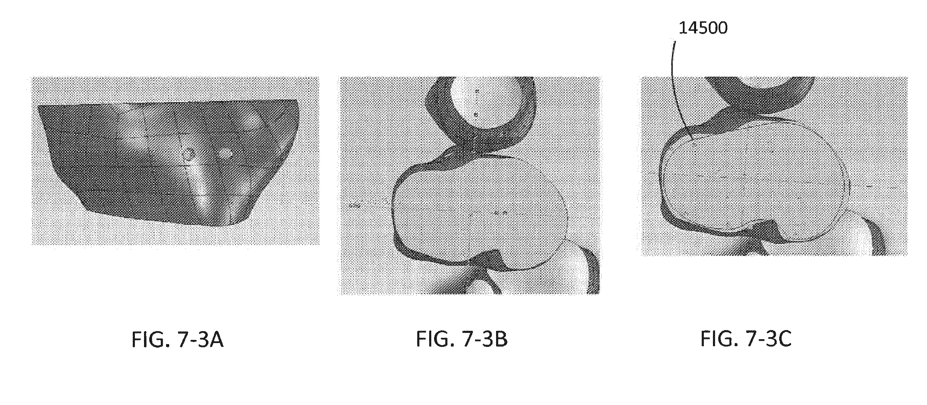

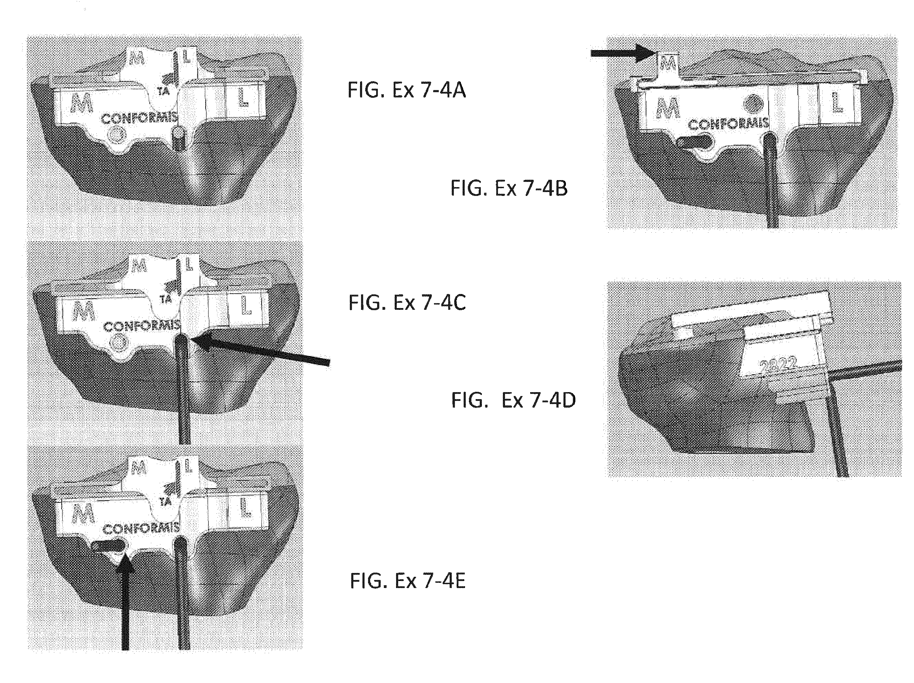

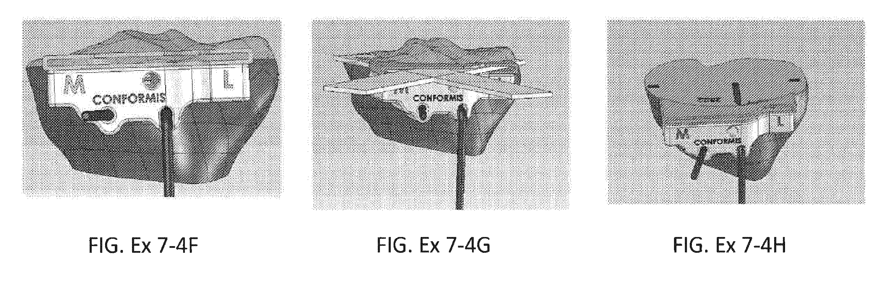

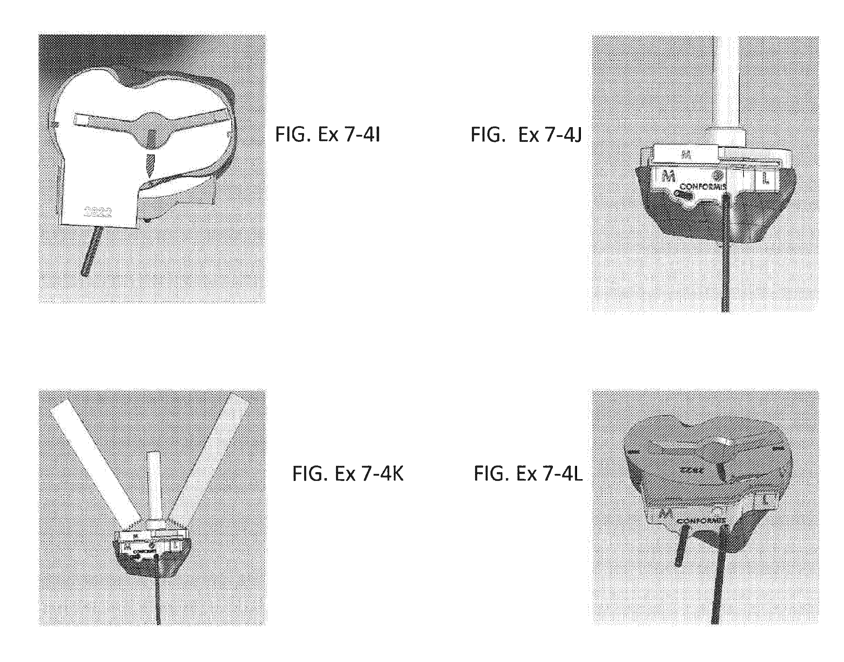

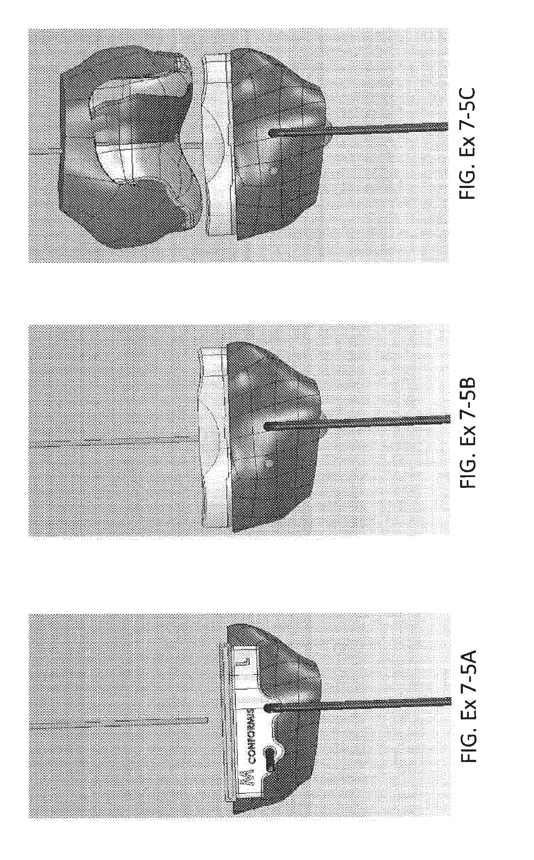

**FIG. 7** is an exemplary implant component showing the intersect of bone cuts on the inner, bone-facing surface of the implant;
**FIG. 7-1** illustrates a computer model of a distal femur having optimized bone cuts for a pre-primary implant overlaid with a traditional primary implant (shown in outline);
**FIGS. 7-2A** and **7-2B** schematically show a traditional implant component that dislocates the joint-line;
**FIG. 7-3** schematically shows a patient-specific implant component in which the existing or natural joint-line is retained;
**FIG. 7-4** depicts an implant or implant design that includes a straight distal cut, a straight anterior cut, a straight posterior cut, and curvilinear chamfer cuts;
**FIGS. 7-5A** and **7-5B** schematically show a patient-specific implant component designed to substantially positively-match the patient's existing or natural joint gap;
**FIGS. 7-6A - 7-6K** show implant components with exemplary aspects that can be included in a library.

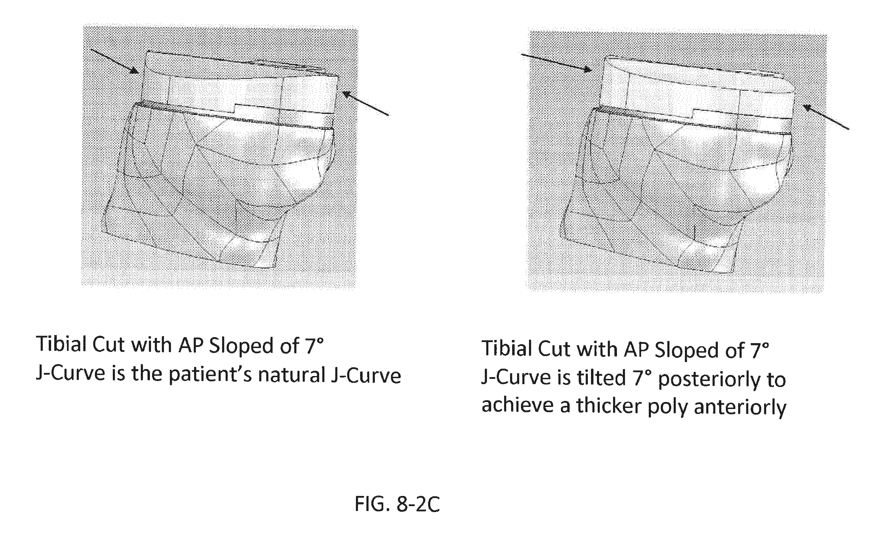

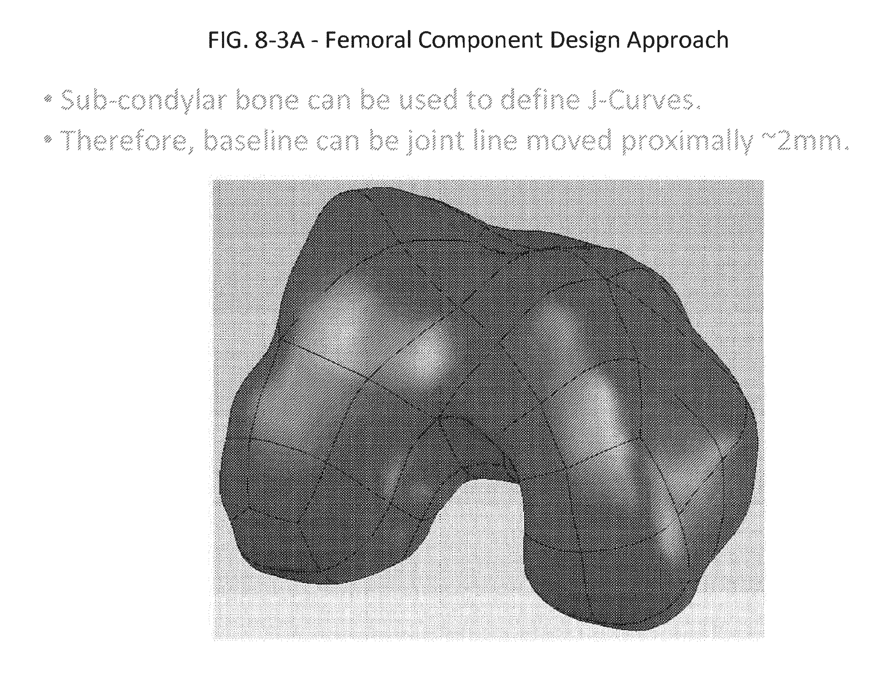

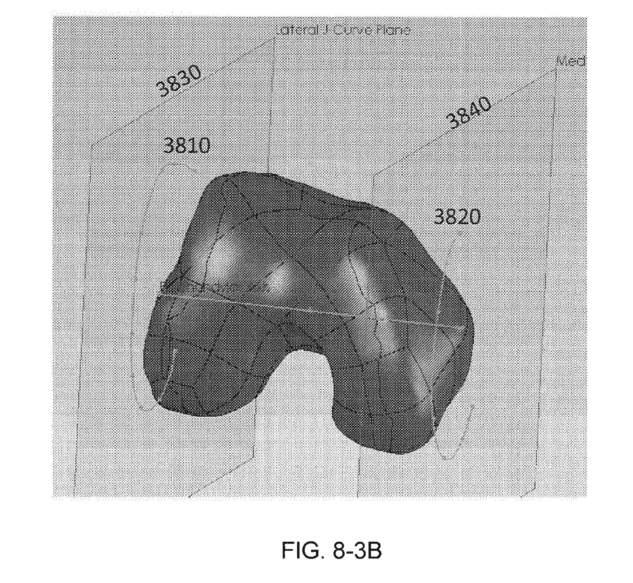

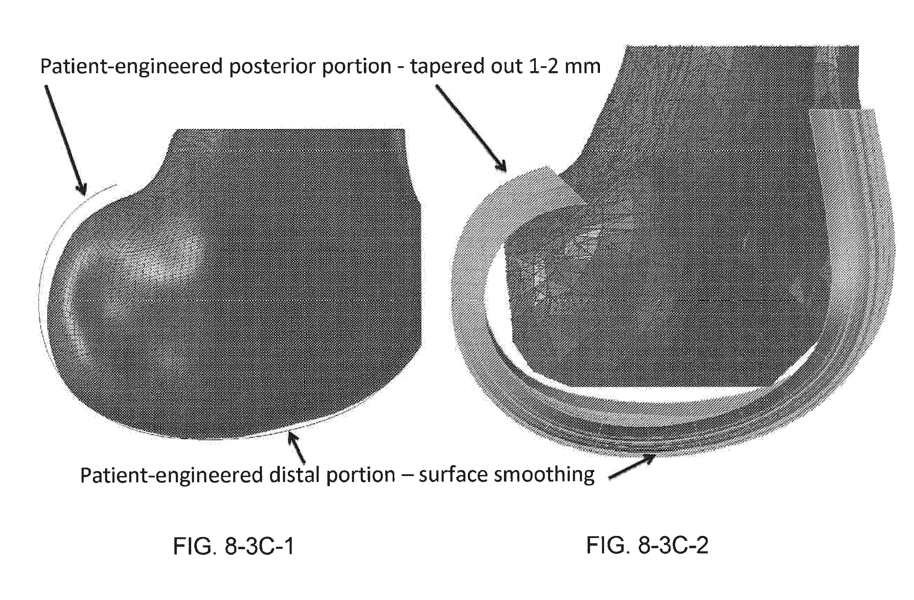

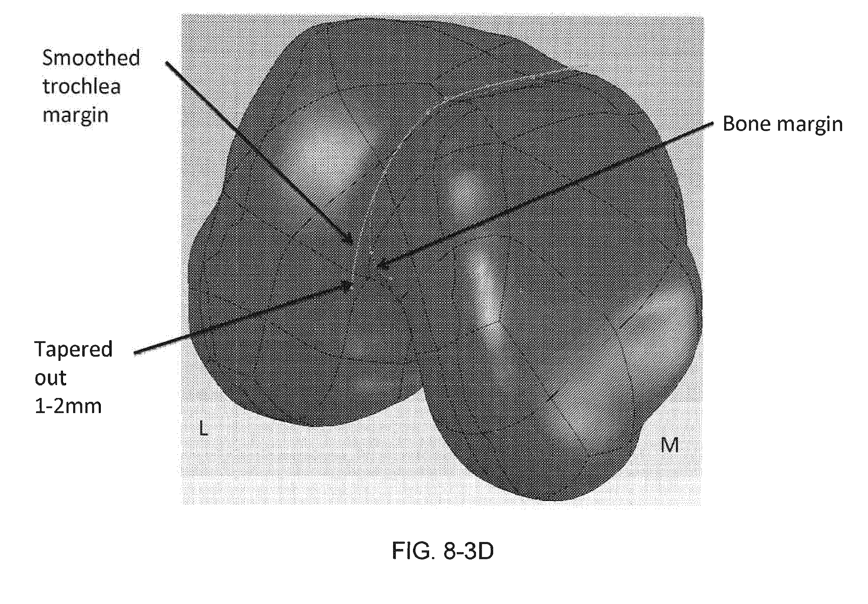

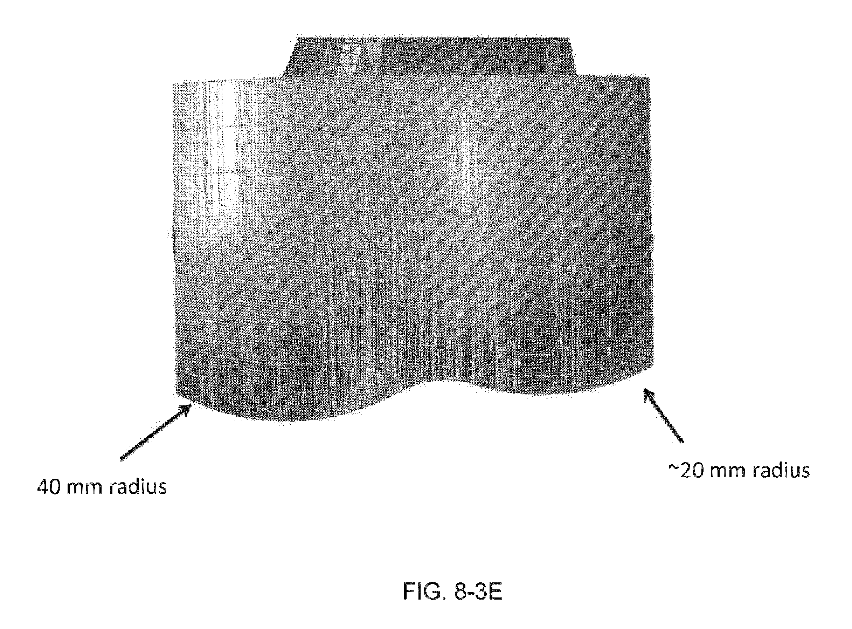

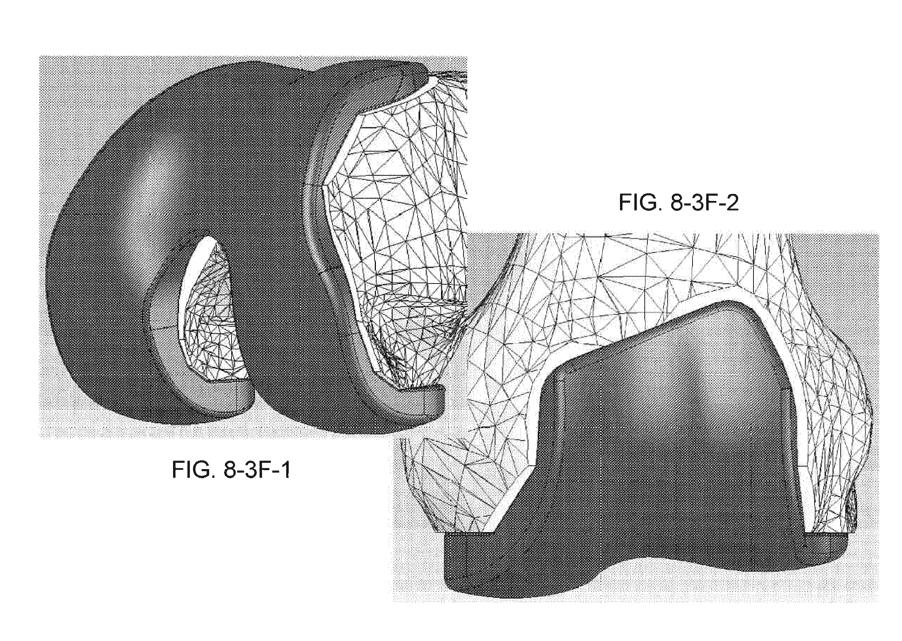

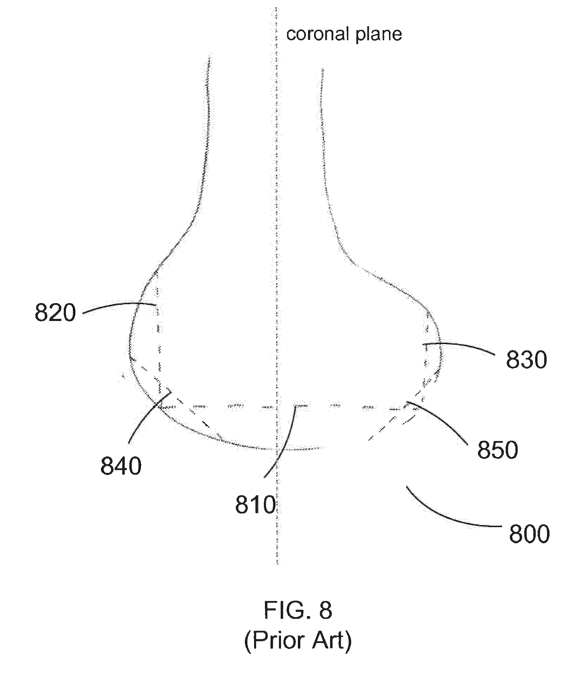

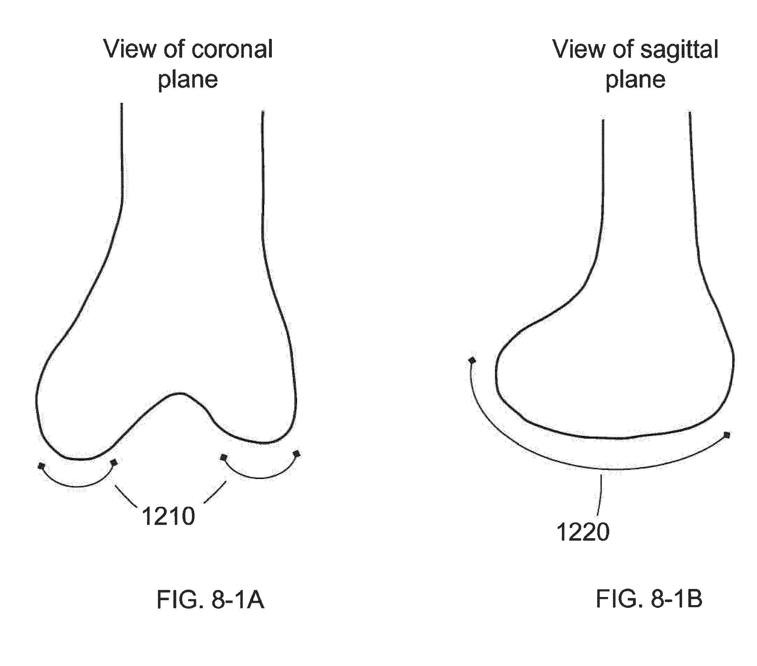

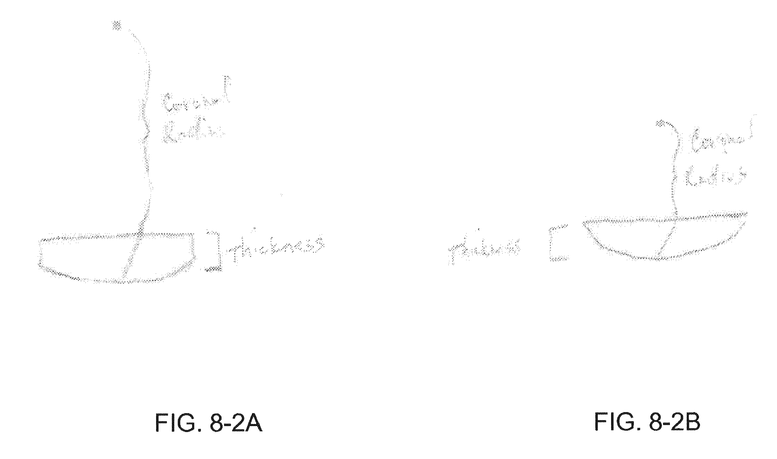

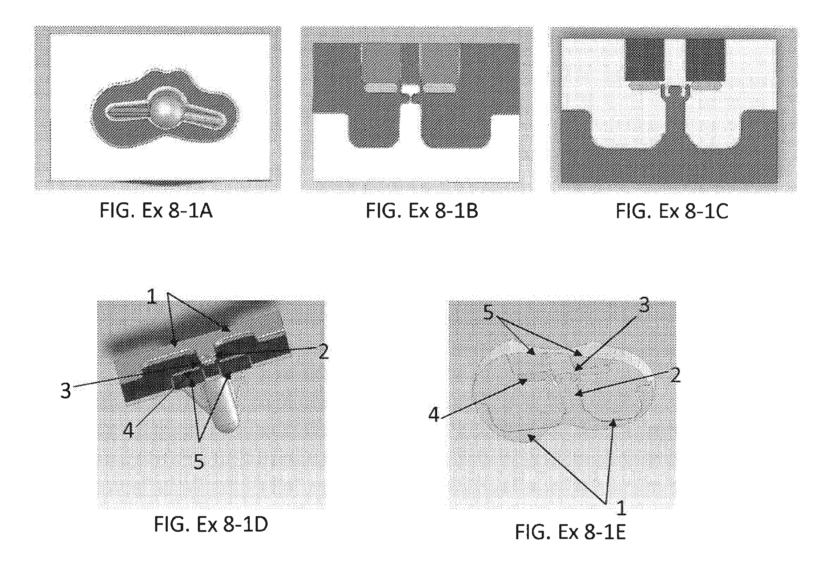

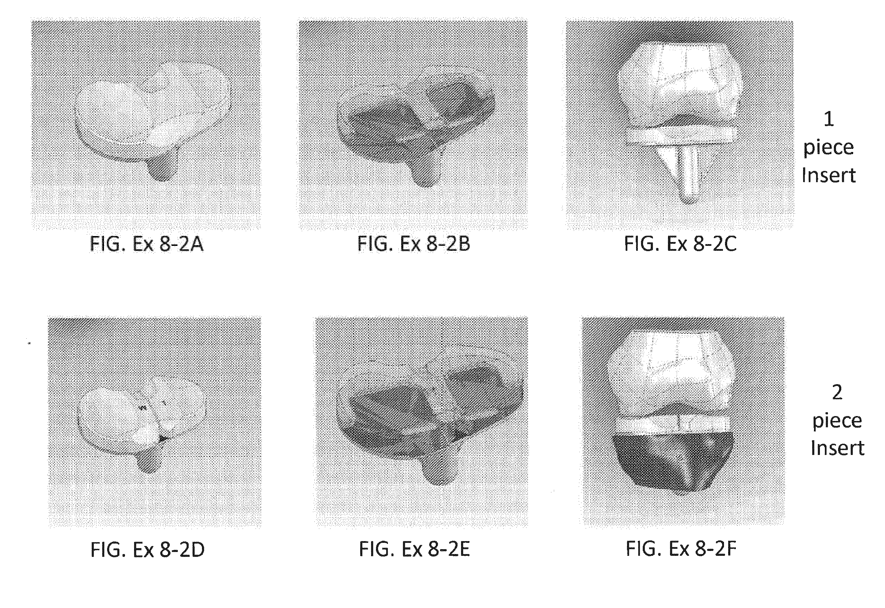

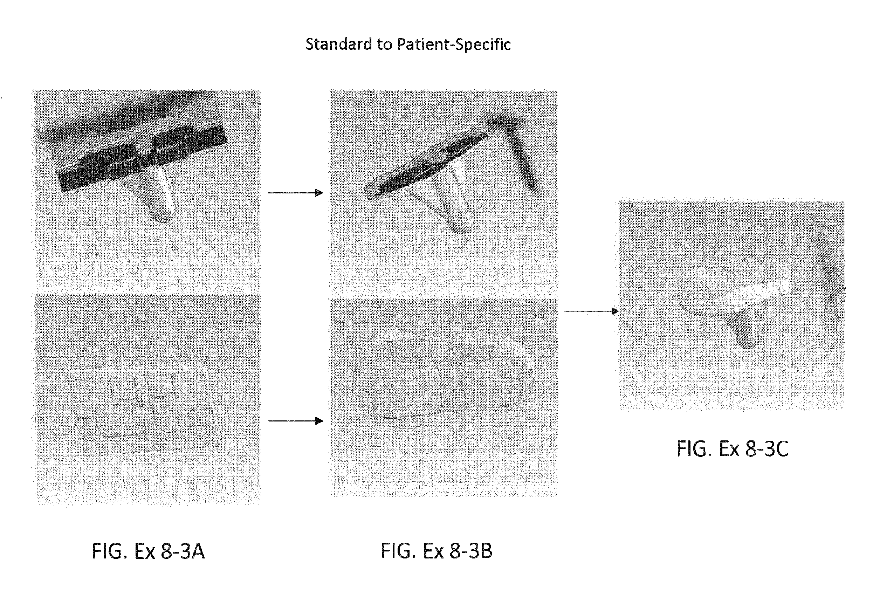

**FIG. 8** shows a coronal view of a patient's femoral bone and, in dashed lines, standard bone cuts performed with a traditional total knee implant;
**FIGS. 8-1A** and **8-1B** show the load bearing surfaces of a femoral implant component in a coronal view **(****FIG. 8-1A**) and in a sagittal view **(****FIG. 8-1B****);**
**FIGS. 8-2A** and **8-2B** show cross-sections from a coronal view of two femoral condyle sections of a femoral component; **FIG. 8-2C** shows thicker material on a tibial implant component that is associated with designing the sagittal or j-curve of the femoral component to be tilted;
**FIG. 8-3A - 8-3E** show various aspects of femoral component design approach;

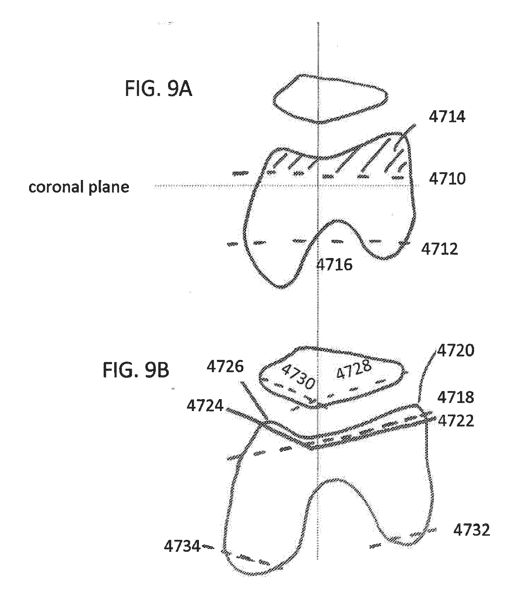

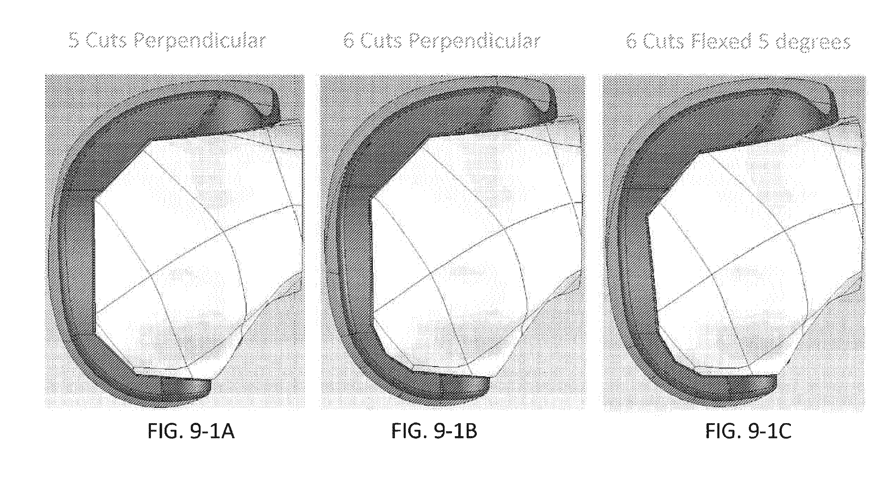

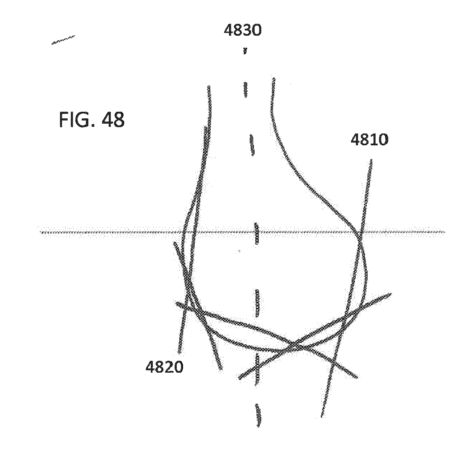

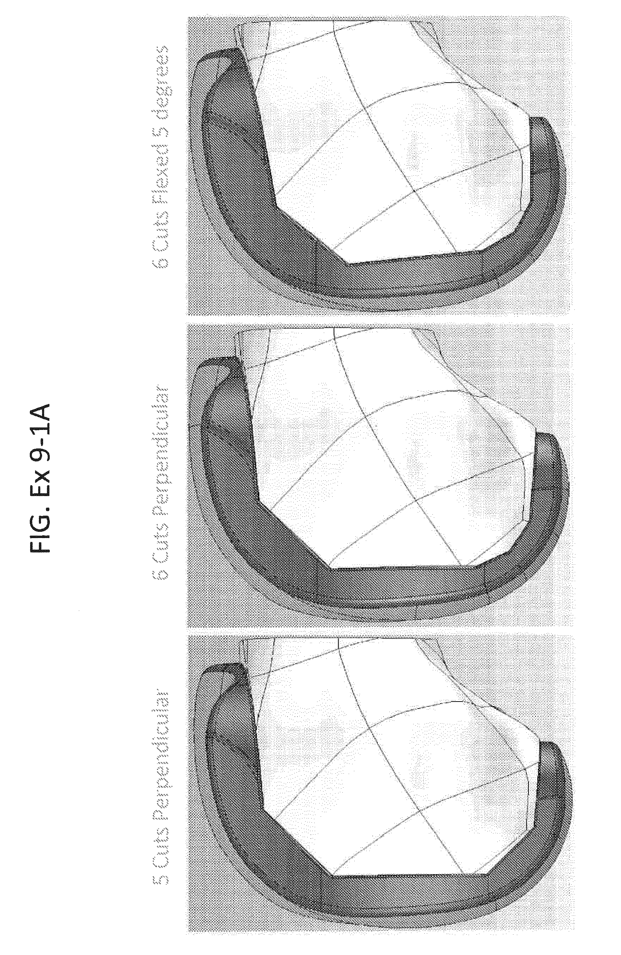

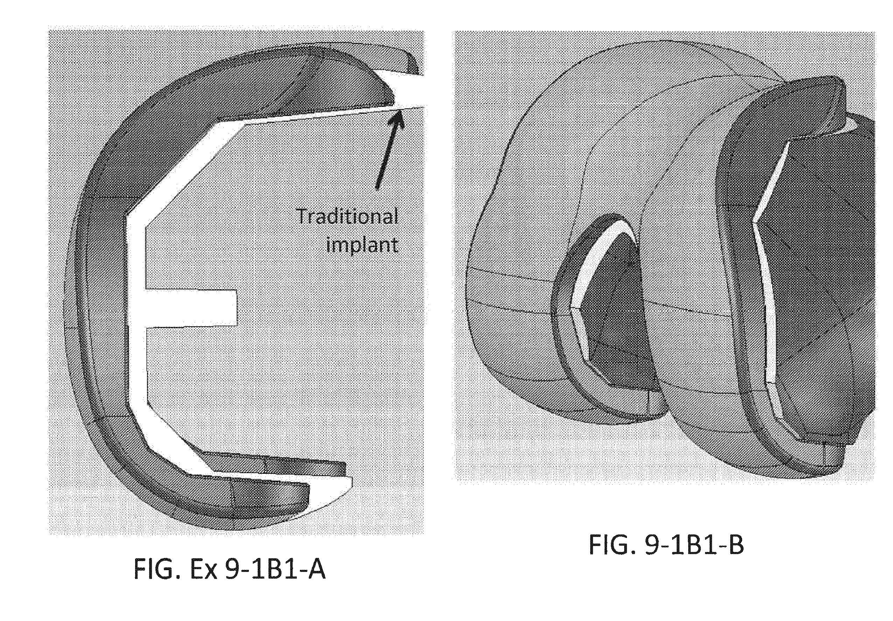

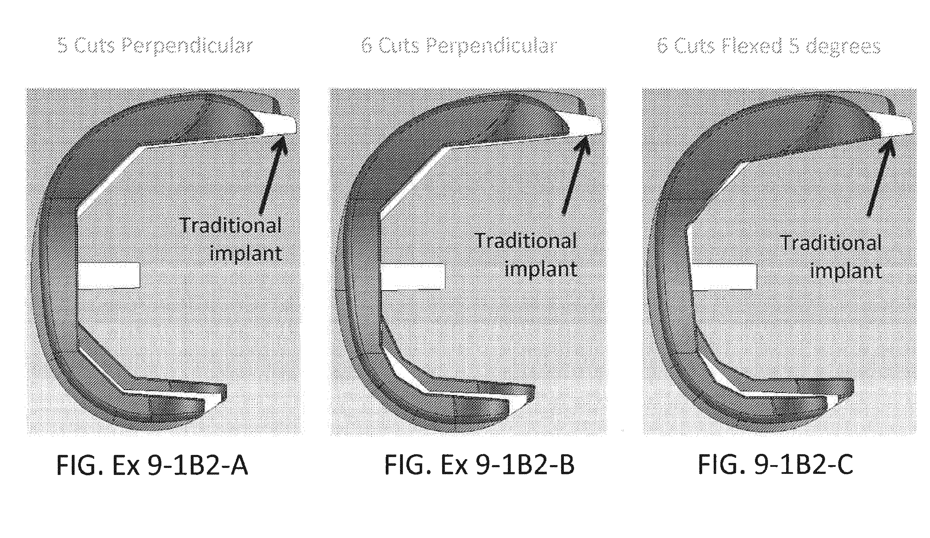

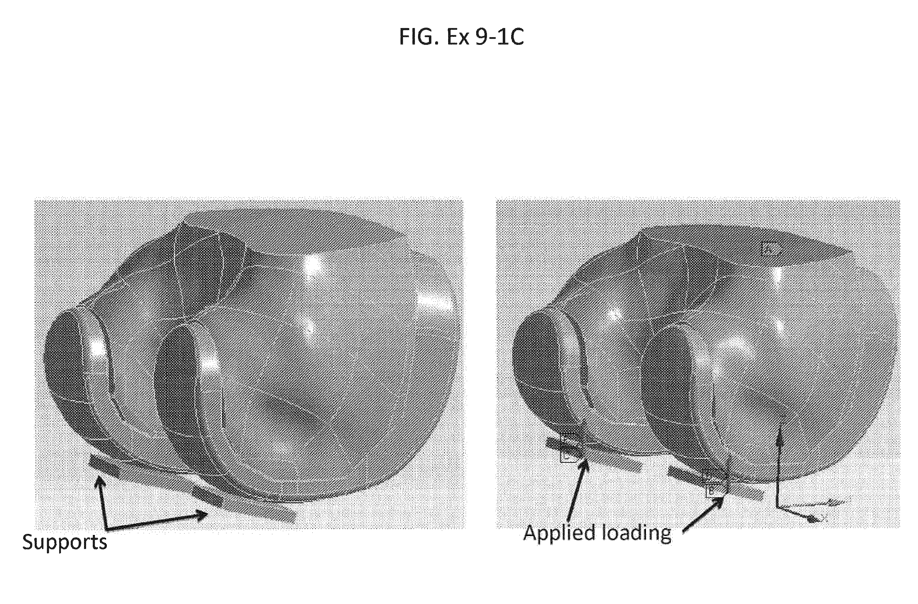

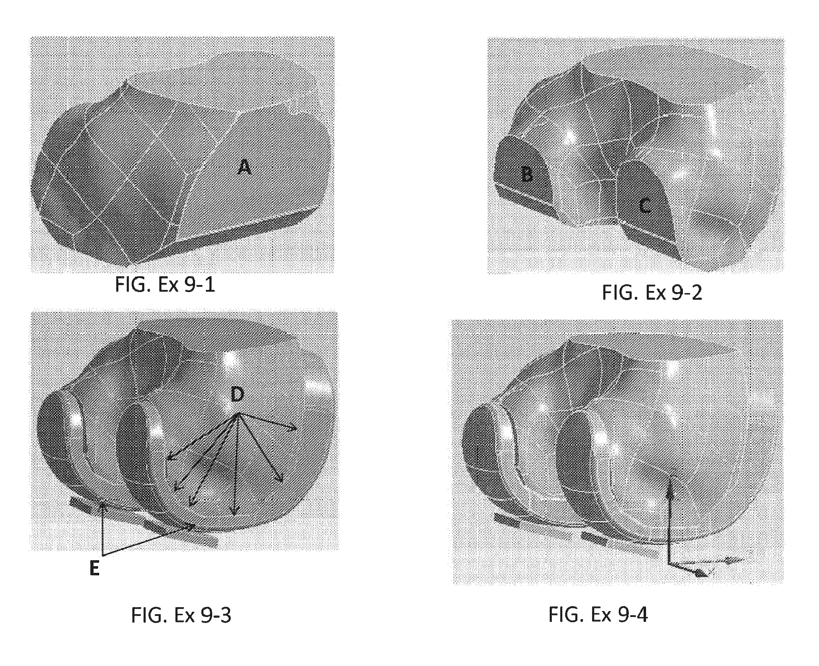

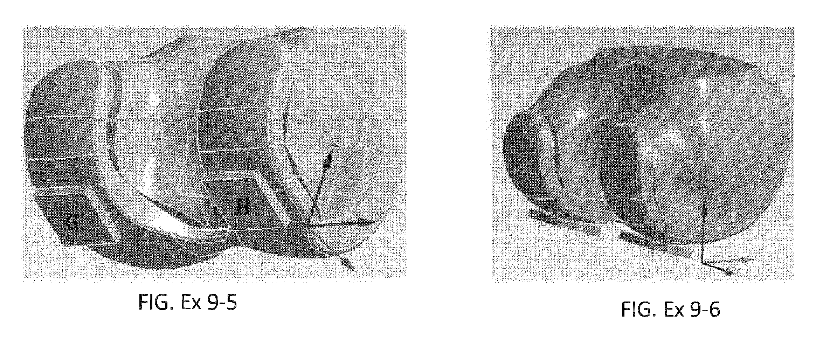

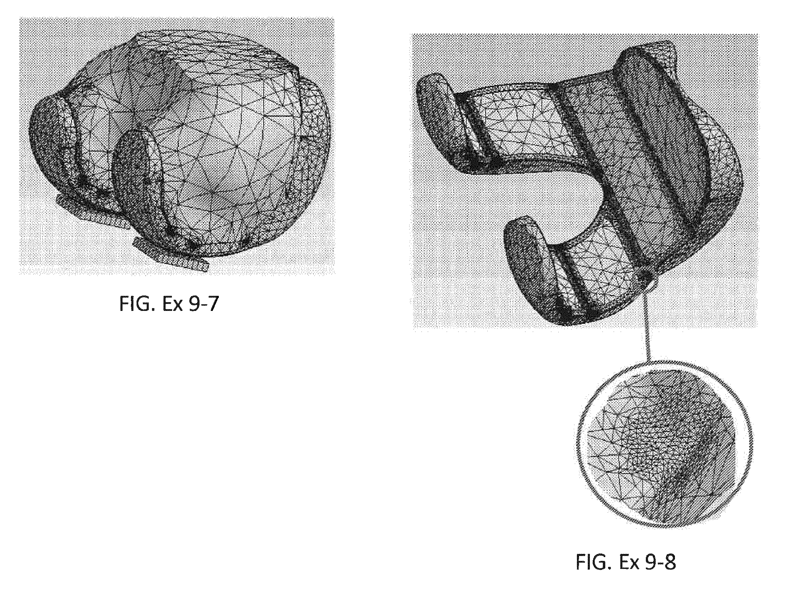

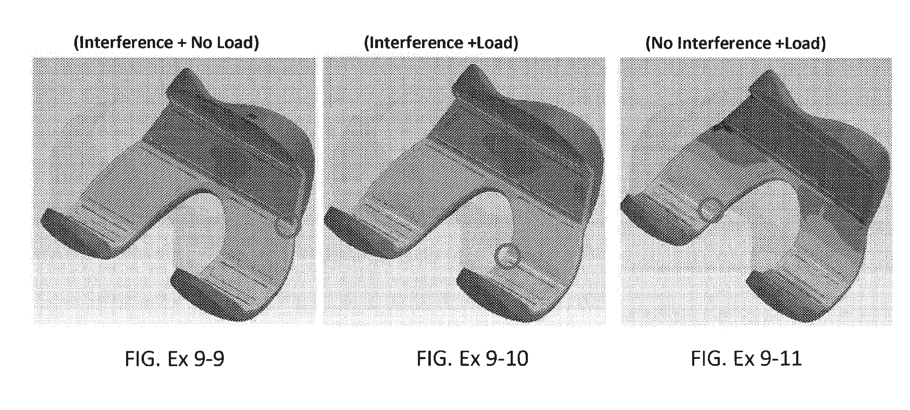

**FIG. 9A and FIG. 9B** are schematic axial views of a femur and patella; **FIG. 9-1A - 9-1C** show three different bone cut for a femoral component;

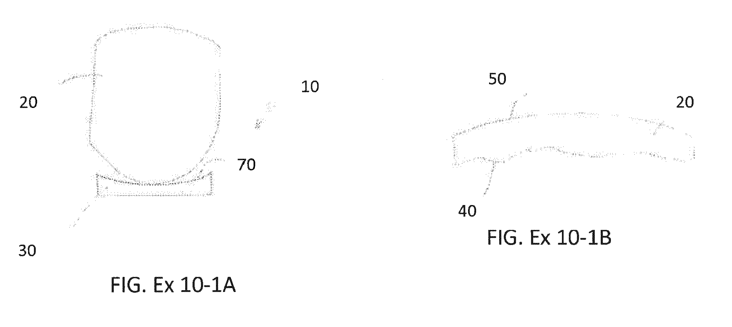

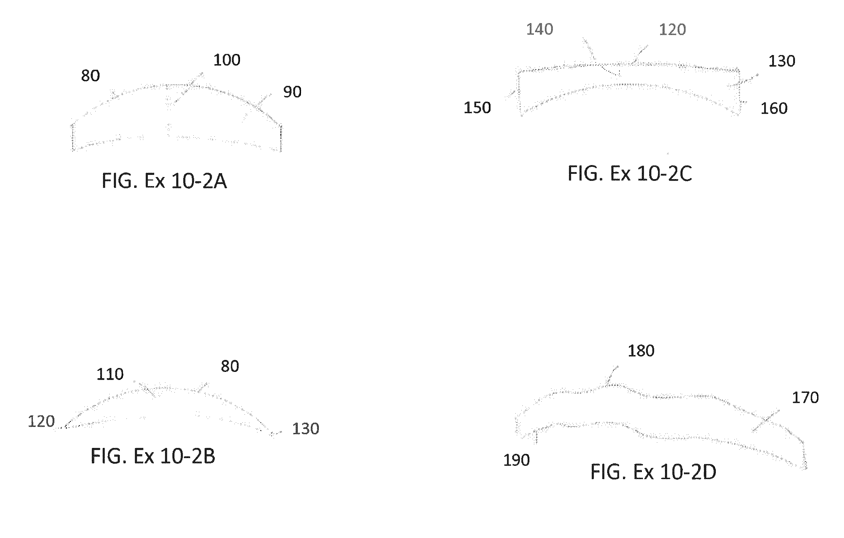

**FIG. 10** is a schematic sagittal view of a femur with facet cuts indicated;

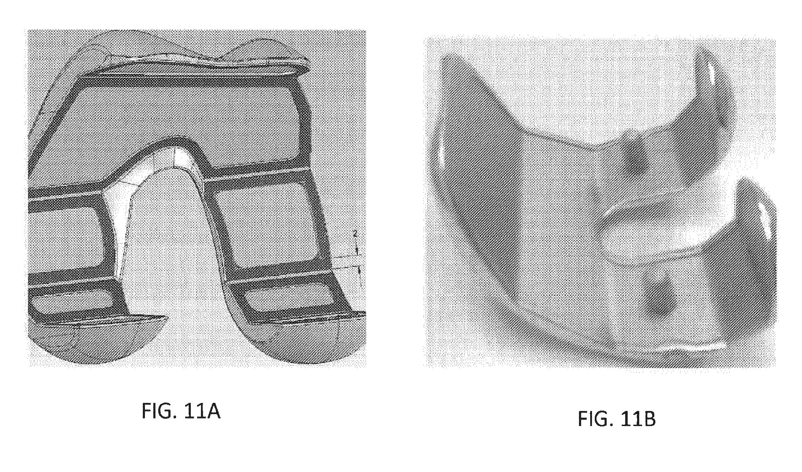

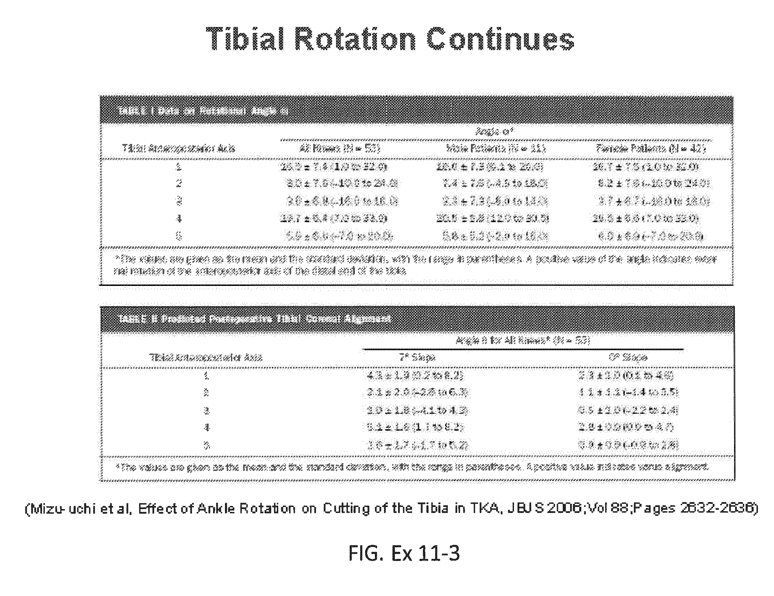

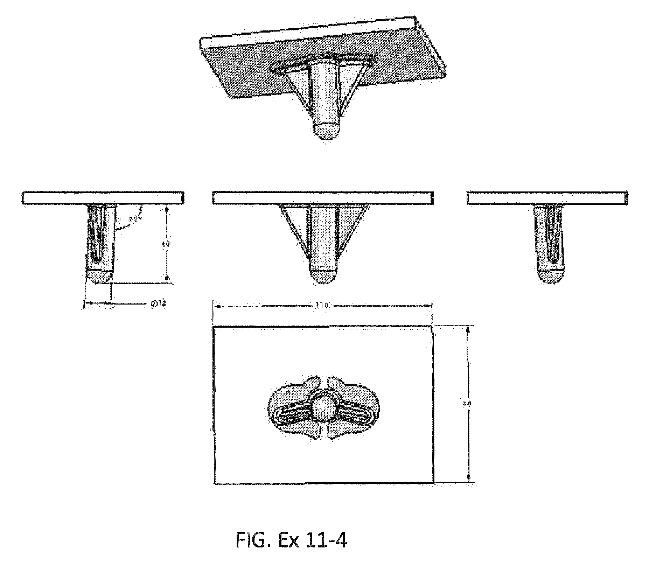

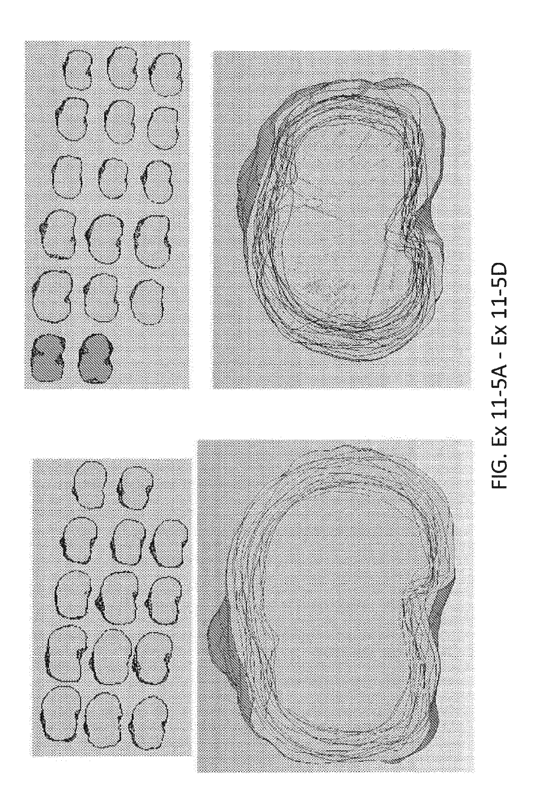

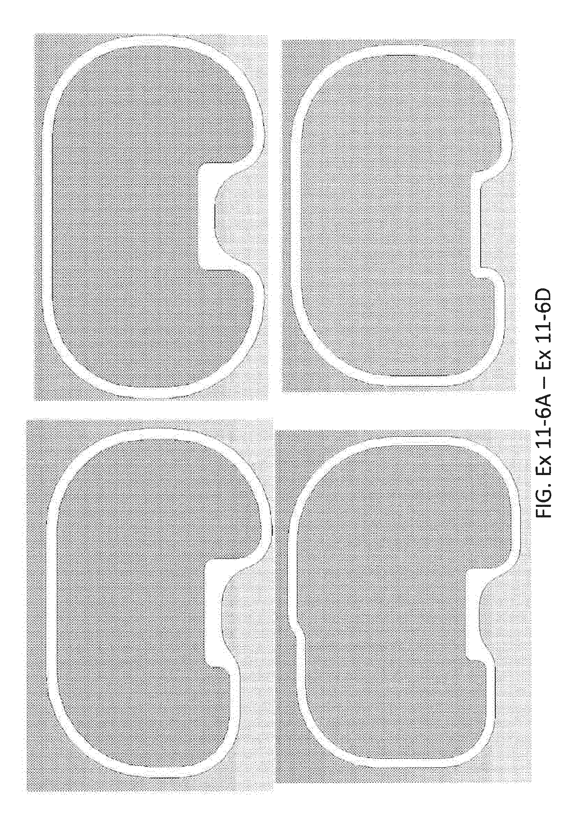

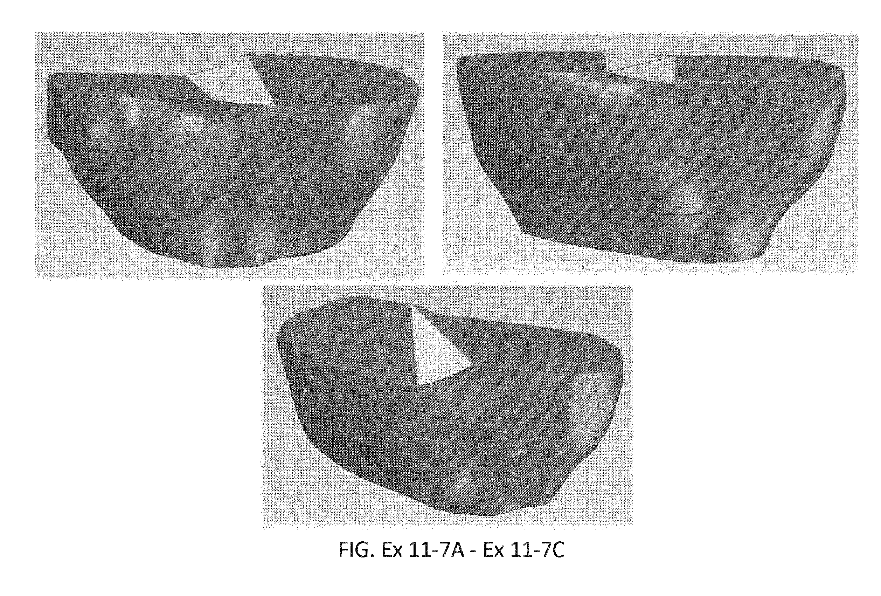

**FIGS. 11A** and **11B** show bone cement pockets in an embodiment of an implant component **(****FIG. 11A****)** and in a traditional component **(****FIG. 11B**);

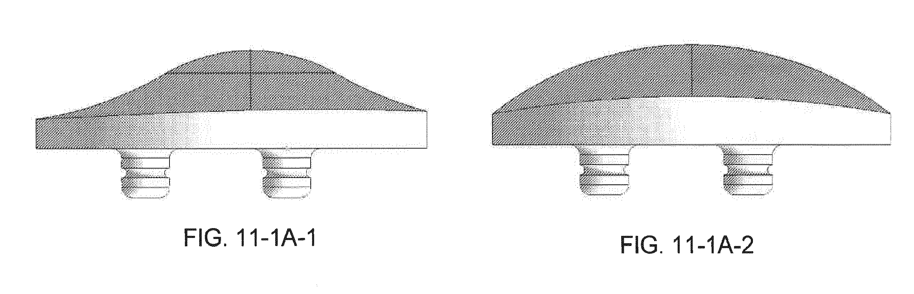

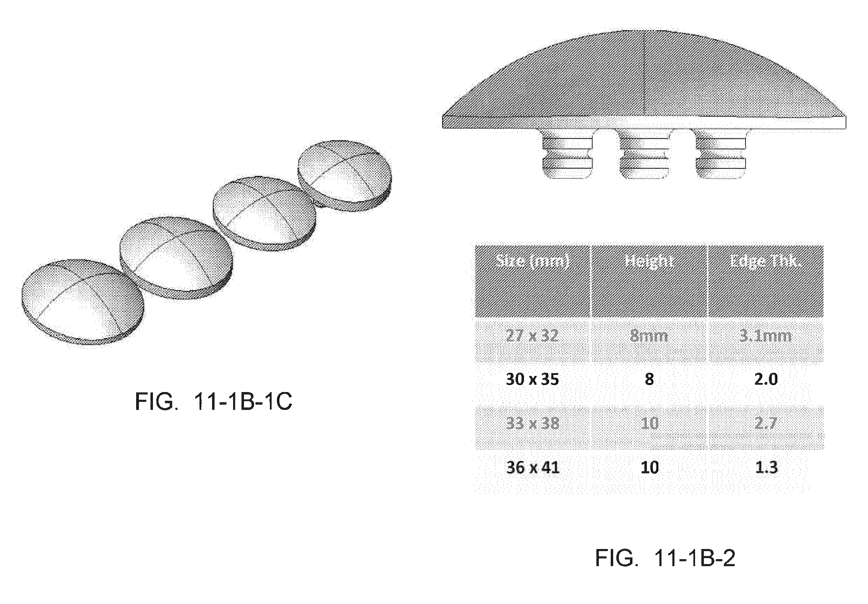

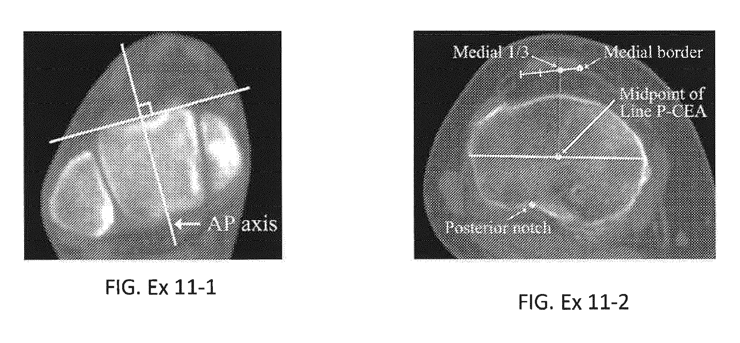

**FIGS. 11-1A** and **11-1B** show exemplary patella implant designs;

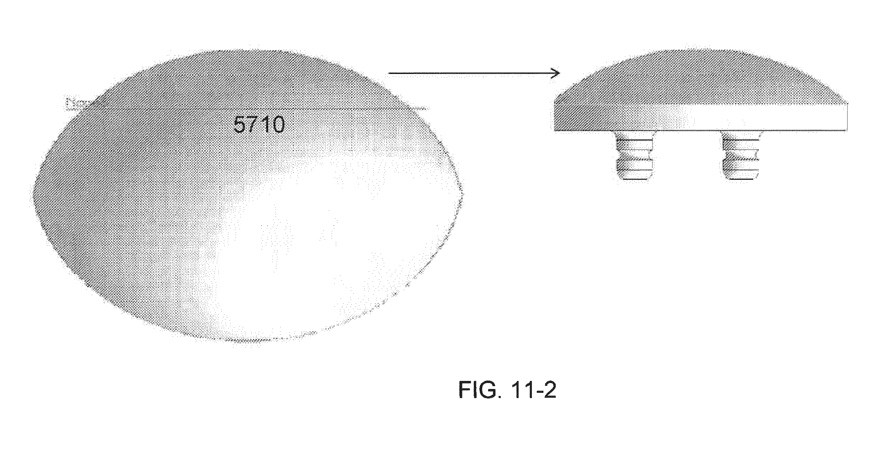

**FIG. 11-2** shows a patella implant component having a prolate shape;

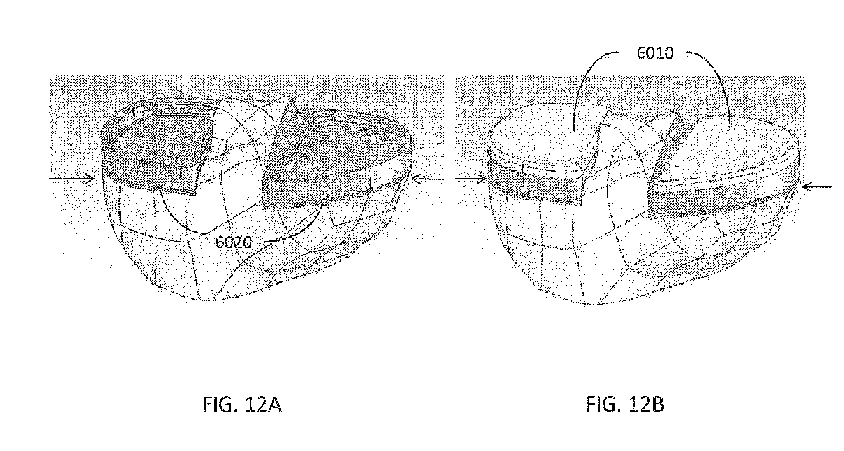

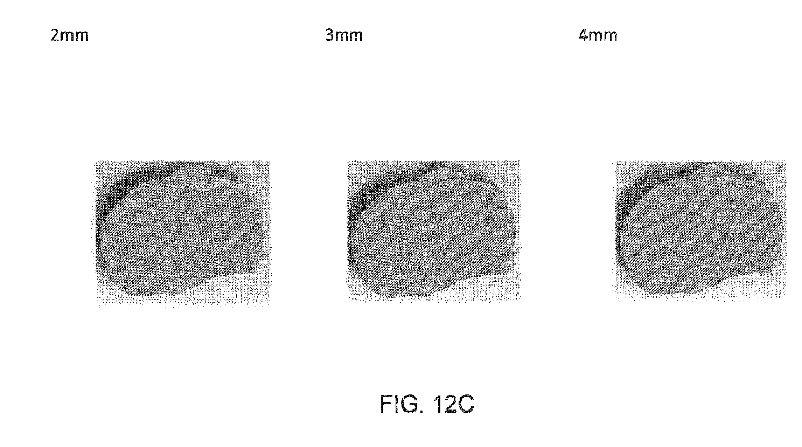

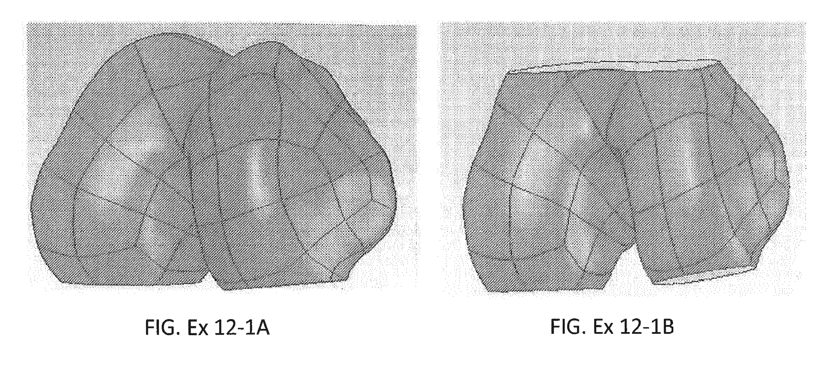

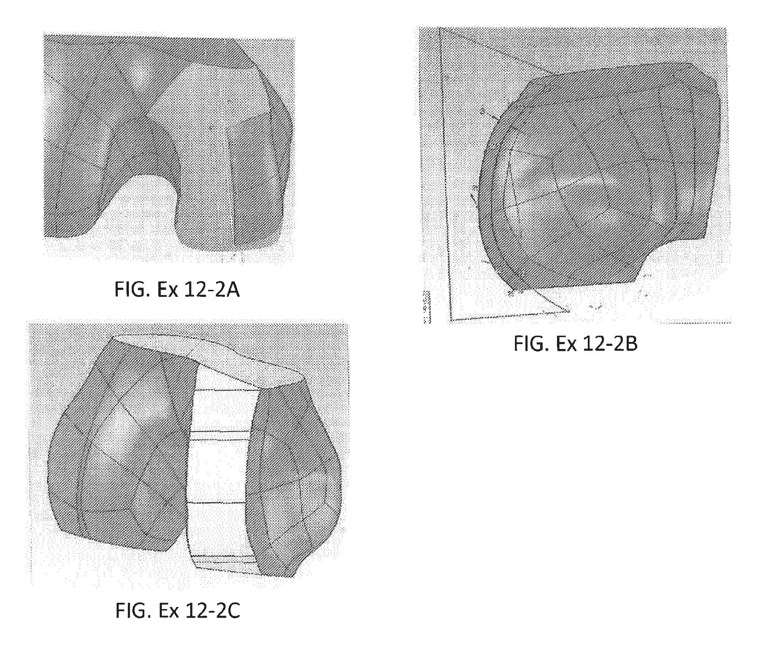

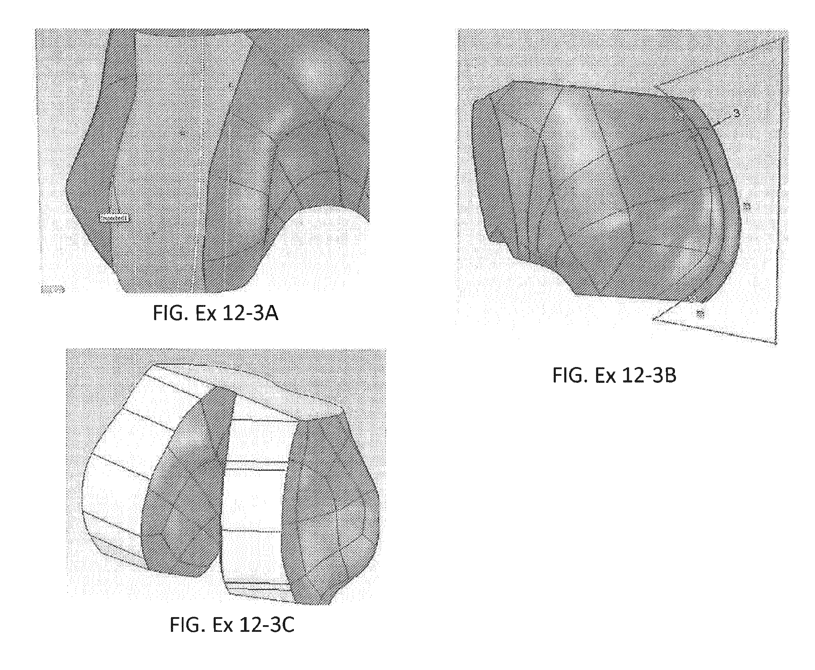

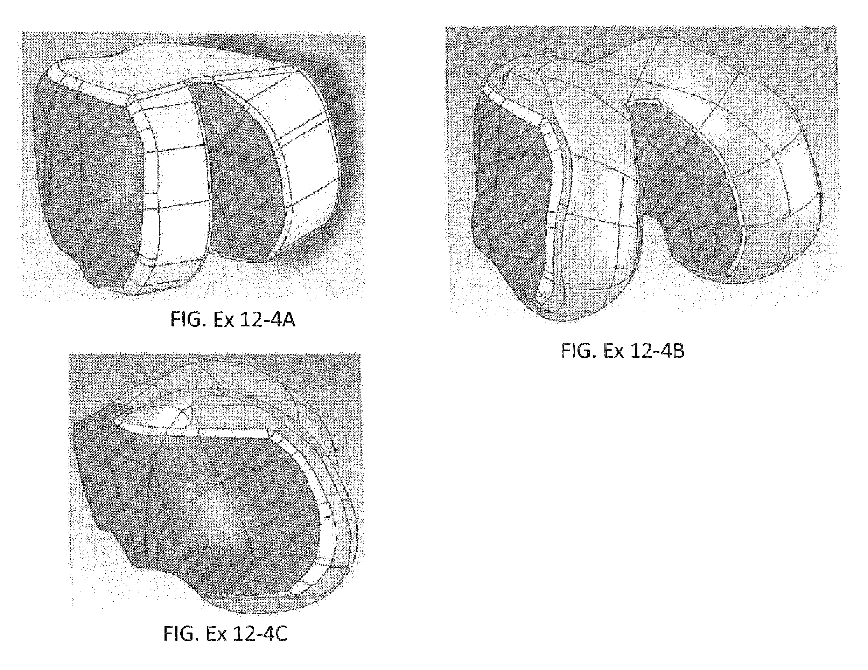

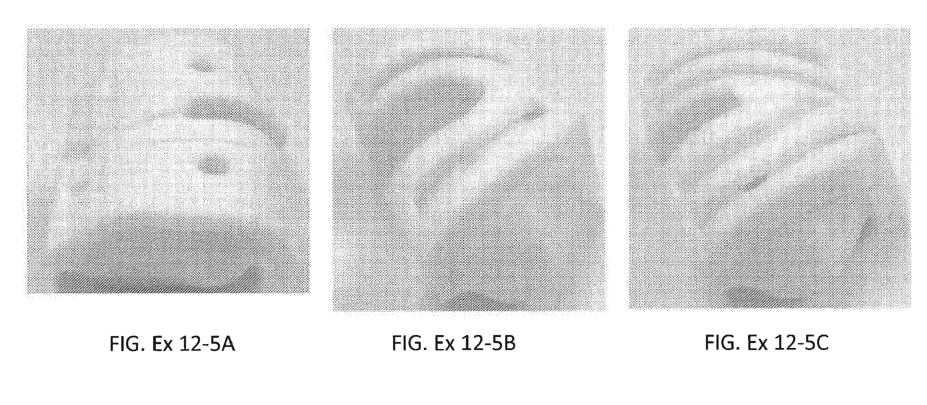

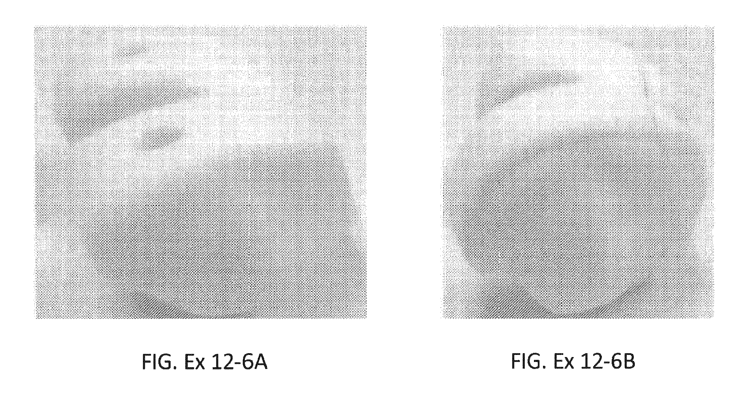

**FIGS. 12A** and **12B** show tibial cuts and unicompartmental medial and lateral components with and without a polyethylene layer having different heights relative to the tibial plateau; **FIGS. 12C - 12E** describe additional considerations of tibial implant designs;

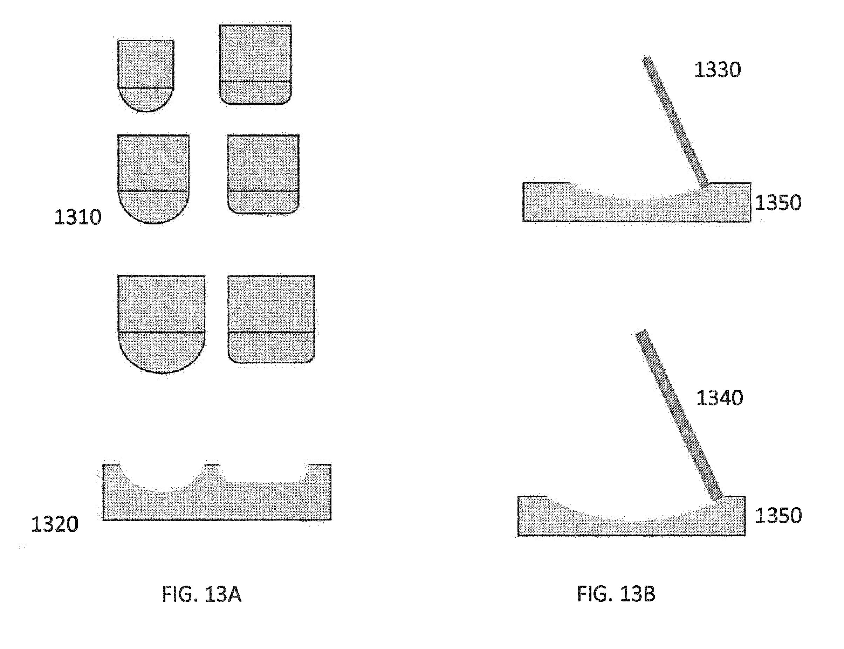

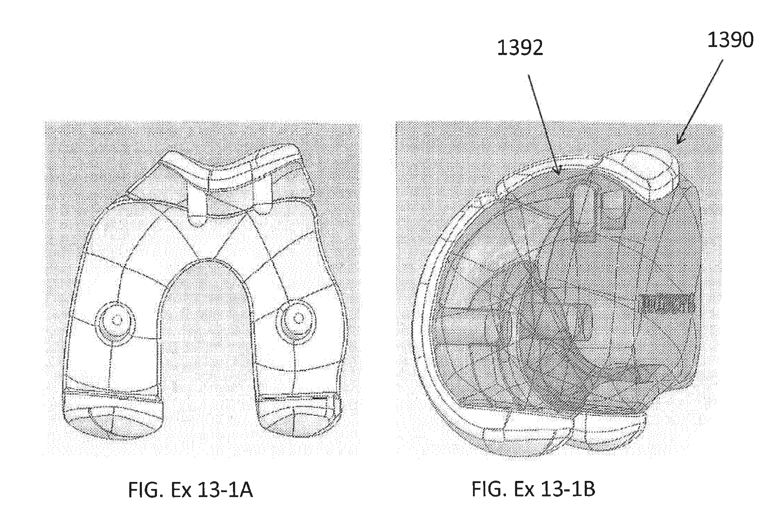

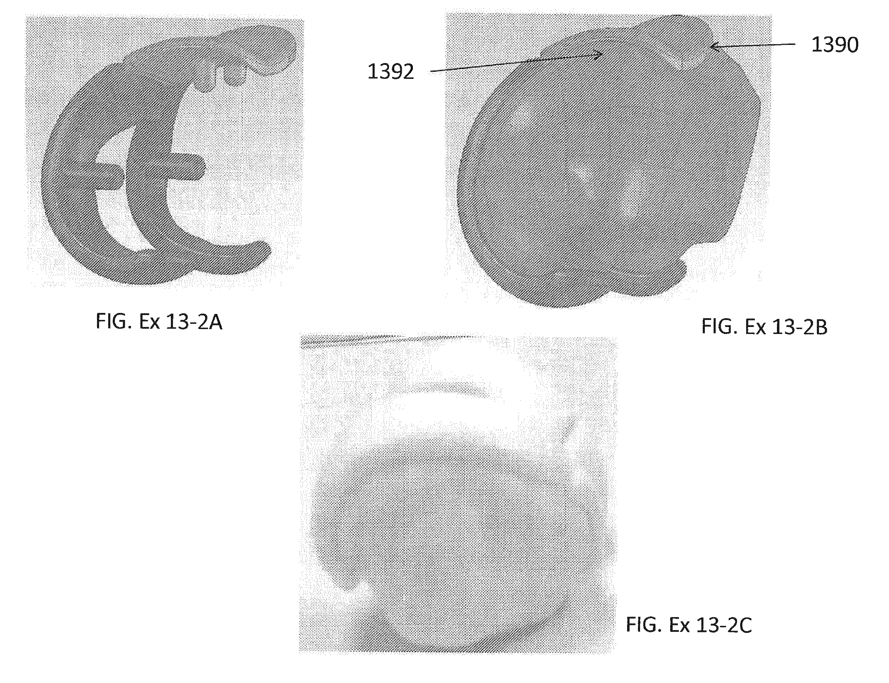

**FIG. 13A** shows six exemplary tool tips and a polyethylene insert in cross-section in the coronal view, the tool tips being used to generate a polyethylene insert having a desired coronal curvature; **FIG. 13B** shows a sagittal view of two exemplary tools sweeping from different distances into a polyethylene insert to create different sagittal curvatures in the polyethylene insert;

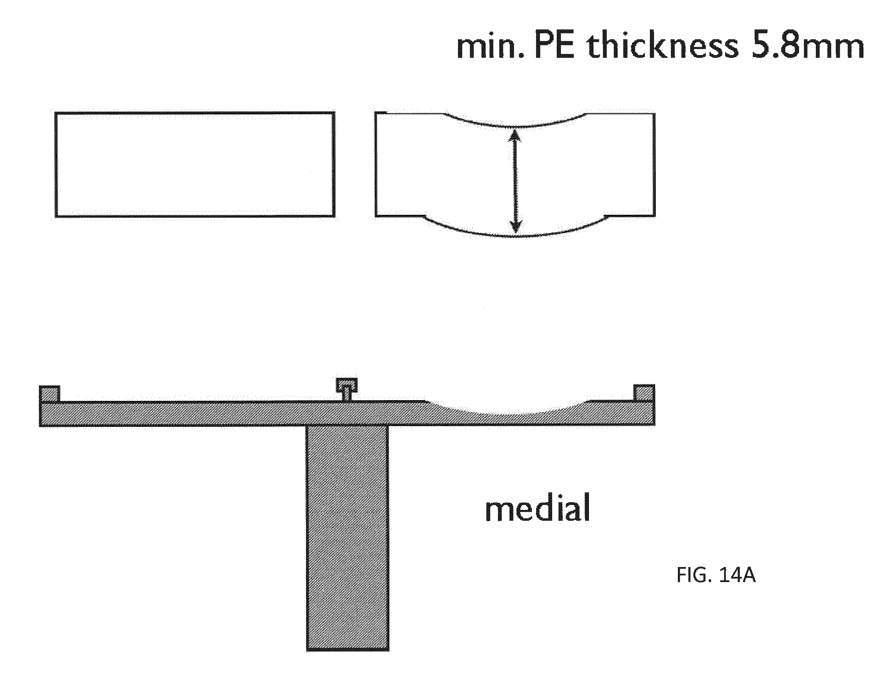

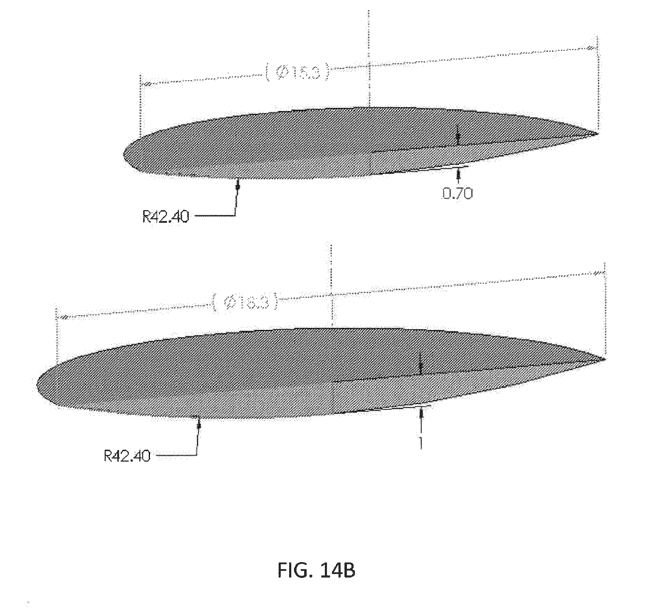

**FIGS. 14A** and **14B** show a tibial implant design with a groove or scallop surface extending through the entire component;
**FIG. Ex 1-1** is a flow chart depicting an exemplary process for designing a patient-specific implant, specifically a total knee implant;
**FIGS. Ex 2-1A - Ex 2-9B** show various aspects of two bone cut design methods;
**FIG. Ex 3-1** shows an exemplary design of an embodiment of an implant component having seven cuts on the inner, bone-facing surface;
**FIG. Ex 3-2A** and **FIG. 3-2B** are sagittal views of exemplary designs of anterior and posterior femoral bone cuts, respectively, which correspond to the inner, bone facing surfaces of the virtual model shown in **FIG. Ex 3-1;**
**FIG. Ex 3-3** shows an exemplary design of an implant component having seven cuts on the inner, bone-facing surface and having cement cut-outs and pegs with particular dimensions;
**FIG. Ex 3-4A** and **FIG. Ex 3-4B** show virtual models of bone cuts and corresponding bone volume for a model having five bone cuts to the femoral articular surface **(FIG. Ex 3-4A)** and for a model having seven bone cuts to the femoral articular surface **(FIG. Ex 3-4B);**
**FIG. Ex 3-5A** and **FIG. Ex 3-5B** show virtual models of bone cuts and corresponding bone volume for a model having five, not flexed bone cuts to the femoral articular surface **(FIG. Ex 3-5A)** and for a model having five, flexed bone cuts to the femoral articular surface **(FIG. Ex 3-5B**);
**FIGS. Ex 3-6A - Ex 3-6D** show exemplary virtual models of bone cuts overlaid (in hatched lines) with the shape of a traditional implant;
**FIG. Ex 4-1A - Ex 4-1F** show various aspects of a knee implant, including a femoral component and a patella component, with a material cutaway region highlighted in red in certain figures;
**FIGS. Ex 5-1A - Ex 5-7B** show various aspects of a set of jigs for guiding patient-specific bone cuts in a femur-first technique;
**FIGS. Ex 6-1 - Ex 6-4** show various aspects of a set of jigs for guiding patient-specific bone cuts in a tibia-first technique;
**FIGS. Ex 7-1A - Ex 7-5** show various aspects of a tibial implant design and cut technique;
**FIGS. Ex 8-1A - Ex 8-3E** show various aspects of tibial tray and insert designs;
**FIGS. Ex 9-1A - Ex 9-11** show various aspects of a finite element analysis ("FEA") conducted on three variations of a femoral implant component;
**FIG. Ex 10-1A** is a front schematic view of a knee implant;
**FIG. Ex 10-1B** is a cross-sectional schematic view in the coronal plane of a femoral component of the implant of **FIG. Ex 10-1A;**
**FIGS. Ex 11-1 - Ex 11-7C** illustrate various aspects of a design for a tibial implant component;
**FIGS. Ex 12-1A and 12-1B** illustrate a computer model of a distal femur with posterior and anterior cut lines;
**FIGS. Ex 12-2A - Ex 12-2C** illustrate a computer model of a distal femur with a design for a curvilinear cut line to the medial condyle;
**FIGS. Ex 12-3A - Ex 12-3C** illustrate a computer model of a distal femur with a design for a curvilinear cut line to the lateral condyle;
**FIGS. Ex 12-4A - Ex 13-4C** illustrate a computer model of a distal femur with a design for all cut lines and with a design for the corresponding implant component;
**FIGS. Ex 12-5A - Ex 12-5C** illustrate models of a distal femur and jigs for making curvilinear cuts;
**FIGS. Ex 12-6A and Ex 12-6B** illustrate models of a distal femur and an implant having curvilinear cuts;
**FIGS. Ex 13-1A and Ex 13-1B** illustrate a design of a femoral implant including a single, posterior cut on its inner, bone-facing surface;
**FIGS. Ex 13-2A and Ex 13-2B** illustrate a design of a femoral implant including no cuts on its inner, bone-facing surface; and
**FIG. Ex 13-2C** illustrates a model of a femur and a femoral implant designed to include no cuts on its inner, bone-facing surface.

### DETAILED DESCRIPTION

When a surgeon uses a traditional off-the-shelf implant to replace a patient's joint, for example, a knee joint, hip joint, or shoulder joint, certain spatial aspects of the implant typically do not match certain spatial aspects of the particular patient's biological structures at the joint. These mismatches cause various complications during and after surgery. For example, surgeons may need to extend the surgery time and apply best guesses and rules of thumb during surgery to address the mismatches. Moreover, to improve the match between a traditional implant and a patient's biological structures, surgeons typically remove substantial portions of the patient's articular bones so that the patient's articular surfaces fit the standard shape of the bone-facing surface of the traditional implant.

For the individual patient, complications associated with mismatches can include pain, discomfort, and an unnatural feeling of the joint, as well as an altered range of movement and an increased likelihood of implant failure. Moreover, the loss of substantial portions of bone associated with implantation of a traditional primary implant typically limits the patient to only one subsequent revision implant.

The present invention relates to patient-specific implants and methods for designing, making, and using the same. Some embodiments relate to articular implant components having one or more patient-specific aspects adapted to a feature of the patient's biology, such as biological structure, alignment, kinematics, and/or soft tissue impingements. The one or more patient-specific aspects can include, but are not limited to, implant component surfaces, such as surface contours, angles or bone cuts, and implant component dimensions, such as thickness, width, or length. The patient-specific aspect(s) of the implant component can be designed from patient-specific data to match an existing feature of the patient's biology. Alternatively, the patient-specific aspect(s) of the implant component can be patient-engineered from patient-specific data to improve an existing feature of the patient's biology.

The implants and methods of certain embodiments can be applied to any joint including, without limitation, a spine, spinal articulations, an intervertebral disk, a facet joint, a shoulder joint, an elbow, a wrist, a hand, a finger joint, a hip, a knee, an ankle, a foot, or a toe joint. Furthermore, various embodiments can be adapted and applied to implant instrumentation used during surgical or other procedures, and to methods of using various patient-specific implants, instruments, and other devices.

In certain aspects, the implants and methods include a patient-specific inner surface for attaching to a patient's resectioned bone. In particular, patient-specific data collected preoperatively is used to determine one or more patient-specific bone cuts to a patient's bone and to the inner, bone-facing surface of an implant component. The bone cuts are optimized (i.e., patient-engineered) to maximize one or more parameters, such as: (1) deformity correction and limb alignment (2) maximum preservation of bone, cartilage, or ligaments, (3) preservation and/or optimization of other features of the patient's biology, such as trochlea and trochlear shape, (4) restoration and/or optimization of joint kinematics, and/or (5) restoration or optimization of joint-line location and/or joint gap width. Based on the optimized bone cuts, the implant's inner, bone-facing surface is designed to, at least in part, negatively-match the shape of the cut bone. In addition, the implant's outer, joint-facing surface can be designed to, at least in part, substantially negatively-match the opposing surface at the joint cavity. Accordingly, certain embodiments are directed to implants and methods that address many of the problems associated with traditional implants, such as mismatches between an implant and a patient's biological structure(s), and substantial bone removal that limits subsequent revisions following a traditional primary implant.

Certain embodiments are directed to patient-specific implants and implant designs applied as a pre-primary implant device, such that a subsequent, replacement implant can be performed with a second (and, optionally, a third) patient-specific pre-primary implant device or with a traditional primary implant. Certain embodiments are directed to patient-specific implants and implant designs applied as a primary implant device, such that a subsequent, replacement implant can be performed as a traditional revision. Certain embodiments are directed to patient-specific implants and implant designs applied as a revision implant device, such that a subsequent revision may be possible with a second, patient-specific implant having one or more patient-specific aspects.

In certain aspects, the implants and methods can include one or more patient-specific aspects and one or more standard aspects. For example, a curved surface of an implant component can include one or more dimensions or radii that are patient-specific, and one or more dimensions or radii that are standard. For example, in certain embodiments, a condyle portion of a femoral implant component and/or a corresponding groove in the bearing surface of a tibial implant component can include a patient-specific sagittal curvature or radii and a standard coronal curvature or radii. The patient-specific curvature or radii can be designed from patient-specific data to adapt to an existing feature of the patient's biology or it can be patient-engineered from patient-specific data to improve an existing feature of the patient's biology. Standard curvature or radii include curvatures or radii used in implants for all, or a collection of, patients.

### 1. Exemplary implant systems and patient-specific features

Various embodiments of implants and implant systems are contemplated herein, including, but not limited to, knee-joint implants, hip-joint implants, and shoulder-joint implants. In certain embodiments, an implant or implant system can include one, two, three, four or more components. An implant component can be designed and/or manufactured to include one or more patient-specific features that substantially match one or more of the patient's biological structures, such as bone, cartilage, tendon, or muscle. In addition or alternatively, an implant component can be designed and/or manufactured to include one or more patient-specific features that substantially match one or more other implant components. In addition, an implant component can be designed and/or manufactured to include one or more non-patient-specific features that substantially match one or more other implant components.

The term "implant component" as used herein is envisioned to include (i) one of two or more devices that work together in an implant or implant system, or (ii) a complete implant or implant or implant system, for example, in embodiments in which an implant is a single, unitary device. The term "match" as used herein is envisioned to include one or both of a negative-match, as a convex surface fits a concave surface, and a positive-match, as one surface is identical to another surface.

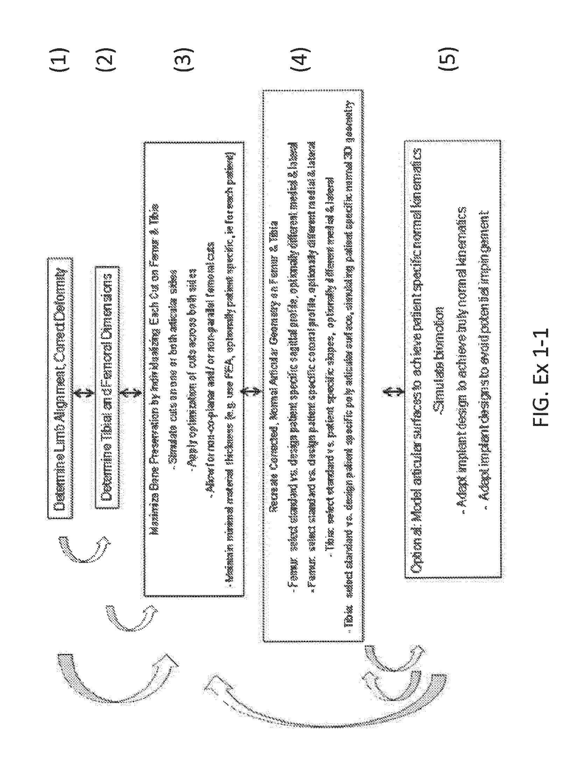

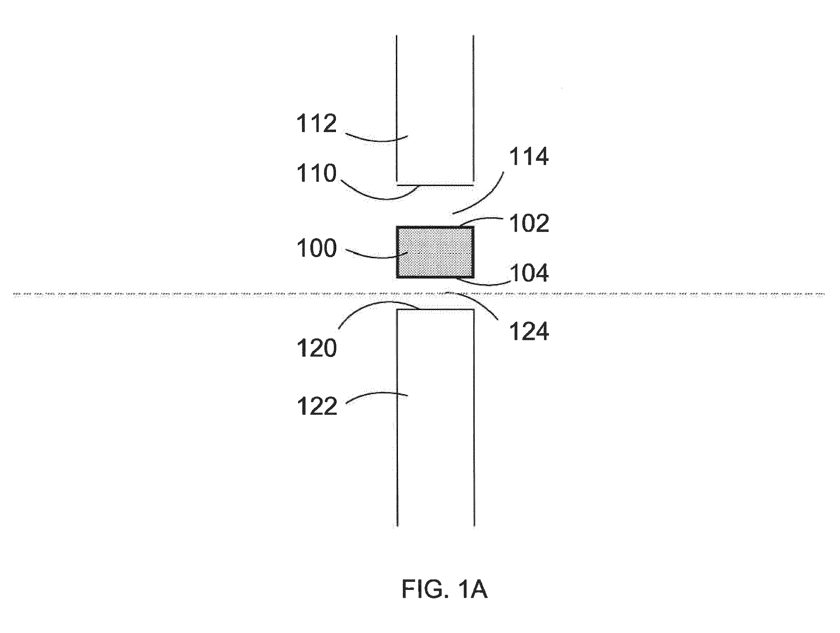

Three exemplary embodiments of implants or implant components are schematically represented in **FIGS. 1A - 1C****.** The dashed line across the figures represents an exemplary joint line. **FIG. 1A** shows an exemplary implant component **100.** The component **100** includes an inner, bone-facing surface **102** and an outer, joint-facing surface **104.** The inner bone-facing surface **102** engages a first articular surface **110** of a first biological structure **112** at a first interface **114.** The articular surface **110** can be a native surface or a cut surface. The outer, joint-facing surface **104** opposes a second articular surface **120** of a second biological structure **122** at a joint interface **124.** In certain embodiments, one or more features of the implant component, for example, an M-L, A-P, or S-I dimension, a feature of the inner, bone-facing surface **102,** and/or a feature of the outer, joint-facing surface **104,** are patient-adapted (i.e., include one or more patient-specific and/or patient-engineered features).

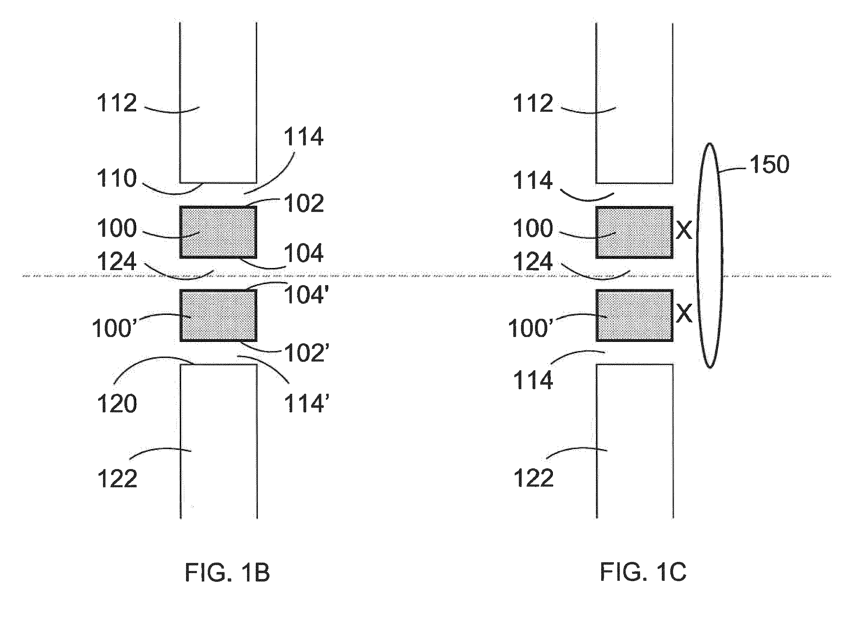

The implant embodiment shown in **FIG. 1B** includes two implant components **100, 100'.** Each implant component **100, 100'** includes an inner, bone-facing surface **102, 102'** and an outer, joint-facing surface **104, 104'.** The first inner, bone-facing surface **102,** engages a first articular surface **110** of a first biological structure **112** at a first interface **114.** The first articular surface **110** can be a native surface or a cut surface. The second bone-facing surface **102'** engages a second articular surface **120** of a second biological structure **122** at a second interface **114'.** The second articular surface **120** can be a native surface or a cut surface. In addition, an outer, joint-facing surface **104** on the first component **100** opposes a second, outer joint-facing surface **104'** on the second component **100'** at the joint interface **124.** In certain embodiments, one or more features of the implant component, for example, one or both of the inner, bone-facing surfaces **102, 102'** and/or one or both of the outer, joint-facing surfaces **104, 104',** are patient-adapted (i.e., include one or more patient-specific and/or patient-engineered features).

The implant embodiment represented in **FIG. 1C** includes the two implant components **100, 100',** the two biological structures **112, 122,** the two interfaces **114, 114',** and the joint interface **124,** as well as the corresponding surfaces described for the embodiment represented in **FIG. 1B****.** However, **FIG. 1C** also includes structure **150,** which can be an implant component in certain embodiments or a biological structure in certain embodiments. Accordingly, the presence of a third structural **150** surface in the joint creates a second joint interface **124',** and possibly a third **124",** in addition to joint interface **124.** Alternatively or in addition to the patient-adapted features described above for components **100** and **100',** the components **100, 100'** can include one or more features, such as surface features at the additional joint interface(s) **124, 124",** as well as other dimensions (e.g., height, width, depth, contours, and other dimensions) that are patient-adapted, in whole or in part. Moreover, structure **150,** when it is an implant component, also can have one or more patient-adapted features, such as one or more patient-adapted surfaces and dimensions.

As mentioned above, traditional off-the-shelf implants and implant components can have inner, bone facing surfaces that are a poor match to a particular patient's biological structure(s). Moreover, the traditional products can have outer, joint-facing surfaces that poorly match a particular patient's healthy or ideal joint. The patient-specific implants and methods of some embodiments that improve upon these deficiencies are described in more detail in the next two subsections, with respect to the bone-facing surface and the joint-facing surface of an implant component; however, the principles described herein are applicable to any surface of an implant or implant component.

### 1.1 Bone-facing surface of an implant component

In certain embodiments, the bone-facing surface of an implant component can be designed to substantially negatively-match one more bone surfaces. For example, in certain embodiments at least a portion of the bone-facing surface of a patient-specific implant component can be designed to substantially negatively-match the shape of subchondral bone, cortical bone, endosteal bone, or bone marrow. A portion of the implant also can be designed for resurfacing, for example by negatively-matching portions of a bone-facing surface of the implant component to the subchondral bone or cartilage.

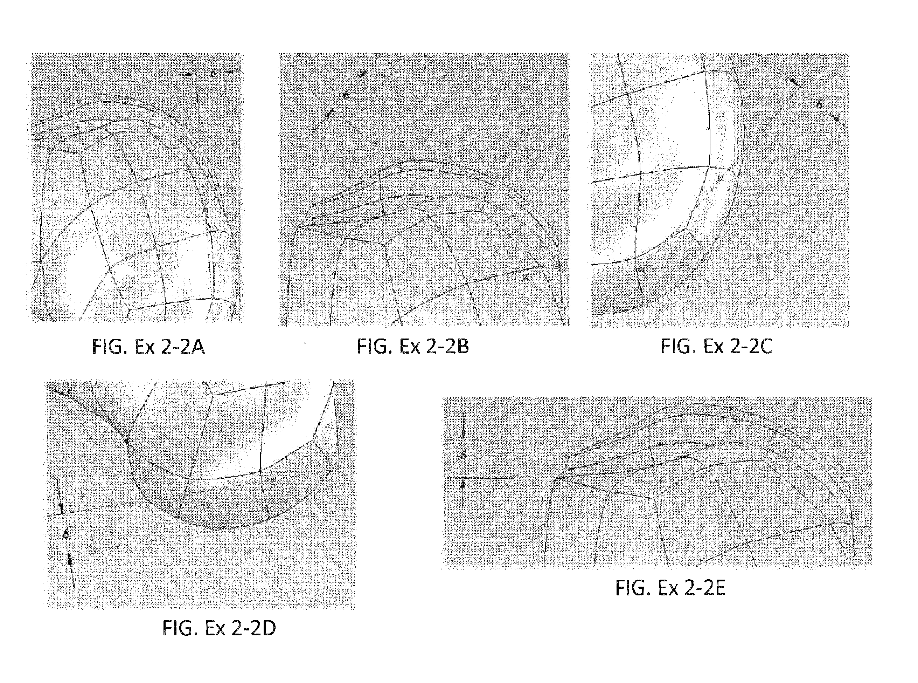

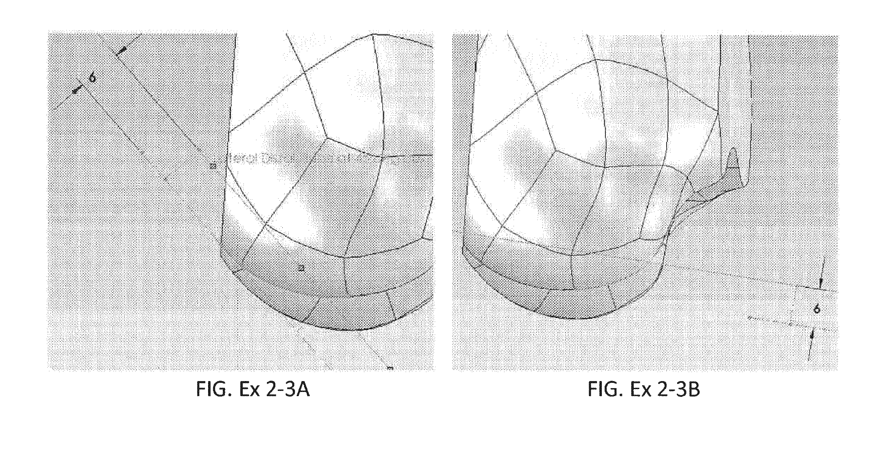

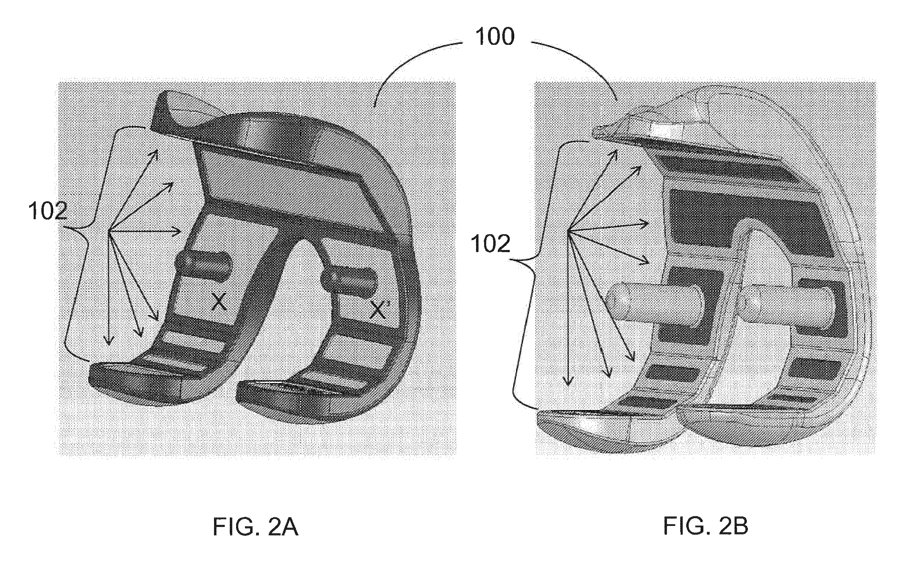

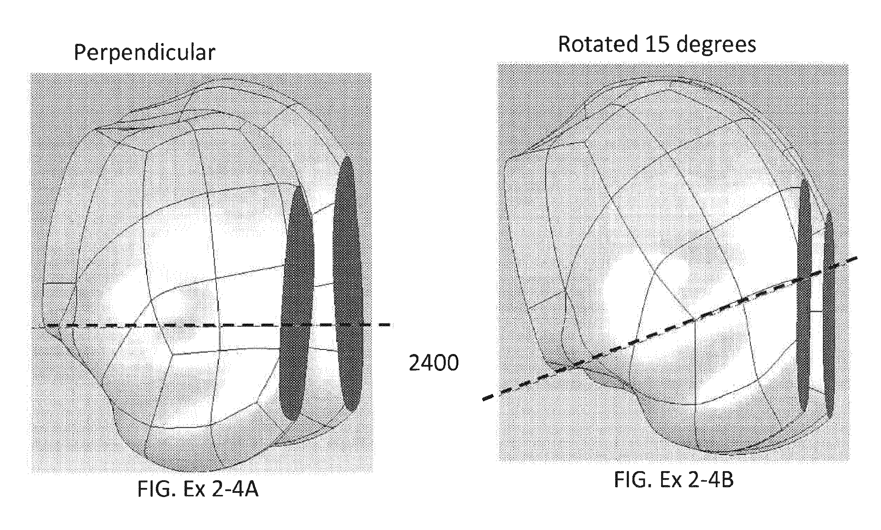

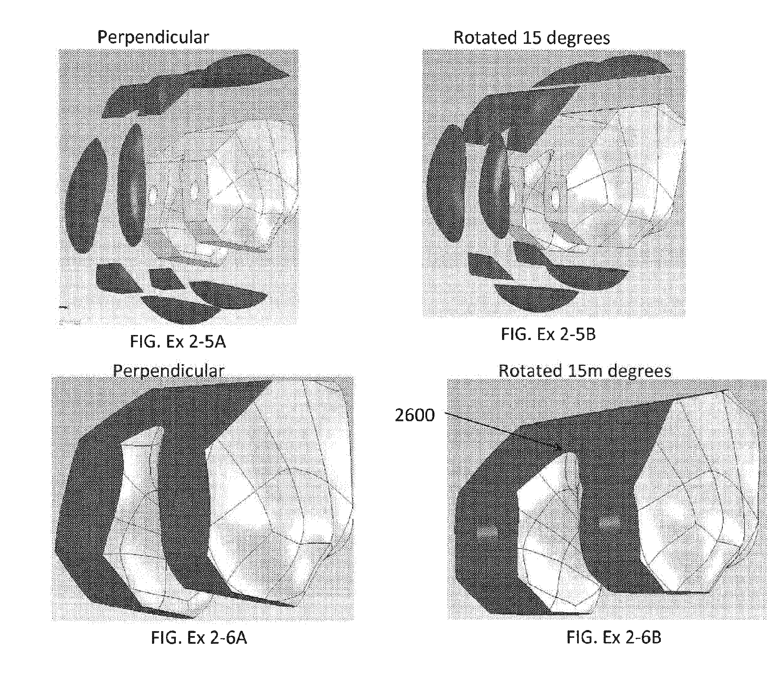

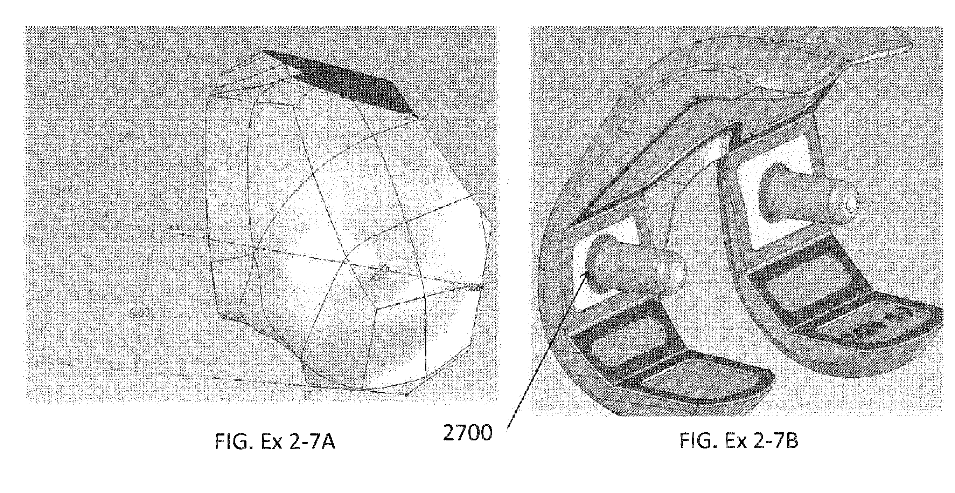

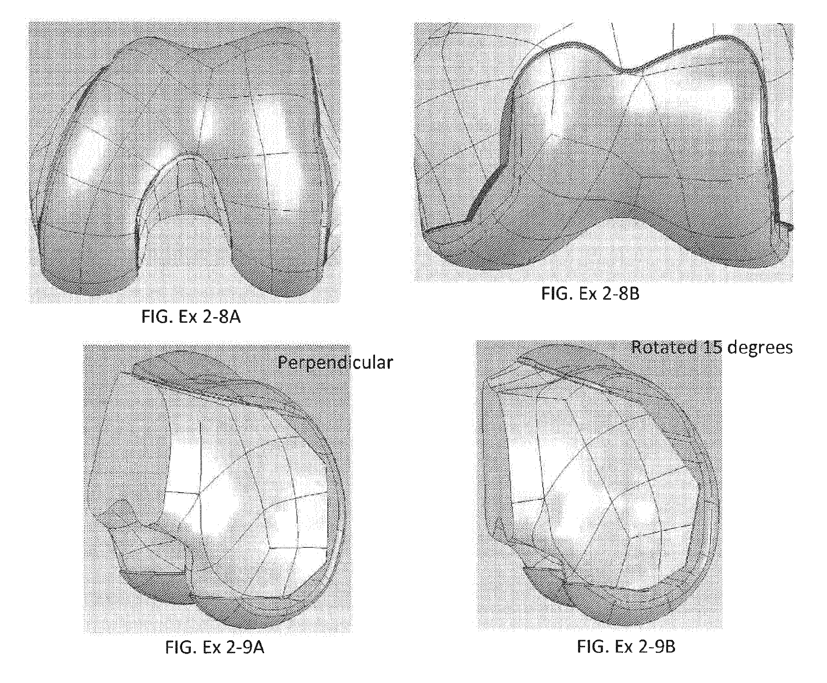

In certain embodiments, the bone-facing surface of a patient-specific implant component includes bone cuts. For example, the bone-facing surface of the implant can be designed to substantially negatively-match one or more bone surfaces derived from one or more cuts to the bone. The bone-facing surface of the implant can include any number of bone cuts, for example, two, three, four, less than five, five, more than five, six, seven, eight, nine or more bone cuts. **FIG. 2A** depicts a design of a femoral implant component **100** having six bone cuts. **FIG. 2B** depicts a design of a femoral implant component **100** having seven bone cuts.

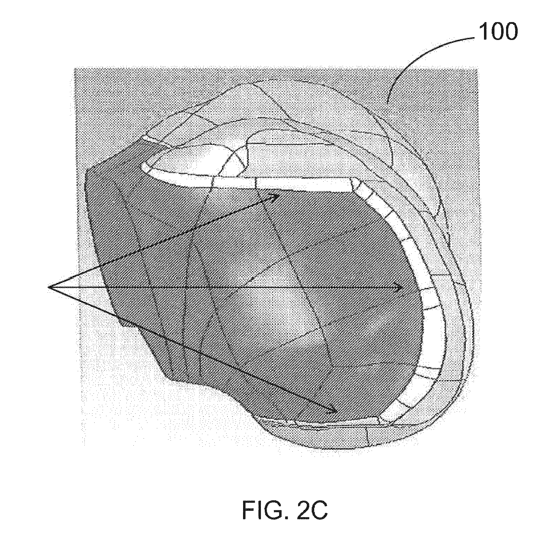

In the figures, the six or seven respective bone cuts are identified by arrows on the inner, bone-facing surface **102** of the implant component **100.** As shown by the implant designs in the figures, each of the bone cuts on the bone-facing surface can be substantially planar. However, in certain embodiments, one or more bone cuts can be curvilinear. In certain embodiments, the entire bone-facing surface can be substantially curvilinear. **FIG. 2C** depicts a design of a femoral implant component **100** having three bone cuts, one of which is a curvilinear bone cut.

In certain embodiments, the thicknesses, surfaces and/or bone cuts on corresponding sections of an implant component can be different. Specifically, one or more of the thicknesses, section volumes, bone cut angles, bone cut surface areas, bone cut curvatures, numbers of bone cuts, peg placements, peg angles, and other features may vary between two or more corresponding sections of an implant component. For example, the corresponding medial and lateral sections identified as X and X' of the femoral implant design in FIG. 2A are shown to include different thicknesses, section volumes, bone cut angles, and bone cut surface areas.

In certain embodiments, the bone-facing surface of the implant component can include one or more portions designed to engage resurfaced bone, for example, by having a surface that negatively-matches uncut subchondral bone or cartilage, and one or more portions designed to engage cut bone, for example, by having a surface that negatively-matches a cut subchondral bone.

### 1.2 Joint-facing surface of an implant component

The outer joint-facing surface of a patient-specific implant component can be designed to match the shape of the patient's articular cartilage. For example, it can substantially positively-match the shape of normal or healthy cartilage on the articulation that the component replaces; or it can substantially-negatively match the shape of cartilage on the opposing articular surface in the joint. Corrections can be performed to the shape of diseased cartilage to re-establish a normal or near normal cartilage shape that can then be incorporated into the shape of the joint-facing surface of the component. These corrections can be implemented and, optionally, tested in virtual two-dimensional and three-dimensional models. The corrections and testing can include kinematic analysis, as described below.

In certain embodiments, the joint-facing surface of the patient-specific implant component can be designed to positively-match the shape of subchondral bone. It can include the shape of normal and/or diseased subchondral bone. Corrections can be performed to the shape of subchondral bone to re-establish a normal or near normal articular shape that can be incorporated into the shape of the component's joint-facing surface. A standard thickness can be added to the joint-facing surface. Alternatively, a variable thickness of the can be applied to the component. The variable thickness can be selected to reflect a patient's actual or healthy cartilage thickness, for example, as measured in the individual patient or selected from a standard reference database.

In certain embodiments, the joint-facing surface of the patient-specific implant component can be designed to positively-match a standard shape. For example, the standard shape can have a fixed radius in one or more directions or it can have variable radii in one or more directions. The implant component can have a constant thickness in select areas or it can have a variable thickness in select areas. The standard shape of the joint-facing surface of the component can include, at least in part, the shape of normal and/or diseased subchondral bone or cartilage. Corrections can be performed to the shape of subchondral bone or cartilage to re-establish a normal or near normal articular shape that can then be incorporated into the shape of the joint-facing surface of the component. A standard thickness can be added to the joint facing surface of the component or, alternatively, a variable thickness can be applied to the implant component. The variable thickness can be selected to reflect cartilage thickness, in at least a portion of the component, for example, as measured in the individual patient or selected from a standard reference database.

Certain embodiments, such as those represented schematically in FIG. 1B and FIG. 1C, include, in addition to a first implant component, a second implant component having an opposing joint-facing surface. In such embodiments, the joint-facing surface on the second component can be designed, at least for a portion of its surface, to negatively-match the joint-facing surface of the first component. Designing the joint-facing surface of the second component as a negative-match of the joint-facing surface of the first component can help reduce implant wear. Thus, in some embodiments, the joint-facing surfaces are not anatomic or near-anatomic in shape, but instead negatively-match or nearly negatively-match the joint facing surface of an opposing component at the joint.

Thus, when the joint-facing surface of the first component is designed to positively-match at least a portion of the shape of the patient's cartilage, the opposing joint-facing surface of the second component is, at least in part, a negative-match to the cartilage shape. When the joint-facing surface of the first component is designed to positively-match at least a portion of the shape of a patient's subchondral bone, the opposing joint-facing surface of the second component is, at least in part, a negative-match to the subchondral bone shape. When the joint-facing surface of the first component is designed to positively-match at least a portion of the shape of a patient's cortical bone, the joint-facing surface of the second component is, at least in part, a negative-match to the cortical bone shape. When the joint-facing surface of the first component is designed to positively-match at least a portion of the shape of the endosteal bone, the opposing joint-facing surface of the second component is, at least in part, a negative-match to the endosteal bone shape. When the joint-facing surface of the first component is designed to positively-match at least a portion of the shape of bone marrow, the opposing joint-facing surface of the second component is, at least in part, a negative-match to the bone marrow shape.

The opposing joint-facing surface of the second component can be substantially negatively-matching to the joint-facing surface of the first component in one plane or dimension, in two planes or dimensions, in three planes or dimensions, or in several planes or dimensions. For example, the opposing joint-facing surface of the second component can be substantially negatively-matching to the joint-facing surface of the first component in the coronal plane only, in the sagittal plane only, or in both the coronal and sagittal planes.

In creating the negatively-matching contour on the opposing joint-facing surface of the second component, geometric considerations can improve wear between the first and second components. For example, the radii on the opposing joint-facing surface on the second component can be selected to be slightly larger in one or more dimensions than the radii on the joint-facing surface of the first component.

The opposing bone-facing surface of the second component itself can be designed to negatively-match, at least in part, the shape of articular cartilage, subchondral bone, cortical bone, endosteal bone or bone marrow. It can have any of the features described above for the bone-facing surface of the first component, such as having one or more patient-specific bone cuts.

Many combinations of bone and joint-facing surfaces of the components on first and second articular surfaces are possible. **Table 1** provides illustrative combinations that may be employed.

**TABLE 1: Illustrative Combinations of Implant Components**

| **1^{st} component bone-facing surface** | **1^{st} component joint-facing surface** | **1^{st} component bone cut(s)** | **2^{nd} component joint facing surface** | **2^{nd} component bone facing surface** | **2^{nd} component bone cuts** |
|---|---|---|---|---|---|
| **Example: Femur** | Example: Femur | Example: Femur | Example: Tibia | Example: Tibia | Example: Tibia |
| **At least one bone cut** | Cartilage | Yes | Negative-match of 1^{st} component joint-facing (opposing cartilage) | At least one bone cut | Yes |
| **At least one bone cut** | Cartilage | Yes | Negative-match of 1^{st} component joint-facing (opposing cartilage) | Subchondral bone | Optional |
| **At least one bone cut** | Cartilage | Yes | Negative-match of 1^{st} component joint-facing (opposing cartilage) | Cartilage (same side, e.g. tibia) | Optional |
| **At least one bone cut** | Subchondral bone | Yes | Negative-match of 1^{st} component joint-facing (opposing subchondral bone) | At least one bone cut | Yes |
| **At least one bone cut** | Subchondral bone | Yes | Negative-match of 1^{st} component joint-facing (opposing subchondral bone) | Subchondral bone | Optional |
| **At least one bone cut** | Subchondral bone | Yes | Negative-match of 1^{st} component joint-facing (opposing subchondral bone) | Cartilage (same side, e.g. tibia) | Optional |
| | | | | | |
| **Subchondral bone** | Cartilage | Optional | Negative-match of 1^{st} component joint-facing (opposing cartilage) | At least one bone cut | Yes |
| **Subchondral bone** | Cartilage | Optional | Negative-match of 1^{st} component joint-facing (opposing cartilage) | Subchondral bone | Optional |
| **Subchondral bone** | Cartilage | Optional | Negative-match of 1^{st} component joint-facing (opposing cartilage) | Cartilage (same side, e.g. tibia) | Optional |
| **Subchondral bone** | Subchondral bone | Optional | Negative-match of 1^{st} component joint-facing (opposing subchondral bone) | At least one bone cut | Yes |
| **Subchondral bone** | Subchondral bone | Optional | Negative-match of 1^{st} component joint-facing (opposing subchondral bone) | Subchondral bone | Optional |
| **Subchondral bone** | Subchondral bone | Optional | Negative-match of 1^{st} component joint-facing (opposing subchondral bone) | Cartilage (same side, e.g. tibia) | Optional |
| | | | | | |
| **Subchondral bone** | Standard / Model | Optional | Negative-match of 1^{st} component joint-facing standard | At least one bone cut | Yes |
| **Subchondral bone** | Standard / Model | Optional | Negative-match of 1^{st} component joint-facing standard | Subchondral bone | Optional |
| **Subchondral bone** | Standard / Model | Optional | Negative-match of 1^{st} component joint-facing standard | Cartilage (same side, e.g. tibia) | Optional |
| | | | | | |
| **Subchondral bone** | Subchondral bone | Optional | Non-matching standard surface | At least one bone cut | Yes |
| **Subchondral bone** | Cartilage | Optional | Non-matching standard surface | At least one bone cut | Yes |

### 1.3 Multi-component implants and implant systems

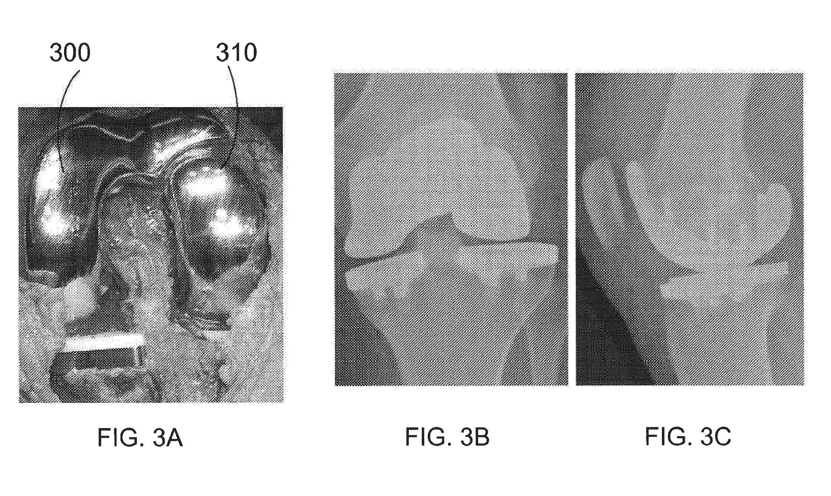

The disclosed implants and implant systems can include any number of patient-specific implant components and any number of non-patient-specific implant components. An exemplary implant or implant system is depicted in **FIGS. 3A - 3C****.** Specifically, **FIG. 3A** shows a photograph of a patient-specific total knee replacement implant system that includes a patient-specific bicompartmental implant component **300** and patient-specific unicompartmental implant component **310.** Both components are patient specific on both their bone-facing surfaces and on their joint-facing surfaces. **FIGS. 3B** and **3C** are x-ray images showing the implant of **FIG. 3A** in the coronal plane **(****FIG. 3B****)** and the sagittal plane **(****FIG. 3C****).**

In certain embodiments, the implants and implant systems can include a combination of implant components, such as a traditional unicompartmental device with a patient-specific bicompartmental device or a combination of a patient-specific unicompartmental device with standard bicompartmental device. Such implant combinations allow for a flexible design of an implant or implant system that includes both standard and patient-specific features and components. This flexibility and level of patient-specificity allows for various optimizations, such as retention of all ligaments and/or restoration of normal or near-normal patient kinematics.

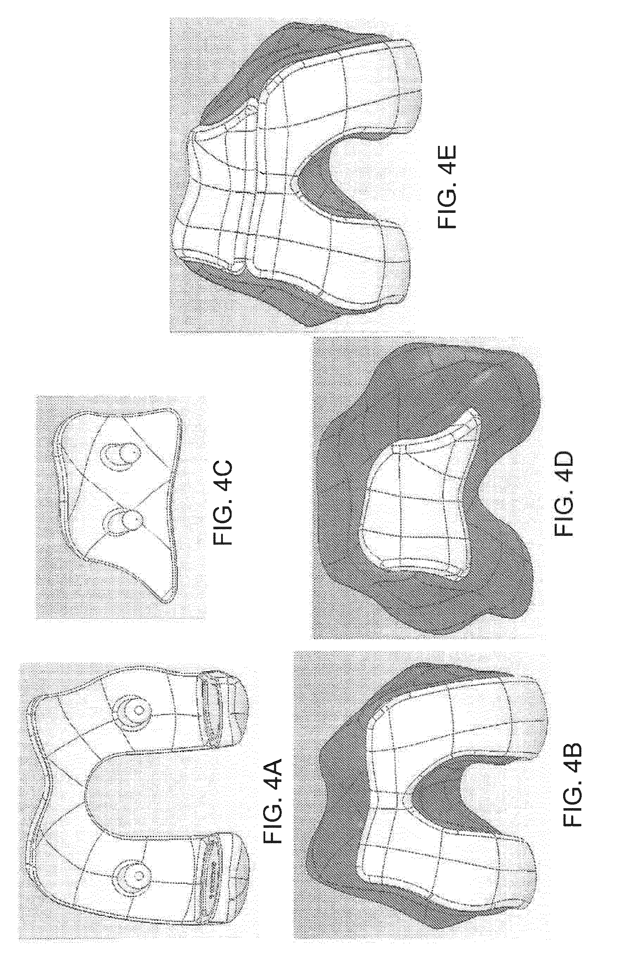

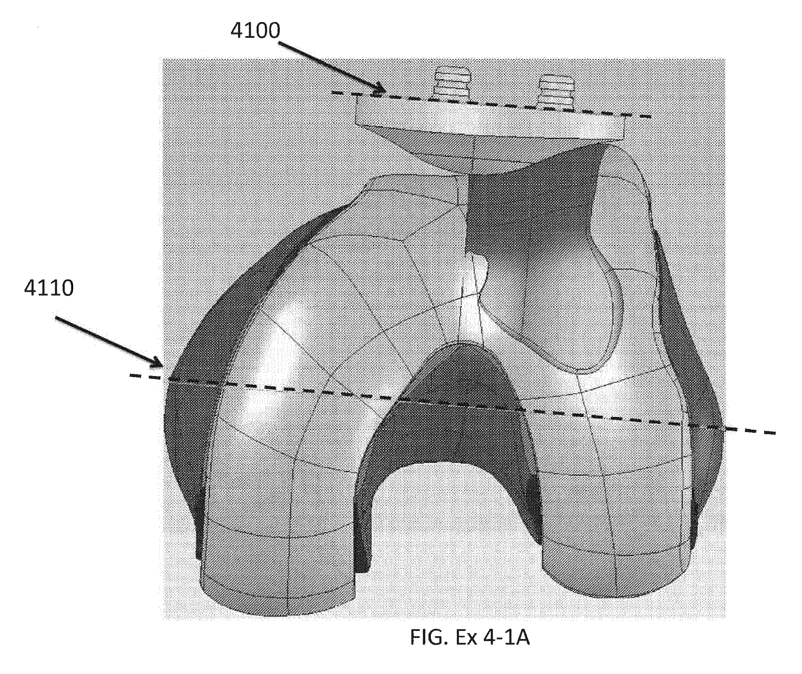

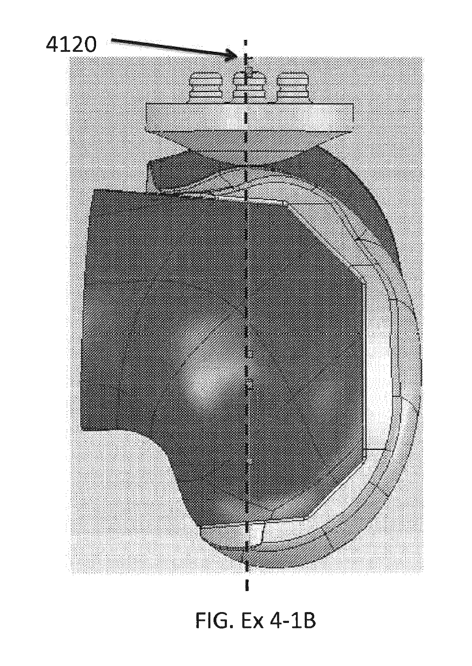

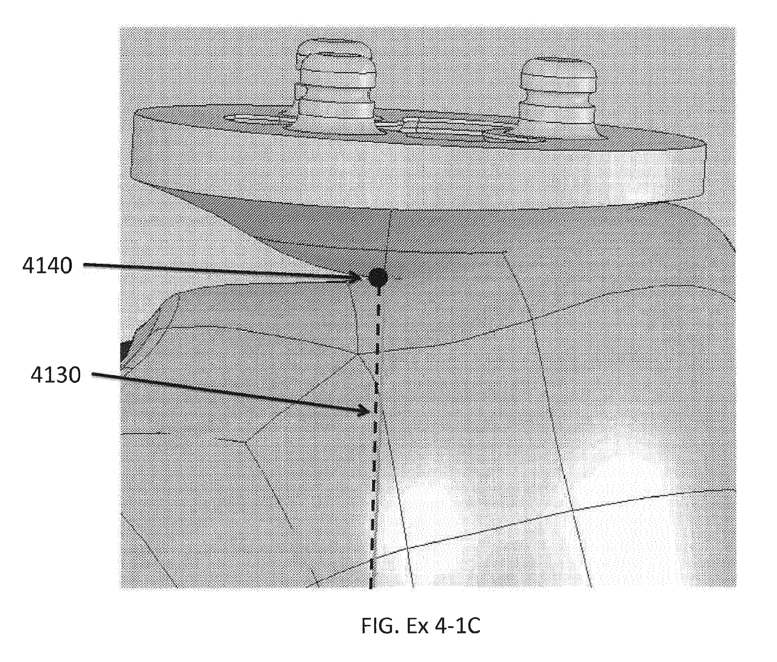

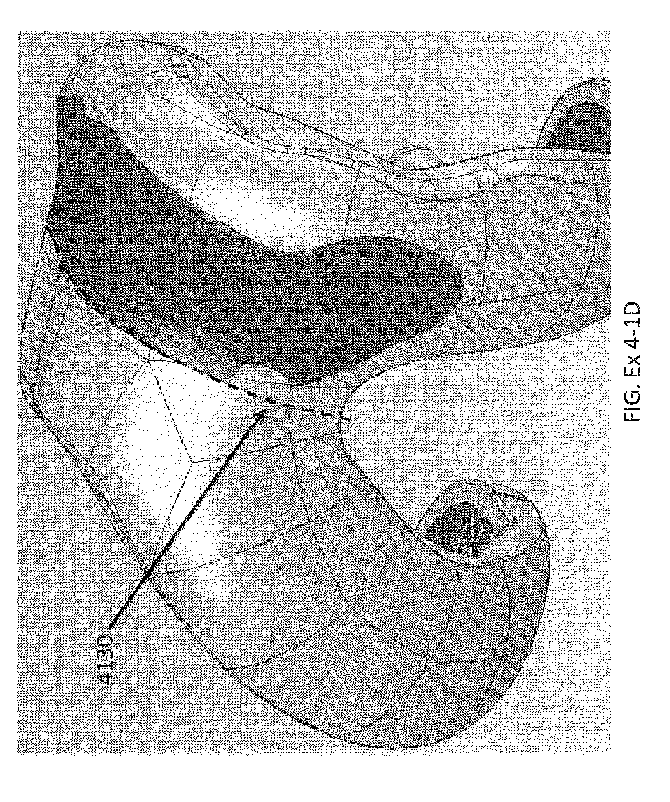

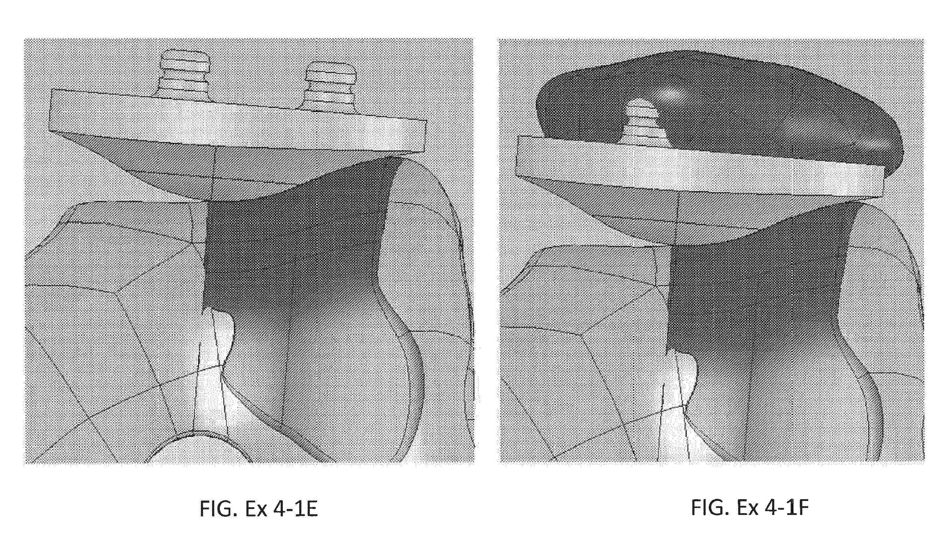

In certain embodiments, an implant component is designed and installed as one or more pieces. For example, **FIGS. 4A****-4E** show an exemplary design of a femoral implant component that can be installed in two pieces.

Embodiments described herein can be applied to partial or total joint replacement systems. Bone cuts or changes to an articular surface described herein can be applied to a portion of an articular surface, to an entire articular surface, or to multiple articular surfaces. Thus, for example, certain embodiments include partial knee replacement, such as patellofemoral knee replacements, unicompartmental knee replacements, bicompartmental knee replacements and total knee replacements. Moreover, embodiments described herein can be applied to hemiarthroplasty systems, for example, femoral hemiarthroplasty in a hip joint, cup arthroplasty in hip joint, or tibial hemiarthroplasty.

### 2. Collecting and using patient data to design and make a patient-specific implant

As mentioned above, in some embodiments the implants are designed and made using patient-specific data that is collected preoperatively. The patient-specific data can include points, surfaces, and/or landmarks, collectively referred to herein as "reference points." In certain embodiments, the reference points are selected and used to derive a varied or altered surface, such as, without limitation, an ideal surface or structure. For example, the reference points can be used to create patient-specific implants having at least one patient-specific surface, dimension, or aspect. Alternatively, or in addition, the reference points can be used to create at least one patient-optimized surface, dimension, or aspect of an implant.

Sets of reference points can be grouped to form reference structures used to create a model of a joint and/or an implant design. Designed implant surfaces can be derived from single reference points, triangles, polygons, or more complex surfaces or models of joint material, such as, for example, articular cartilage, subchondral bone, cortical bone, endosteal bone or bone marrow. Various reference points and reference structures can be selected and manipulated to derive a varied or altered surface, such as, without limitation, an ideal surface or structure.

The reference points can be located on or in the joint that will receive the patient-specific implant. For example, the reference points can include weight-bearing surfaces or locations in or on the joint, a cortex in the joint, or an endosteal surface of the joint. The reference points also can include surfaces or locations outside of but related to the joint. Specifically, reference points can include surfaces or locations functionally related to the joint. For example, in embodiments directed to the knee joint, reference points can include one or more locations ranging from the hip down to the ankle or foot. The reference points also can include surfaces or locations homologous to the joint receiving the implant. For example, in embodiments directed to a knee, a hip, or a shoulder joint, reference points can include one or more surfaces or locations from the corresponding knee, hip, or shoulder joint.

### 2.1 Variations to biological surfaces at the joint

In certain embodiments, the reference points can be processed using mathematical functions to derive virtual, corrected surfaces, which may represent an ideal or desired surface from which a patient-specific implant can be designed. For example, one or more surfaces or dimensions of a biological structure can be modeled, altered, added to, changed, deformed, eliminated, corrected and/or otherwise manipulated (collectively referred to herein as "variation" of an existing surface or structure within the joint).

Variation of the joint or portions of the joint can include, without limitation, variation of one or more of external surfaces, internal surfaces, joint facing surfaces, uncut surfaces, cut surfaces, altered surfaces, and/or partial surfaces as well as osteophytes, subchondral cysts, geodes or areas of eburnation, joint flattening, contour irregularity, and loss of normal shape. The surface or structure can be or reflect any surface or structure in the joint, including, without limitation, bone surfaces, ridges, plateaus, cartilage surfaces, ligament surfaces, or other surfaces or structures. The surface or structure derived can be an approximation of a healthy joint surface or structure or can be another variation. The surface or structure can be made to include pathological alterations of the joint. The surface or structure also can be made whereby the pathological joint changes are virtually removed in whole or in part.

Once one or more reference points, structures, surfaces, models, or combinations thereof have been selected or derived, the resultant shape can be varied, deformed or corrected. In some embodiments, the variation can be designed to derive an ideal implant shape. In one application of this embodiment, the preferred implant shape is similar to the joint of the patient before he or she developed the arthritis.

The variation can include additional alterations to the joint, such as the virtual removal of osteophytes or the virtual building of structural support deemed beneficial to a final outcome for a patient.

### 2.1.1 Variations to address osteophytes

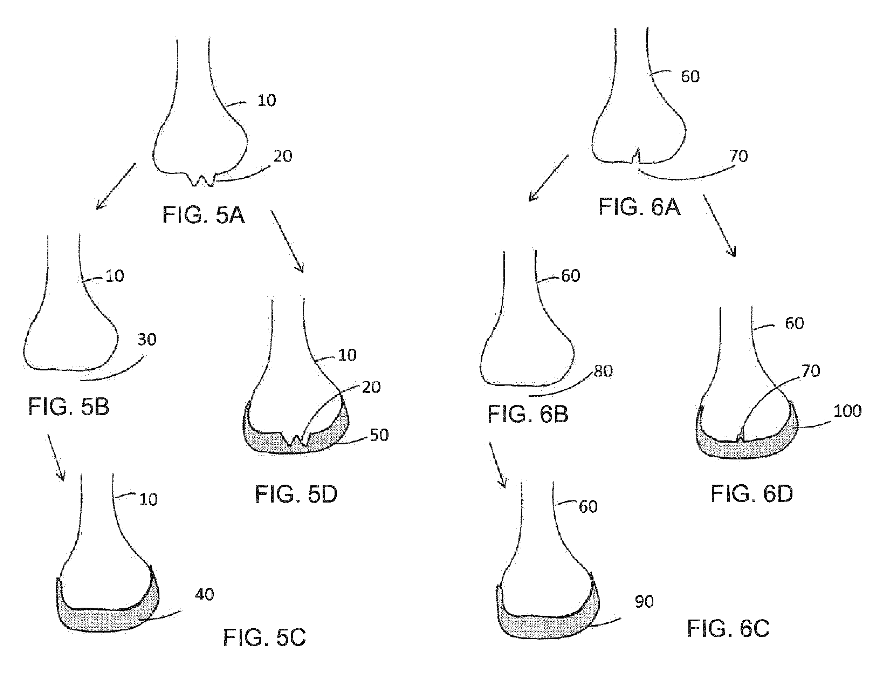

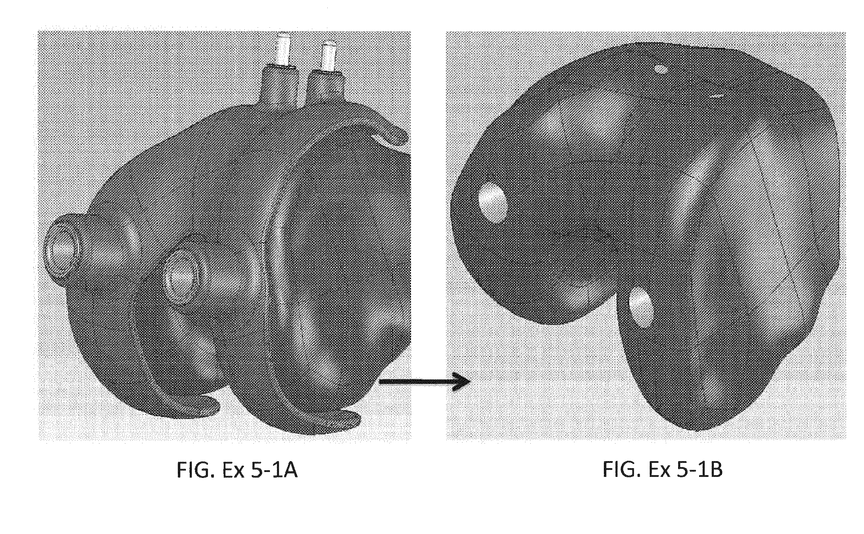

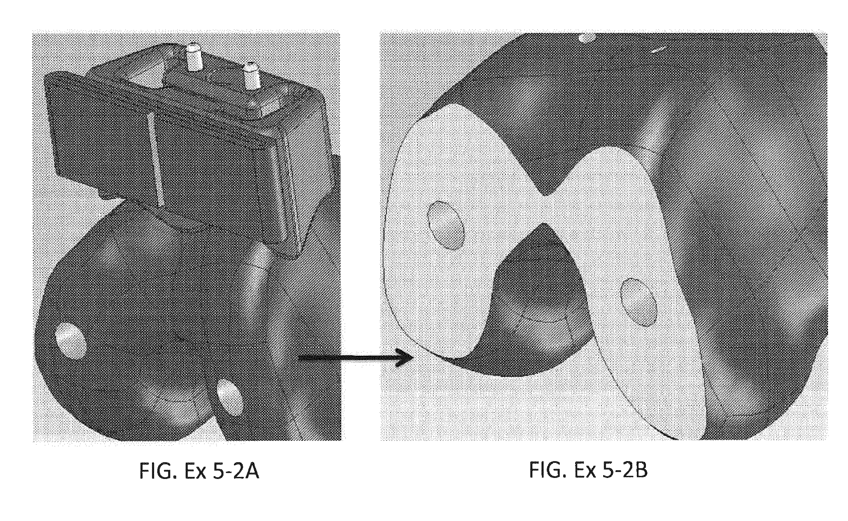

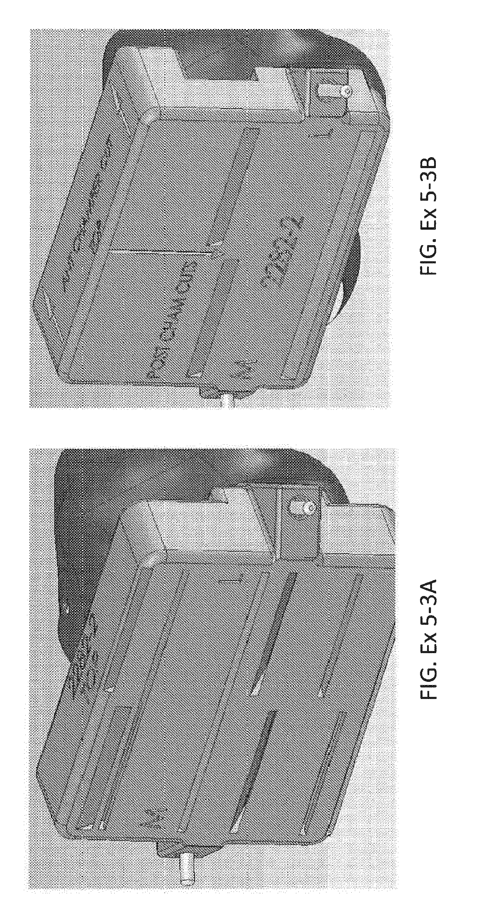

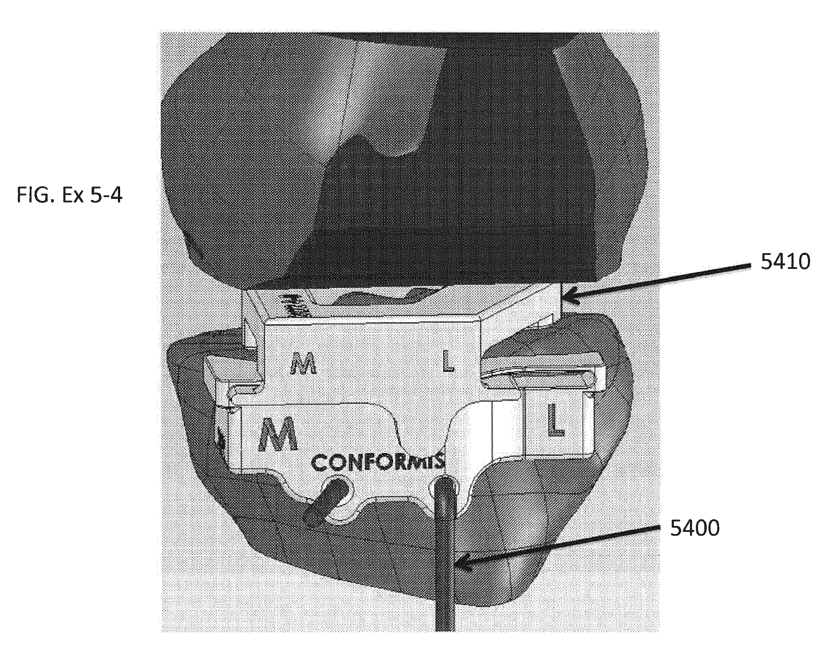

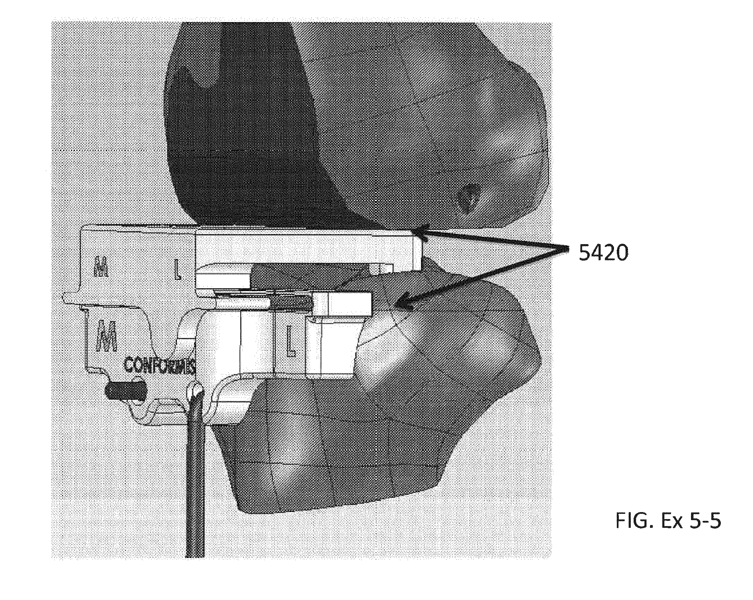

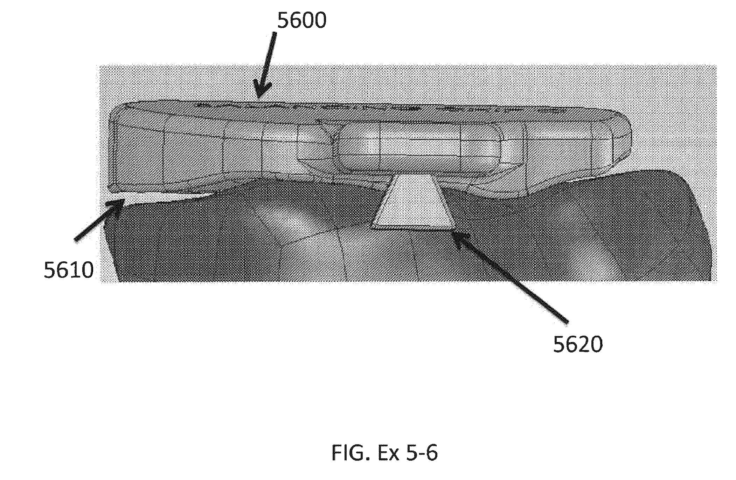

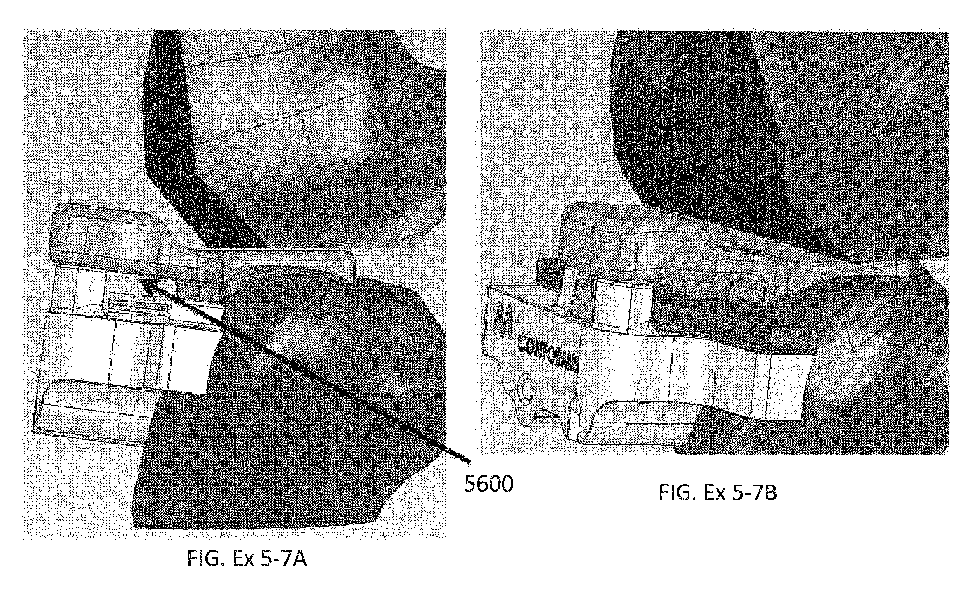

In the case of removing the osteophytes, the bone-facing surface of the implant then is derived after the osteophyte has been virtually removed. Alternatively, the osteophyte can be integrated in the shape of the bone facing surface of the implant. For example, **FIGS. 5A****-5D** are drawings of an end of a femur **10** having an osteophyte **20.** During development of an implant, the image can be transformed such that the osteophyte **20** is virtually removed as shown in **FIG. 5B** at removed osteophyte **30** to produce, as shown in **FIG. 5C****,** an implant **40** based on a smooth surface at the end of femur **10.** Alternatively, as shown in **FIG. 5D****,** an implant **50** can be developed to conform to the shape of osteophyte **20.** In the case of building additional or improved structure, the bone-facing surface of the implant then is derived after the additional structure is modeled.

### 2.1.2 Variations to address subchondral voids

A subchondral void can be integrated in the shape of the bone-facing surface of the implant. For example, **FIGS. 6A****-6D** are drawings of an end of a femur **60** having a subchondral void **70.** During development of an implant, the image can be transformed such that the void **70** is virtually removed as shown in **FIG. 6B** at removed void **80** to produce, as shown in **FIG. 6C****,** an implant **90** based on a smooth surface at the end of femur **60.** Alternatively, implant **100** can be developed to conform to the shape of void **70,** as shown in **FIG. 6D****.** Note that, while virtually conforming to void **70,** implant **100** may not practically be able to be inserted into the void. Therefore, in an alternate embodiment, the implant may only partially protrude into a void in the bone.

### 2.1.3 Variations to address other patient-specific defects or phenomena

In another embodiment, a correction can include the virtual removal of subchondral cysts. The bone-facing surface of the implant is then derived after the subchondral cyst has been virtually removed.

In another embodiment, a correction can include the virtual removal of articular defects. The bone facing surface of the implant is then derived after the articular defect has been virtually removed. In this embodiment, the defect may then be filled intraoperatively with bone cement, bone graft or other bone fillers. Alternatively, the articular defect can be integrated in the shape of the bone facing surface of the implant.

The variation can include the virtual removal of flattening of a rounded articular surface. The joint facing and/or the bone facing surface of the implant then can be derived after the flattening has been virtually corrected. This correction can, for example, be designed to re-establish a near normal shape. Alternatively, the correction can be designed to establish a standardized shape or surface. Alternatively, the flattening can be integrated in the shape of the bone facing surface of the implant. In this case, the joint-facing surface of the articular implant can be designed to re-establish a near normal anatomic shape reflecting, for example, at least in part the shape of normal cartilage or subchondral bone. Alternatively, it can be designed to establish a standardized shape.

### 2.2 Determining articular dimensions

In certain embodiments, an imaging test, for example, x-ray imaging, digital tomosynthesis, cone beam CT, non-spiral or spiral CT, non-isotropic or isotropic MRI, SPECT, PET, ultrasound, laser imaging, photo-acoustic imaging, is used to determine articular dimensions and/or shape in two or three dimensions. Determining articular dimensions and/or shape can include determining articular dimensions and/or shape for one or more of normal cartilage, diseased cartilage, a cartilage defect, an area of denuded cartilage, subchondral bone, cortical bone, endosteal bone, bone marrow, a ligament, a ligament attachment or origin, menisci, labrum, a joint capsule, or articular structures. Determining dimensions can include determining shape, curvature, size, area, thickness, and/or volume.

### 2.2.1 Blanks, sizing, and library options

Using these articular dimensions and, optionally, other data, a patient-specific implant component can be designed and manufactured to have matching, patient-specific articular dimensions. Alternatively, these patient-specific articular dimensions can be used to select an implant from a selection, for example, small, medium, or large, of blank implants, or from a library of implants. The selected blank implant or the selected library implant then can be tailored to include patient-specific features.

### 2.3 Determining limb alignment

Proper joint and limb function depend on correct limb alignment. For example, in repairing a knee joint with one or more knee implant components, optimal functioning of the new knee depends on the correct alignment of the anatomical and/or mechanical axes of the lower extremity. Accordingly, an important consideration in designing and/or replacing a natural joint with one or more implant components is proper limb alignment or, when the malfunctioning joint contributes to a misalignment, proper realignment of the limb.

Some embodiments include collecting and using data from imaging tests to virtually determine in one or more planes one or more of an anatomic axis and a mechanical axis and the related misalignment of a patient's limb. The misalignment of a limb joint relative to the axis can identify the degree of deformity, for example, varus or valgus deformity in the coronal plane or genu antecurvatum or recurvatum deformity in the sagittal plane. Then, one or more of the patient-specific implant components and/or the implant procedure steps, such as bone resection, can be designed to help correct the misalignment.

The imaging tests that can be used to virtually determine a patient's axis and misalignment can include one or more of such as x-ray imaging, digital tomosynthesis, cone beam CT, non-spiral or spiral CT, non-isotropic or isotropic MRI, SPECT, PET, ultrasound, laser imaging, and photoacoustic imaging, including studies utilizing contrast agents. Data from these tests can be used to determine anatomic reference points or limb alignment, including alignment angles within the same and between different joints or to simulate normal limb alignment. Any anatomic features related to the misalignment can be selected and imaged. For example, in certain embodiments, such as for a knee or hip implant, the imaging test can include data from at least one of, or several of, a hip joint, knee joint and ankle joint. The imaging test can be obtained in lying, prone, supine or standing position. The imaging test can include only the target joint, or both the target joint and also selected data through one or more adjoining joints.

Using the image data, one or more mechanical or anatomical axes, angles, planes or combinations thereof can be determined. In certain embodiments, such axes, angles, and/or planes can include, or be derived from, one or more of a Whiteside's line, Blumensaat's line, transepicondylar line, femoral shaft axis, femoral neck axis, acetabular angle, lines tangent to the superior and inferior acetabular margin, lines tangent to the anterior or posterior acetabular margin, femoral shaft axis, tibial shaft axis, transmalleolar axis, posterior condylar line, tangent(s) to the trochlea of the knee joint, tangents to the medial or lateral patellar facet, lines tangent or perpendicular to the medial and lateral posterior condyles, lines tangent or perpendicular to a central weight-bearing zone of the medial and lateral femoral condyles, lines transecting the medial and lateral posterior condyles, for example through their respective centerpoints, lines tangent or perpendicular to the tibial tuberosity, lines vertical or at an angle to any of the aforementioned lines, and/or lines tangent to or intersecting the cortical bone of any bone adjacent to or enclosed in a joint. Moreover, estimating a mechanical axis, an angle, or plane also can be performed using image data obtained through two or more joints, such as the knee and ankle joint, for example, by using the femoral shaft axis and a centerpoint or other point in the ankle, such as a point between the malleoli.

As one example, if surgery of the knee or hip is contemplated, the imaging test can include acquiring data through at least one of, or several of, a hip joint, knee joint or ankle joint. As another example, if surgery of the knee joint is contemplated, a mechanical axis can be determined. For example, the centerpoint of the hip knee and ankle can be determined. By connecting the centerpoint of the hip with that of the ankle, a mechanical axis can be determined in the coronal plane. The position of the knee relative to said mechanical axis can be a reflection of the degree of varus or valgus deformity. The same determinations can be made in the sagittal plane, for example to determine the degree of genu antecurvatum or rccurvatum. Similarly, any of these determinations can be made in any other desired planes, in two or three dimensions.

### 2.3.1 Virtual limb alignment for designing a knee implant and implant procedure

From a three-dimensional perspective, the lower extremity of the body ideally functions within a single plane known as the median anterior-posterior plane (MAP-plane) throughout the flexion-extension arc. In order to accomplish this, the femoral head, the mechanical axis of the femur, the patellar groove, the intercondylar notch, the patellar articular crest, the tibia and the ankle remain within the MAP-plane during the flexion-extension movement. During movement, the tibia rotates as the knee flexes and extends in the epicondylar axis, which is perpendicular to the MAP-plane.

As shown in **FIG. 6-1****,** the mechanical axis of a patient's lower extremity can be defined by the center of hip **1902** (located at the head **1930** of the femur **1932**), the center of the knee **1904** (located at the notch where the intercondylar tubercle **1934** of the tibia **1936** meet the femur) and the center of the ankle **1906.** In the figure, the long axis of the tibia **1936** is collinear with the mechanical axis of the lower extremity **1910.** The anatomic axis **1920** aligns 5-7 degrees offset θ from the mechanical axis in the valgus, or outward, direction. A variety of image slices can be taken at each joint, for example, at one or more of the knee joint **1950,** the hip joint **1952,** and the ankle joint, to determine the mechanical centerpoint for each joint.

In certain preferred embodiments, anatomic reference points are used to virtually determine a patient's misalignment and the proper mechanical axis of his or her lower extremity. Based on the difference between the patient's misalignment and the proper mechanical axis, a knee implant and implant procedure is virtually designed to include implant and/or resection dimensions that substantially realign the patient's limb to have a proper mechanical axis. The implant design process can include manufacturing the implant (e.g., using CAM software) and, optionally, the implant can be surgically implanted into the patient according to the virtually designed procedure.

In certain embodiments, a patient's proper mechanical axis of the lower extremity, and the extent of misalignment of the extremity, is virtually determined using an appropriate computer-aided design software program, such as SolidWorks software (Dassault Systèmes SolidWorks Corp., 300 Baker Avenue, Concord, MA 01742). Using the software, patient-specific information, for example, a collection of anatomic reference points, is used to generate a virtual model that includes the patient's knee joint.

The virtual model also can include reference points from the hip and/or ankle joints. Using the virtual model, a user can determine virtually the misalignment of and mechanical axis of the patient's lower extremity by determining in the model the patient's tibial mechanical axis, femoral mechanical axis, and one or more planes from each axis. For example, the patient's tibial mechanical axis can be determined virtually in the model as a line connecting the center of the patient's ankle and the center of the patient's tibia. The patient's femoral mechanical axis can be determined virtually in the model as a line connecting the center of the patient's hip and the center of the patient's distal femur. The center of the patient's ankle, tibia, hip, and/or distal femur can be determined based on the patient-specific anatomic reference points or landmarks used to generate the virtual model.

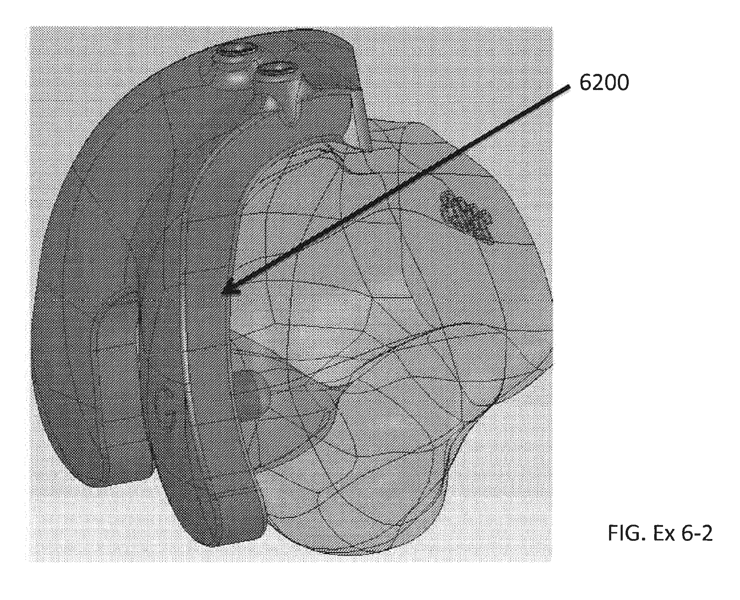

Then, the user can align virtually the lower extremity by collinearly aligning the tibial and femoral mechanical axes. This collinear alignment can be achieved by adjusting the angle of the intersecting axes at the knee joint to be zero. The axes can be aligned axially by aligning one or more planes common to both axes, such as the sagittal or coronal planes. **FIGS. 6**-**2A** - **6-2C** each illustrate a model showing the existing misalignment of a patient's lower extremity (gray and solid line) and the virtual alignment (white and dashed line) determined using the model.

Exemplary methods for determining the tibial mechanical axis, the femoral mechanical axis, and the sagittal and coronal planes for each axis are described in more detail in the following subsections.

### 2.3.2 Tibial mechanical axis and its sagittal and coronal planes

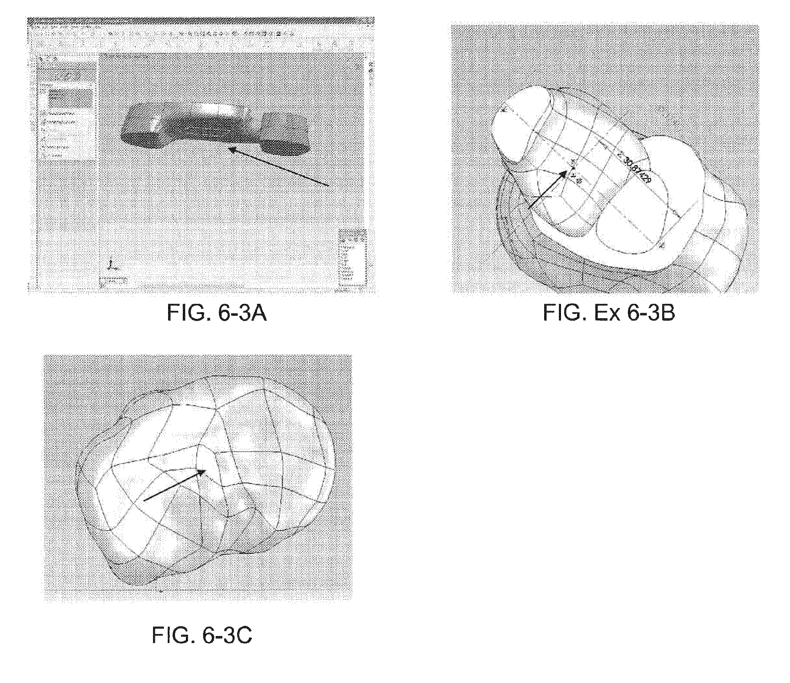

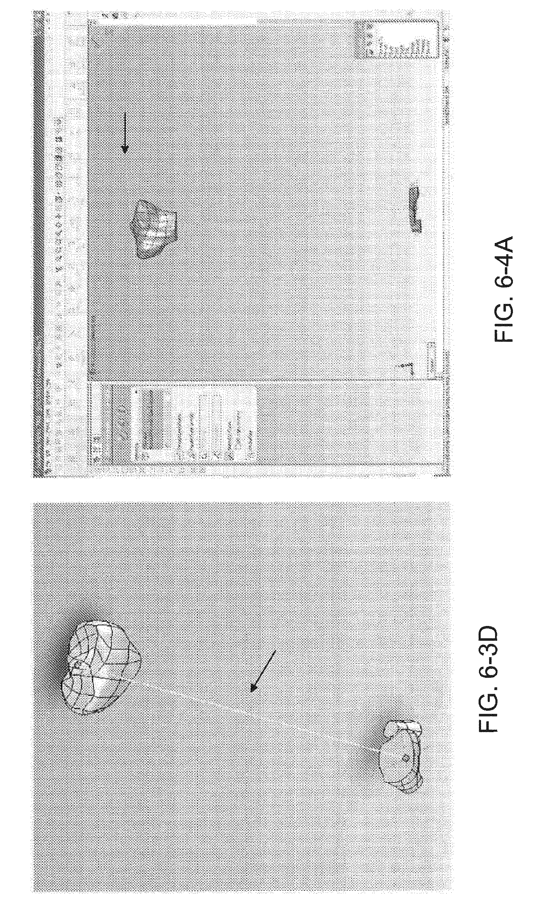

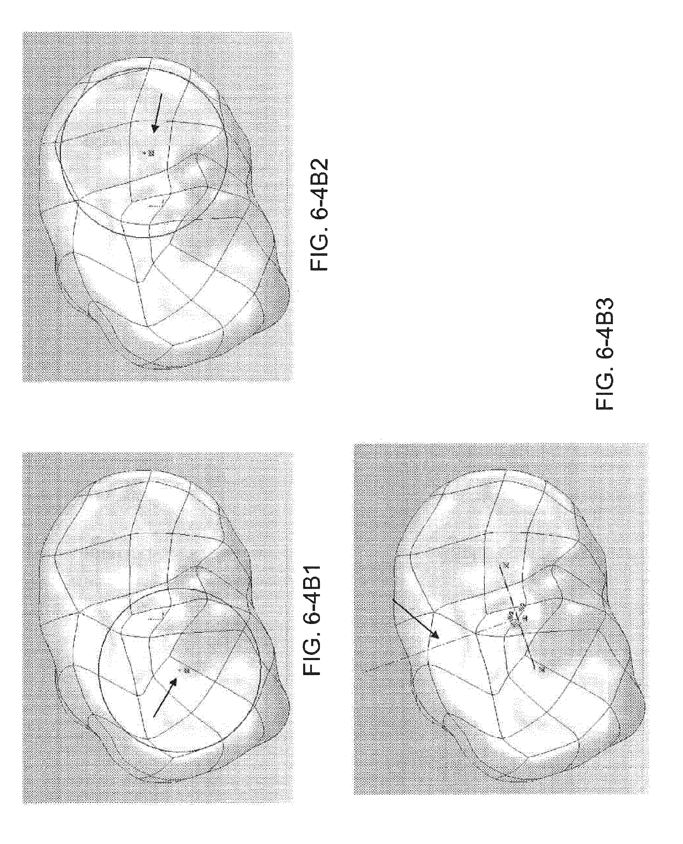

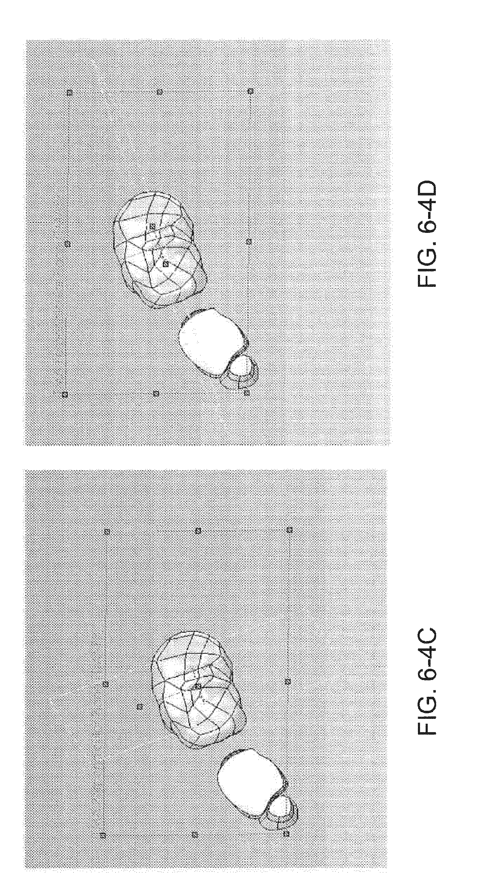

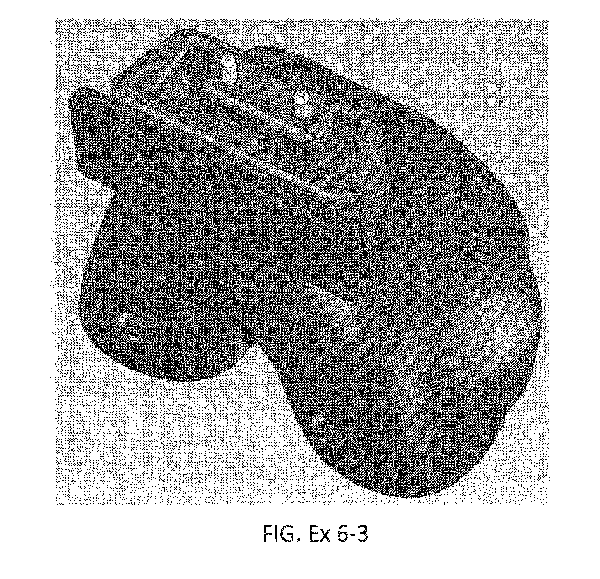

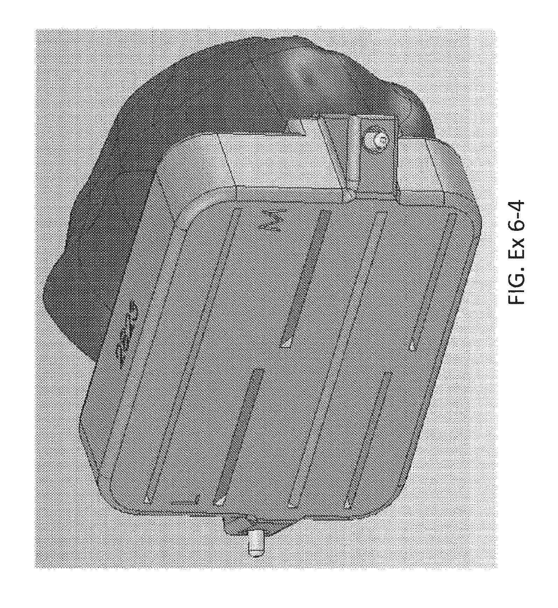

In certain embodiments, the tibial mechanical axis and the tibial sagittal and coronal planes are determined virtually using a model that includes reference points from a patient's knee and ankle joints, as follows:
1. *Tibial mechanical axis*.
   1a. *Axial plane of the ankle*. As shown in **FIG. 6-3A****,** an axial plane at the ankle is identified using three or more points at the inferior articular surface of the tibia. The three or more points are selected from the same or closely similar elevation(s) on the inferior articular surface of the tibia. This optional step can be used to establish an initial plane of reference for subsequent virtual determinations.
   1b. *Distal point of the tibial mechanical axis.* The distal point of the patient's tibial mechanical axis can be defined as the center of the ankle. As shown in **FIG. 6-****3B,** the center of the ankle can be determined virtually by connecting a line from the medial to the lateral malleoli and marking 4 percent medial from the center of the line. For example, if the distance between the malleoli is 100, then the center of the line is at 50 and the center of the ankle is 4 percent medial from the center of the line or, in other words, at 46 from the medial malleoli and 54 from the lateral malleoli.
   1c. *Proximal point of the tibial mechanical axis.* The proximal point of the tibial mechanical axis can be determined virtually as the posterior aspect of the ACL insertion point, as shown in **FIG. 6-3C****.**
   1d. *Tibial mechanical axis.* The tibial mechanical axis can be determined virtually as the line connecting the distal and the proximal points of the tibial mechanical axis, as shown in **FIG. 6-3D****.**
2. *Sagittal or A-P plane of the tibia.*
   2a. *Tibial axis perpendicular plane ("TAPP").* The TAPP can be determined virtually as the plane perpendicular to the tibial mechanical axis line and including the proximal point of the tibial mechanical axis, as shown in **FIG. 6-4A****.** This optional step can be used to establish a plane of reference for subsequent virtual determinations. The TAPP, optionally tilted in an A-P orientation, also can be used to determine the tibial cut line.
   2b *A-P line of the tibia - derived from Cobb method.* The A-P line of the tibia can be determined virtually based on method derived from Cobb et al. (2008) "The anatomical tibial axis: reliable rotational orientation in knee replacement" J Bone Joint Surg Br. 90(8):1032-8. Specifically, the A-P line of the tibia can be determined virtually as the line perpendicular to the line connecting the diametric centers of the lateral and medial condyles of the tibia. For example, as shown in **FIG. 6-4B1** and **6-4B2,** a best-fit circle can be sketched to determine the diametric center of the lateral condyle (i.e., the lateral plateau of the tibia). In addition, a best-fit circle can be sketched to determine the diametric center of the medial condyle (i.e., the medial plateau of the tibia).
      In certain embodiments, one or both of the circles can be sketched to best fit the corresponding condyle(s) at the superior articular surface of the tibia. Alternatively, one or both of the circles can be sketched to best fit a portion of the wear pattern at the superior articular surface of the tibia. Still yet, one or both of the circles can be sketched to best fit the condyle(s) at a certain distance distal to the superior articular surface of the tibia. For example, the circle for the medial condyle can be sketched to best fit the medial condyle at 10 mm, 15 mm, 20 mm, 25 mm or more below, or distal to, the superior articular surface of the tibia; and then the circle can be adjusted proximally to lie on the plane of the superior articular surface of the tibia.
      Then, as shown in **FIG. 6-4B3****,** A-P line of the tibia is determined virtually as the line perpendicular to, and including the midpoint of, the line connecting the diametric centers of the lateral and medial condyles of the tibia. If the midpoint of the line connecting the diametric centers of the lateral and medial condyles is not in the same location as the proximal point of the tibial mechanical axis, then the A-P line can be shifted away from the midpoint to include the proximal point of the tibial mechanical axis while remaining perpendicular to the line connecting the diametric centers of the lateral and medial condyles.
      *A-P line of the tibia - derived from Agaki method.* An alternative method for determining virtually the A-P line can be derived from other published methods, such as Agaki (2004) "An Anteroposterior Axis of the Tibia for Total Knee Arthroplasty," Clin Orthop 420: 213-219.
   2c. *Sagittal or A-P plane of the tibia.* As shown in **FIG. 6-4C****,** the sagittal or A-P plane of the tibia can be determined virtually as the plane including both the A-P line of the tibia and the tibial mechanical axis line. The sagittal or A-P plane also is perpendicular to the TAPP.
3. *Coronal or medial-lateral ("M-L") plane of the tibia.* As shown in **FIG. 6-4D****,** the coronal or M-L plane of the tibia can be determined virtually as the plane perpendicular to the A-P plane (or perpendicular to the A-P line) of the tibia and including the tibial mechanical axis line. The coronal or M-L plane also is perpendicular to the TAPP.

### 2.3.3 Femoral mechanical axis and its sagittal and coronal planes

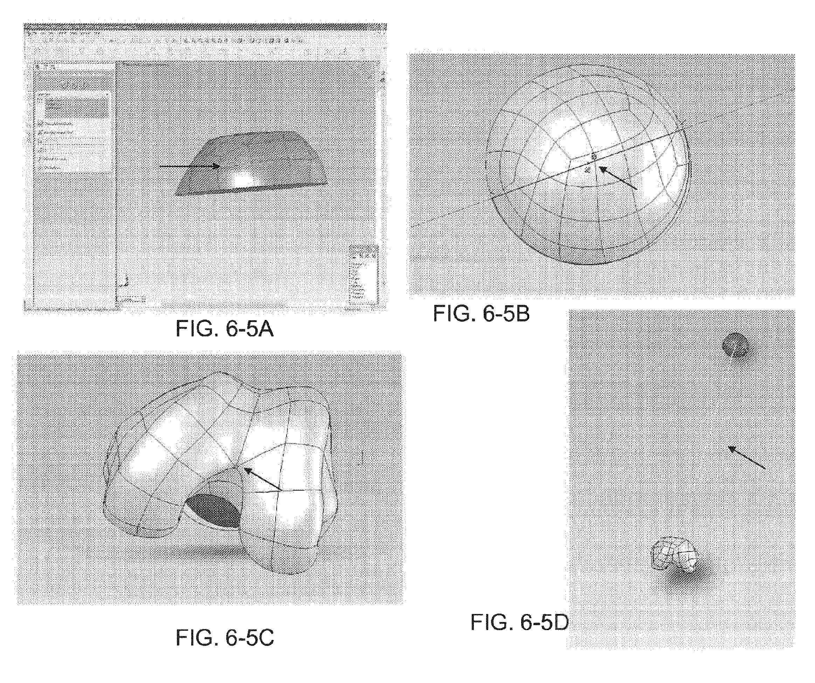

In certain embodiments, the femoral mechanical axis and the femoral sagittal and coronal planes are determined virtually using a model that includes reference points from a patient's knee and hip joints, as follows:
1. *Femoral mechanical axis.*
   1a. *Axial plane of the femur.* As shown in **FIG. 6-5A****,** an axial plane of the femur is selected virtually using three or more points within the spherical femoral head that substantially lie in the same axial plane. This optional step can be used to establish an initial plane of reference for subsequent virtual determinations.
   1b. *Proximal point of the femoral mechanical axis.* As shown in **FIG. 6-5B****,** the proximal point of the patient's femoral mechanical axis can be determined virtually as the center of the spherical femoral head.
   1c. *Distal point of the femoral mechanical axis*. As shown in **FIG. 6-5C****,** the distal point of the femoral mechanical axis is determined virtually as the point at the posterior aspect of the femoral trochlear notch.
   1d. *Femoral mechanical axis.* The femoral mechanical axis can be determined virtually as the line connecting the distal and the proximal points of the femoral mechanical axis, as shown in **FIG. 6-5D****.**
2. *Sagittal or A-P plane of the femur.*
   2a. *Femoral mechanical axis perpendicular plane (FMAPP).* The FMAPP can be determined virtually as a plane perpendicular to the femoral mechanical axis line and including the distal point of the femoral mechanical axis, as shown in **FIG. 6-****6A.** This optional step can be used to establish a plane of reference for subsequent virtual determinations. In certain embodiments of implant procedures that require femoral cuts, the distal femoral cut is applied at the FMAPP.
   2b. *A-P line of the femur - derived from Whiteside's line.* As shown in **FIG. 6-6B****,** the A-P line of the femur can be determined virtually as the line perpendicular to the epicondylar line and passing through distal point of the femoral mechanical axis. The epicondylar line is the line connecting medial and lateral epicondyles (furthest out points).
   2c. *Sagittal or A-P plane of the femur.* As shown in **FIG. 6-6C****,** the sagittal or A-P plane of the femur can be determined virtually as the plane including both the A-P line of the femur (derived from the Whiteside's line) and the femoral mechanical axis line. The sagittal or A-P plane also is perpendicular to the plane perpendicular to the femoral axis.
3. *Coronal or medial*-*lateral ("M-L") plane of the femur.* As shown in **FIG. 6-6D****,** the coronal or M-L plane of the femur can be determined virtually as the plane perpendicular to the A-P plane (or perpendicular to the A-P line) of the femur and including the femoral mechanical axis line. The coronal or M-L plane also is perpendicular to the plane perpendicular to the femoral axis.

After determining virtually the tibial and femoral mechanical axis, and their sagittal and coronal planes, the lower extremity can be aligned virtually by adjusting the angle of the intersecting mechanical axes at the knee joint to be zero. The axes can be aligned axially by aligning one or both of the sagittal or coronal planes from each axis, as shown in **FIGS. 6-6E** and **6-6F,** respectively. **FIGS. 6-7A** and **6-7B** show a model before and after virtual alignment as it appears in axial view looking distally from a section of the femoral head, to a section of the distal femur, and on to a section of the tibia. Similarly, **FIGS. 6-7C** and **6-7D** show a model before and after virtual alignment as it appears in axial view looking proximally from a section of the distal tibia, to a section of the distal femur, and in **FIG. 6-7C****,** on to a section of the femoral head. **FIGS. 6-7E****-7G** show a model before and after virtual alignment (**FIGS. 6-7E** and **G**), and overlay of both before and after virtual alignment **(****FIG. 6-7F****).**

### 2.4 Estimating deformity

Cartilage loss in one compartment can lead to progressive joint deformity. For example, cartilage loss in a medial compartment of the knee can lead to varus deformity. In certain embodiments, cartilage loss can be estimated in the affected compartments. The estimation of cartilage loss can be done using an ultrasound MRI or CT scan or other imaging modality, optionally with intravenous or intra-articular contrast. The estimation of cartilage loss can be as simple as measuring or estimating the amount of joint space loss seen on x-rays. For the latter, typically standing x-rays are preferred. If cartilage loss is measured from x-rays using joint space loss, cartilage loss on one or two opposing articular surfaces can be estimated by, for example, dividing the measured or estimated joint space loss by two to reflect the cartilage loss on one articular surface. Other ratios or calculations are applicable depending on the joint or the location within the joint. Subsequently, a normal cartilage thickness can be virtually established on one or more articular surfaces by simulating normal cartilage thickness. In this manner, a normal or near normal cartilage surface can be derived. Normal cartilage thickness can be virtually simulated using a computer, for example, based on computer models, for example using the thickness of adjacent normal cartilage, cartilage in a contralateral joint, or other anatomic information including subchondral bone shape or other articular geometries. Cartilage models and estimates of cartilage thickness can also be derived from anatomic reference databases that can be matched, for example, to a patient's weight, sex, height, race, gender, or articular geometry(ies).

The limb alignment can be virtually corrected by realigning the knee after establishing a normal cartilage thickness or shape in the affected compartment by moving the joint bodies, for example, femur and tibia, so that the opposing cartilage surfaces including any augmented or derived or virtual cartilage surface touch each other, typically in the preferred contact areas. These contact areas can be simulated for various degrees of flexion or extension.

Any current and future method for determining limb alignment and simulating normal knee alignment can be used.

### 3. Parameters for designing a patient-specific implant

The patient-specific implants of certain embodiments can be designed based on patient-specific data to optimize one or more parameters including, but not limited to: (1) deformity correction and limb alignment (2) maximum preservation of bone, cartilage, or ligaments, (3) preservation and/or optimization of other features of the patient's biology, such as trochlea and trochlear shape, (4) restoration and/or optimization of joint kinematics, and (5) restoration or optimization of joint-line location and/or joint gap width. Various aspects of an implant component that can be designed or engineered based on the patient-specific data to help meet any number of user-defined thresholds for these parameters. The aspects of an implant that can be designed and/or engineered patient-specifically can include, but are not limited to, (a) implant shape, external and internal, (b) implant size, (c) and implant thickness.

There are several advantages that a patient-specific implant designed and/or engineered to meet or improve one of more of these parameters can have over a traditional implant. These advantages can include, for example: improved mechanical stability of the extremity; opportunity for a pre-primary or additional revision implant; improved fit with existing or modified biological features; improved motion and kinematics, and other advantages.

### 3.1 Deformity correction and optimizing limb alignment

Information regarding the misalignment and the proper mechanical alignment of a patient's limb can be used to preoperatively design and/or select one or more features of a joint implant and/or implant procedure. For example, based on the difference between the patient's misalignment and the proper mechanical axis, a knee implant and implant procedure can be designed and/or selected preoperatively to include implant and/or resection dimensions that substantially realign the patient's limb to correct or improve a patient's alignment deformity. In addition, the process can include selecting and/or designing one or more surgical tools (e.g., guide tools or cutting jigs) to direct the clinician in resectioning the patient's bone in accordance with the preoperatively designed and/or selected resection dimensions.

In certain embodiments, the degree of deformity correction that is necessary to establish a desired limb alignment is calculated based on the imaging data. The desired deformity correction can be to achieve any degree of varus or valgus alignment or antecurvatum or recurvatum alignment. In a preferred embodiment, the desired deformity correction returns the leg to normal alignment, for example, a zero degree biomechanical axis in the coronal plane and absence of genu antecurvatum and recurvatum in the sagittal plane. The correction can be performed in a single plane, for example, in the coronal plane or in the sagittal plane. The correction can be performed in multiple planes, for example, in coronal and sagittal planes. Moreover, the correction can be performed in three dimensions. For this purpose, three-dimensional representations of the joints can be used.

### 3.2 Preserving bone, cartilage or ligament

Traditional orthopedic implants incorporate bone cuts. These bone cuts achieve two objectives: they establish a shape of the bone that is adapted to the implant and they help achieve a normal or near normal axis alignment. For example, bone cuts can be used with a knee implant to correct an underlying varus of valgus deformity and to shape the articular surface of the bone to fit a standard, bone-facing surface of a traditional implant component. With a traditional implant, multiple bone cuts are placed. However, since traditional implants are manufactured off-the-shelf without use of patient-specific information, these bone cuts are pre-set for a given implant without taking into consideration the unique shape of the patient. Thus, by cutting the patient's bone to fit the traditional implant, more bone is discarded than is necessary with an implant designed to address the particularly patient's structures and deficiencies.

### 3.2.1 Planning bone cuts for one or more articular surfaces

In certain embodiments, bone cuts are optimized to preserve the maximum amount of bone for each individual patient, based on a series of two-dimensional images or a three-dimensional representation of the patient's articular anatomy and geometry and the desired limb alignment and/or desired deformity correction. Bone cuts on two opposing articular surfaces can be optimized to achieve the minimum amount of bone resection on both articular surfaces.

By adapting bone cuts in the series of two-dimensional images or the three-dimensional representation on two opposing articular surfaces such as, for example, a femoral head and an acetabulum, one or both femoral condyle(s) and a tibial plateau, a trochlea and a patella, a glenoid and a humeral head, a talar dome and a tibial plafond, a distal humerus and a radial head and/or an ulna, or a radius and a scaphoid, certain embodiments allow for patient individualized, bone-preserving implant designs that can assist with proper ligament balancing and that can help avoid "overstuffmg" of the joint, while achieving optimal bone preservation on one or more articular surfaces in each patient.

The bone cuts also can be designed to meet or exceed a certain minimum material thickness, for example, the minimum amount of thickness required to ensure biomechanical stability and durability of the implant. In certain embodiments, the limiting minimum implant thickness can be defined at the intersection of two adjoining bone cuts on the inner, bone-facing surface of an implant component. For example, in the femoral implant component **700** shown in **FIG. 7****,** the minimum thickness of the implant component appears at one or more intersections **710.** In certain embodiments of a femoral implant component, the minimum implant thickness can be less than 10 mm, less than 9 mm, less than 8 mm, less than 7 mm, and/or less than 6 mm.

These optimizations can be performed for one, two, or three opposing articular surfaces, for example, in a knee they can be performed on a tibia, a femur and a patella.

### 3.2.2 Optimized bone cuts for articular surfaces in knee replacement

In a knee, different bone cuts can be planned for a medial and lateral femoral condyle. The medial and lateral femoral condyles have different geometry, including, for example, width, length and radii in multiple planes, for example, the coronal and the sagittal plane. Bone cuts can be optimized in the femur individually for each condyle, resulting in bone cuts placed at a different depth or angle in one condyle relative to the other condyle. For example, a horizontal cut in a medial condyle may be anatomically placed more inferior relative to the limb than a horizontal cut in a lateral condyle. The distance of the horizontal cut from the subchondral bone may be, however, in each condyle approximately the same. Chamfer cuts in the medial and lateral condyle may be placed along different rather than the same plane in order to optimize bone preservation. Moreover, chamfer cuts in the medial and lateral condyle may be placed at a different angle in order to maximize bone preservation. Posterior cuts may be placed in a different plane, parallel or non-parallel, in a medial and a lateral femoral condyle in order to maximize bone preservation. A medial condyle may include more bone cuts than a lateral condyle in order to enhance bone preservation or vice versa.

In certain embodiments, a measure of bone preservation can include total volume of bone resected, volume of bone resected from one or more resection cuts, volume of bone resected to fit one or more implant component bone cuts, average thickness of bone resected, average thickness of bone resected from one or more resection cuts, average thickness of bone resected to fit one or more implant component bone cuts, maximum thickness of bone resected, maximum thickness of bone resected from one or more resection cuts, maximum thickness of bone resected to fit one or more implant component bone cuts.

Certain embodiments are directed to a femoral implant component having more than five bone cuts, for example, six, seven, eight or more bone cuts on the inner, bone-facing surface of the implant component. Alternatively, certain embodiments are directed to different orientations of five bone cuts, for example, a flexed orientation. A patient-specific implant with a higher number of bone cuts and/or a different orientation of bone cuts can allow for enhanced bone preservation over a traditional femoral implant with five standard bone cuts and therefore can perform as a pre-primary implant. However, a patient-specific implant having bone cuts that are non-parallel to the cuts of a subsequent primary can result in the primary implant having small gaps between the bone and the inner, bone-facing surface of the primary implant. These small gaps can result misalignment intersects between the pre-primary implant and the subsequent primary implant. For example, as shown in **FIG. 7-1****,** the bone cuts (shown in gray) for a pre-primary implant component having a 5-flex cut can retain bone as compared to a traditional primary implant (shown in outline), but a small gap **730** also can be created by the pre-primary cut. Any such small gaps **730** can be filled with bone cement when fitting a subsequent primary implant.

In addition to optimizing bone preservation, another factor in determining the depth, number, and/or orientation of resection cuts and/or implant component bone cuts is desired implant thickness. A minimum implant thickness can be included as part of the resection cut and/or bone cut design to ensure a threshold strength for the implant in the face of the stresses and forces associated with joint motion, such as standing, walking, and running. **Table 2** shows the results of a finite element analysis (FEA) assessment for femoral implant components of various sizes and with various bone cut numbers and orientations. The maximum principal stress observed in FEA analysis can be used to establish an acceptable minimum implant thickness for an implant component having a particular size and, optionally, for a particular patient (e.g., having a particular weight, age, activity level, etc). Before, during, and/or after establishing a minimum implant component thickness, the optimum depth of the resection cuts and the optimum number and orientation of the resection cuts and bone cuts, for example, for maximum bone preservation, can designed.

In certain embodiments, an implant component design or selection can depend, at least in part, on a threshold minimum implant component thickness. In turn, the threshold minimum implant component thickness can depend, at least in part, on patient-specific data, such as condylar width, femoral transepicondylar axis length, and/or the patient's specific weight. In this way, the threshold implant thickness, and/or any implant component feature, can be adapted to a particular patient based on a combination of patient-specific geometric data and on patient-specific anthropometric data. This approach can apply to any implant component feature for any joint, for example, the knee, the hip, or the shoulder.

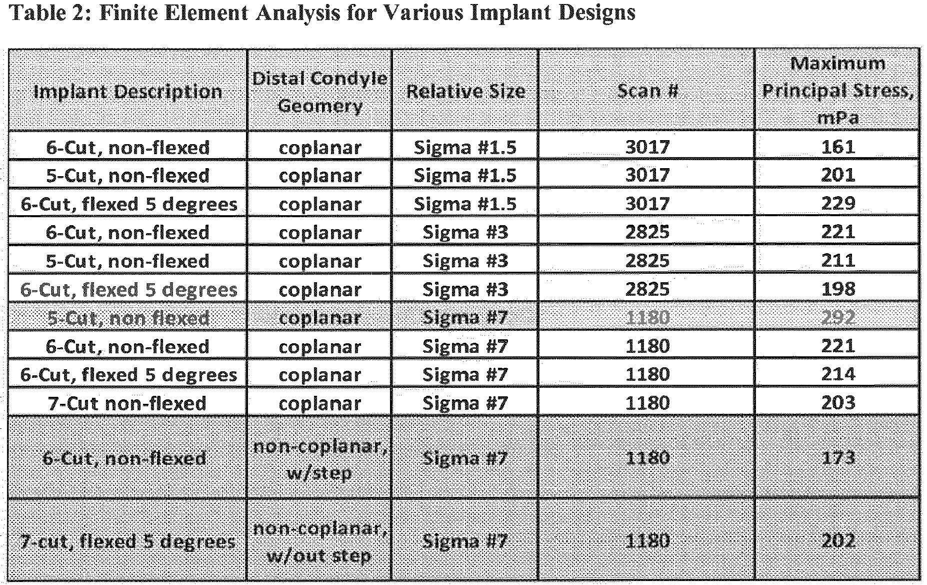

A weighting optionally can be applied to each bone with regard to the degree of bone preservation achieved. For example, if the maximum of bone preservation is desired on a tibia or a sub-segment of a tibia, femoral bone cuts can be adapted and moved accordingly to ensure proper implant alignment and ligament balancing. Conversely, if maximum bone preservation is desired on a femoral condyle, a tibial bone cut can be adjusted accordingly. If maximum bone preservation is desired on a patella, a bone cut on the opposing trochlea can be adjusted accordingly to ensure maximal patellar bone preservation without inducing any extension deficits. If maximum bone preservation is desired on a trochlea, a bone cut on the opposing patella can be adjusted accordingly to ensure maximal patellar bone preservation without inducing any extension deficits. Any combination is possible and different weightings can be applied. The weightings can be applied using mathematical models or, for example, data derived from patient reference databases.

### 3.2.3 Ligament preservation

Implant design and modeling also can be used to achieve ligament sparing, for example, with regard to the PCL and/or the ACL. An imaging test can be utilized to identify, for example, the origin and/or the insertion of the PCL and the ACL on the femur and tibia. The origin and the insertion can be identified by visualizing, for example, the ligaments directly, as is possible with MRI or spiral CT arthrography, or by visualizing bony landmarks known to be the origin or insertion of the ligament such as the anterior and posterior tibial spines.

An implant system can then be selected or designed based on the image data so that, for example, the femoral component preserves the ACL and/or PCL origin, and the tibial component preserves the ACL and/or PCL attachment. The implant can be selected or designed so that bone cuts adjacent to the ACL or PCL attachment or origin do not weaken the bone to induce a potential fracture.

For ACL preservation, the implant can have two unicompartmental tibial components that can be selected or designed and placed using the image data. Alternatively, the implant can have an anterior bridge component. The width of the anterior bridge in AP dimension, its thickness in the superoinferior dimension or its length in mediolateral dimension can be selected or designed using the imaging data and, specifically, the known insertion of the ACL and/or PCL.

Any implant component can be selected and/or adapted in shape so that it stays clear of important ligament structures. Imaging data can help identify or derive shape or location information on such ligamentous structures. For example, the lateral femoral condyle of a unicompartmental, bicompartmental or total knee system can include a concavity or divet to avoid the popliteus tendon. In a shoulder, the glenoid component can include a shape or concavity or divet to avoid a subscapularis tendon or a biceps tendon. In a hip, the femoral component can be selected or designed to avoid an iliopsoas or adductor tendons.

### 3.3 Establishing normal or near-normal joint kinematics

In certain embodiments, bone cuts and implant shape including at least one of a bone-facing or a joint-facing surface of the implant can be designed or selected to achieve normal joint kinematics.

In certain embodiments, a computer program simulating biomotion of one or more joints, such as, for example, a knee joint, or a knee and ankle joint, or a hip, knee and/or ankle joint can be utilized. In certain embodiments, patient-specific imaging data can be fed into this computer program. For example, a series of two-dimensional images of a patient's knee joint or a three-dimensional representation of a patient's knee joint can be entered into the program. Additionally, two-dimensional images or a three-dimensional representation of the patient's ankle joint and/or hip joint may be added.

Alternatively, patient-specific kinematic data, for example obtained in a gait lab, can be fed into the computer program. Alternatively, patient-specific navigation data, for example generated using a surgical navigation system, image guided or non-image guided can be fed into the computer program. This kinematic or navigation data can, for example, be generated by applying optical or RF markers to the limb and by registering the markers and then measuring limb movements, for example, flexion, extension, abduction, adduction, rotation, and other limb movements.

Optionally, other data including anthropometric data may be added for each patient. These data can include but are not limited to the patient's age, gender, weight, height, size, body mass index, and race. Desired limb alignment and/or deformity correction can be added into the model. The position of bone cuts on one or more articular surfaces as well as the intended location of implant bearing surfaces on one or more articular surfaces can be entered into the model.

A patient-specific biomotion model can be derived that includes combinations of parameters listed above. The biomotion model can simulate various activities of daily life including normal gait, stair climbing, descending stairs, running, kneeling, squatting, sitting and any other physical activity. The biomotion model can start out with standardized activities, typically derived from reference databases. These reference databases can be, for example, generated using biomotion measurements using force plates and motion trackers using radiofrequency or optical markers and video equipment.

The biomotion model can then be individualized with use of patient-specific information including at least one of, but not limited to the patient's age, gender, weight, height, body mass index, and race, the desired limb alignment or deformity correction, and the patient's imaging data, for example, a series of two-dimensional images or a three-dimensional representation of the joint for which surgery is contemplated.

An implant shape including associated bone cuts generated in the preceding optimizations, for example, limb alignment, deformity correction, bone preservation on one or more articular surfaces, can be introduced into the model. Parameters measured in the patient-specific biomotion model can include, but are not limited to:

### In a knee:

- Medial femoral rollback during flexion
- Lateral femoral rollback during flexion
- Patellar position, medial, lateral, superior, inferior for different flexion and extension angles
- Internal and external rotation of one or more femoral condyles
- Internal and external rotation of the tibia
- Flexion and extension angles of one or more articular surfaces
- Anterior slide and posterior slide of at least one of the medial and lateral femoral condyles during flexion or extension
- Medial and lateral laxity throughout the range of motion
- Contact pressure or forces on at least one or more articular surfaces, e.g. a femoral condyle and a tibial plateau, a trochlea and a patella
- Contact area on at least one or more articular surfaces, e.g. a femoral condyle and a tibial plateau, a trochlea and a patella
- Forces between the bone facing surface of the implant, an optional cement interface and the adjacent bone or bone marrow, measured at least one or multiple bone cut or bone facing surface of the implant on at least one or multiple articular surfaces or implant components.
- Ligament location, e.g. ACL, PCL, MCL, LCL, retinacula, joint capsule, estimated or derived, for example using an imaging test.
- Ligament tension, strain, shear force, estimated failure forces, loads for example for different angles of flexion, extension, rotation, abduction, adduction, with the different positions or movements optionally simulated in a virtual environment.
- Potential implant impingement on other articular structures, e.g. in high flexion, high extension, internal or external rotation, abduction or adduction or any combinations thereof or other angles / positions / movements.

### Similar parameters can be measured in other joints, e.g. in a hip or shoulder:

- Internal and external rotation of one or more articular surfaces
- Flexion and extension angles of one or more articular surfaces
- Anterior slide and posterior slide of at least one or more articular surfaces during flexion or extension, abduction or adduction, elevation, internal or external rotation
- Joint laxity throughout the range of motion
- Contact pressure or forces on at least one or more articular surfaces, e.g. an acetabulum and a femoral head, a glenoid and a humeral head
- Contact pressure or forces on at least one or more articular surfaces, e.g. an acetabulum and a femoral head, a glenoid and a humeral head
- Forces between the bone facing surface of the implant, an optional cement interface and the adjacent bone or bone marrow, measured at least one or multiple bone cut or bone facing surface of the implant on at least one or multiple articular surfaces or implant components.
- Ligament location, e.g. transverse ligament, glenohumeral ligaments, retinacula, joint capsule, estimated or derived, for example using an imaging test.
- Ligament tension, strain, shear force, estimated failure forces, loads for example for different angles of flexion, extension, rotation, abduction, adduction, with the different positions or movements optionally simulated in a virtual environment.
- Potential implant impingement on other articular structures, e.g. in high flexion, high extension, internal or external rotation, abduction or adduction or elevation or any combinations thereof or other angles / positions / movements.

The above list is not meant to be exhaustive, but only exemplary. Any other biomechanical parameter known in the art can be included in the analysis.

The resultant biomotion data can be used to further optimize the implant design with the objective to establish normal or near normal kinematics. The implant optimizations can include one or multiple implant components. Implant optimizations based on patient-specific data including image based biomotion data include, but are not limited to:
- Changes to external, joint facing implant shape in coronal plane
- Changes to external, joint facing implant shape in sagittal plane
- Changes to external, joint facing implant shape in axial plane
- Changes to external, joint facing implant shape in multiple planes or three dimensions
- Changes to internal, bone facing implant shape in coronal plane
- Changes to internal, bone facing implant shape in sagittal plane
- Changes to internal, bone facing implant shape in axial plane
- Changes to internal, bone facing implant shape in multiple planes or three dimensions
- Changes to one or more bone cuts, for example with regard to depth of cut, orientation of cut

Any single one or combinations of the above or all of the above on at least one articular surface or implant component or multiple articular surfaces or implant components.

When changes are made on multiple articular surfaces or implant components, these can be made in reference to or linked to each other. For example, in the knee, a change made to a femoral bone cut based on patient-specific biomotion data can be referenced to or linked with a concomitant change to a bone cut on an opposing tibial surface, for example, if less femoral bone is resected, the computer program may elect to resect more tibial bone.

Similarly, if a femoral implant shape is changed, for example on an external surface, this can be accompanied by a change in the tibial component shape. This is, for example, particularly applicable when at least portions of the tibial bearing surface negatively-match the femoral joint-facing surface.

Similarly, if the footprint of a femoral implant is broadened, this can be accompanied by a widening of the bearing surface of a tibial component. Similarly, if a tibial implant shape is changed, for example on an external surface, this can be accompanied by a change in the femoral component shape. This is, for example, particularly applicable when at least portions of the femoral bearing surface negatively-match the tibial joint facing surface.

Similarly, if a patellar component radius is widened, this can be accompanied by a widening of an opposing trochlear bearing surface radius, or vice-versa.

These linked changes also can apply for hip and/or shoulder implants. For example, in a hip, if a femoral implant shape is changed, for example on an external surface, this can be accompanied by a change in an acetabular component shape. This is, for example, particularly applicable when at least portions of the acetabular bearing surface negatively-match the femoral joint facing surface. In a shoulder, if a glenoid implant shape is changed, for example on an external surface, this can be accompanied by a change in a humeral component shape. This is, for example, particularly applicable when at least portions of the humeral bearing surface negatively-match the glenoid joint facing surface, or vice-versa.

Any combination is possible as it pertains to the shape, orientation, and size of implant components on two or more opposing surfaces.

By optimizing implant shape in this manner, it is possible to establish normal or near normal kinematics. Moreover, it is possible to avoid implant related complications, including but not limited to anterior notching, notch impingement, posterior femoral component impingement in high flexion, and other complications associated with existing implant designs. For example, certain designs of the femoral components of traditional knee implants have attempted to address limitations associated with traditional knee implants in high flexion by altering the thickness of the distal and/or posterior condyles of the femoral implant component or by altering the height of the posterior condyles of the femoral implant component. Since such traditional implants follow a one-size-fits-all approach, they are limited to altering only one or two aspects of an implant design. However, with the design approaches described herein, various features of an implant component can be designed for an individual to address multiple issues, including issues associated with high flexion motion. For example, designs as described herein can alter an implant component's bone-facing surface (for example, number, angle, and orientation of bone cuts), joint-facing surface (for example, surface contour and curvatures) and other features (for example, implant height, width, and other features) to address issues with high flexion together with other issues.

The biomotion model can be supplemented with patient-specific finite element modeling or other biomechanical models known in the art.

Resultant forces in the knee joint can be calculated for each component for each specific patient. The implant can be engineered to the patient's load and force demands. For instance, a 1251b. patient may not need a tibial plateau as thick as a patient with 2801bs. Similarly, the polyethylene can be adjusted in shape, thickness and material properties for each patient. A 3mm polyethylene insert can be used in a light patient with low force and a heavier or more active patient may need an 8mm polymer insert or similar device.

### 3.4 Restoration or optimization of joint-line location and joint gap width

Traditional implants frequently can alter the location of a patient's existing or natural joint-line. For example, with a traditional implant a patient's joint line can be offset proximally or distally as compared to the corresponding joint-line on the corresponding limb. This can cause mechanical asymmetry between the limbs and result in uneven movement or mechanical instability when the limbs are used together. An offset joint-line with a traditional implant also can cause the patient's body to appear symmetrical.

Traditional implants frequently alter the location of a patient's existing or natural joint-line because they have a standard thickness that is thicker or thinner than the bone and/or cartilage that they are replacing. For example, a schematic of a traditional implant component is shown in **FIGS. 7-2A** and **7-2B.** In the figure, the dashed line represents the patient's existing or natural joint line **740** and the dotted line represents the offset joint line **742** following insertion of the traditional implant component **750.** As shown in **FIG. 7-2A****,** the traditional implant component **750** with a standard thickness replaces a resectioned piece **752** of a first biological structure **754** at an articulation between the first biological structure **754** and a second biological structure **756.** The resectioned piece **752** of the biological structure can include, for example, bone and/or cartilage, and the biological structure **754** can include bone and/or cartilage. In the figure, the standard thickness of the traditional implant component **750** differs from the thickness of the resectioned piece **752.** Therefore, as shown in **FIG. 7-****2B,** the replacement of the resectioned piece **752** with the traditional implant component **750** creates a wider joint gap **758** and/or an offset joint-line. Surgeons can address the widened joint gap **758** by pulling the second biological structure **756** toward the first biological structure **754** and tightening the ligaments associated with the joint. However, while this alteration restores some of the mechanical instability created by a widened joint gap, it also exacerbates the displacement of the joint-line.

Certain embodiments are directed to implant components, and related designs and methods, having one or more aspects that are engineered from patient-specific data to restore or optimize the particular patient's joint-line location. In addition or alternatively, certain patient-specific implant components, and related designs and methods, can have one or more aspects that are engineered from patient-specific data to restore or optimize the particular patient's joint gap width.

### 3.4.1 Joint-line location

In certain embodiments, an implant component can be designed based on patient-specific data to include a thickness profile between its joint-facing surface and its bone-facing surface to restore and/or optimize the particular patient's joint-line location. For example, as schematically depicted in **FIG. 7-3****,** the thickness profile (shown as **A)** of the patient-specific implant component **760** can be designed to, at least in part, substantially positively-match the distance from the patient's existing or natural joint-line **740** to the articular surface of the biological structure **754** that the implant **760** engages. In the schematic depicted in the figure, the patient joint gap width also is retained.

The matching thickness profile can be designed based on one or more of the following considerations: the thickness (shown as **A'** in **FIG 7-3****)** of a resectioned piece of biological structure that the implant replaces; the thickness of absent or decayed biological structure that the implant replaces; the relative compressibility of the implant material(s) and the biological material(s) that the implant replaces; and the thickness of the saw blade(s) used for resectioning and/or material lost in removing a resectioned piece.

For embodiments directed to an implant component thickness that is engineered based on patient-specific data to optimize joint-line location (and/or other parameters such as preserving bone), the minimum acceptable thickness of the implant can be a significant consideration. Minimal acceptable thickness can be determined based on any criteria, such as a minimum mechanical strength, for example, as determined by FEA. Accordingly, in certain embodiments, an implant or implant design includes an implant component having a minimal thickness profile. For example, in certain embodiments a pre-primary or primary femoral implant component can include a thickness between the joint-facing surface and the bone-facing surface of the implant component that is less than 5 mm, less than 4 mm, less than 3 mm, and/or less than 2 mm

In certain embodiments, the implant component thickness can range from about 2 mm to about 3 mm. Therefore, for patients that require only minimal bone resectioning of no more than 2-3 mm depth from the joint line, an implant component designed with a thickness to substantially positively-match the 2-3 mm bone resectioning can maintain the joint-line location. Moreover, a subsequent traditional primary implant, for example, of 5 mm or greater thickness can be applied later with an additional cut depth of 3-2 mm or greater (for a total 5 mm cut depth). This can allow for maintenance of the joint-line with the subsequent, traditional primary as well.

Certain embodiments directed to implants or implant designs optimized to achieve minimal implant thickness can include a higher number of bone cuts, for example, six, seven, eight or more bone cuts, on the inner, bone-facing surface of the implant. The bone cuts can be oriented in various dimensions, for example, in a flexed-orientation. Moreover, certain embodiments can include on the inner, bone-facing surface any combination of straight cuts, curvilinear cuts, and/or portions that substantially positively-match an uncut articular bone surface. For example, as described in Example 13, an implant or implant design can include a straight anterior cut, a straight posterior cut, and a curvilinear cut in between. As depicted in **FIG. 7-4****,** an implant or implant design **770** can include a straight distal cut **772,** a straight anterior cut **774,** a straight posterior cut **776,** and curvilinear chamfer cuts **778** in between to substantially negatively-match corresponding designed cuts to the femur **780.** As described in Example 14, an implant or implant design can on the inner, bone-facing surface one or no straight cuts and portions that substantially positively-match an uncut articular bone surface.

The inner, bone-facing surface of the implant component can be designed to substantially negatively-match the cut bone surface, both curved and straight portions. The curved cuts to the bone can be performed with a router saw, as described in Example 13. Any number of the cuts can have a depth of 2-3 mm, and the implant component thickness can be designed to positively-match the cut depth across a portion of the implant or across the entire implant.

By positively-matching the implant component thickness profile with the cut depth profile, and by negatively matching the component bone-facing surface with the cut articular surface of the biological structure, certain aspects of the component joint-facing surface can positively-match corresponding aspects of biological structure surface that it replaces. For example, if the component inner surface and thickness match the corresponding features of the biological structure, the component joint-facing curvature, such as a j-curve, also can match the corresponding surface curvature of the patient's biological structure.

### 3.4.2 Joint gap width

In certain embodiments, one or more implant components can be designed based on patient-specific data to include a thickness profile that retains, restores, and/or optimizes the particular patient's joint gap width. For example, as schematically depicted in **FIG. 7-5A** and **7-5B,** the patient-specific implant components **785, 786** can be designed to, at least in part, substantially positively-match the patient's existing or natural joint gap **788.** In the figure, the dashed line represents the patient's existing or natural joint line **790.** The patient-specific implant components **785, 786** do not have thicknesses that match the corresponding resectioned pieces **792, 794** of biological structures **796, 798.** However, as shown in **FIG. 7-5B****,** the implant components **785,** 786 are designed to retain the patient's specific gap width **788.**

If the thickness of an implant component is greater than the depth of the corresponding bone cut, then the thicker implant component can shift the joint-line down. However, as shown in **FIG. 7-5A** and **7-5B,** the joint gap width can be retained by designing a second implant component to offset the greater thickness of the first implant component. For example, in total knee replacements that include both a femoral implant component and a tibial implant component, if the femoral implant component is thicker than the depth of the corresponding bone cut, more tibial bone can be cut and/or a thinner tibial implant can be used. In certain embodiments, a tibial bone cut and/or the thickness of a corresponding portion of a tibial implant component may be less than about 6 mm, less than about 5 mm, less than about 4 mm, less than about 3 mm, and/or less than about 2 mm

One or more components of a tibial implant can be designed thinner to retain, restore, and/or optimize a patient's joint-line and/or joint gap width. For example, one or both of a tibial tray and a tibial tray insert (e.g., a poly insert) can be designed and/or selected (e.g., preoperatively selected) to be thinner in one or more locations in order to address joint-line and/or joint-gap issues for a particular patient. In certain embodiments, a tibial bone cut and/or the thickness of a corresponding portion of a tibial implant component may be less than about 6 mm, less than about 5 mm, less than about 4 mm, less than about 3 mm, and/or less than about 2 mm

In certain embodiments, one or more implant components can designed based on patient-specific data to include a thickness profile that retains or alters a particular patient's joint gap width to retain or correct another patient-specific feature. For example, the patient-specific data can include data regarding the length of the patient's corresponding limbs (e.g., left and right limbs) and the implant component(s) can be designed to, at least in part, alter the length of one limb to better match the length of the corresponding limb.

### 4. Analyzing parameters and computer-aided design

Any combination of the above embodiments is possible. For example, a series of operations can be performed, optionally with a software-directed computer, to transform patient data into an output to identify to a user the best compromise between one or more of the following parameters: (a) limb alignment and deformity correction, (b) bone preservation via adjustment of orientation and location of bone cuts, (c) establishing normal or near normal joint kinematics including ligament function and implant impingement, (d) implant shape, external and internal, (e) implant size, (f) implant thickness, (g) joint line location, and (h) location and preservation of trochlea and trochlear shape. Optimization of joint kinematics can include, as another parameter, the goal of not moving the joint line postoperatively or minimizing any movements of the joint line, or any threshold values or cut off values for moving the joint line superiorly or inferiorly. Optimization of joint kinematics also can include ligament loading or function during motion. Optimization of implant thickness can include femur and/or condyle size and patient weight. For example, in certain embodiments, a patient-specific implant component design can depend, at least in part, on the optimization of implant thickness. However, the optimized implant thickness can depend, at least in part, on the patient's specific condylar width or femur transepicondylar axis length and the patient's specific weight. Accordingly, implant thickness or any of the above-identified implant parameters, can be optimized (i.e., adapted to a particular patient) based on patient-specific geometric data and on patient anthropometric data. This approach can apply to any implant parameter for any joint, for example, the knee, the hip, or the shoulder.

### 4.1 Process flow

Any one of the above-identified parameters can be the first parameter set or determined in an optimization. Alternatively, the process can be iterative in nature. It can be fully automated or it can be partially automated allowing for user interaction. User interaction can be particularly useful for quality assurance purposes. Different weighting can be applied to any of these parameters, for example, based on the patient's age, the surgeon's preference or the patient's preference. Feedback mechanisms can be used to show the user or the software what changes in certain parameter values based on desired changes to one or more parameters. For example, a feedback mechanism can indicate the changes that occur for limb alignment and deformity correction or joint kinematics parameters if a desired change is applied to minimize bone cuts for bone preservation. Accordingly, implant shape can be modeled and modified to achieve the optimum solution.

In certain embodiments, mathematical modeling can be applied to find an ideal solution, for example, according to user-selected parameter thresholds or values and/or user-selected relative weightings for the parameters included in the model. Alternatively, a solution can be defined using clinical data, for example obtained from clinical trials, or intraoperative data.

### 4.2 Computer-aided design

The processing of the patient data, the design of one or more components of a patient-specific implant, the manufacture of the designed implant, and/or the implantation procedure(s) may be partially or wholly automated. For example, the patient data, with optional user-defined parameters, may be inputted or transferred by a user and/or by electronic transfer into a software-directed computer system that performs a series of operations to generate one or more virtual models or implant design specifications. Implant design data, with optional user-defined parameters, may be inputted or transferred by a user and/or by electronic transfer into a software-directed computer system that performs a series of operations to transform the data and optional parameters into one or more implant manufacturing specifications. Implant design data or implant manufacturing data, optionally with user-defined parameters, may be inputted or transferred by a user and/or by electronic transfer into a software-directed computer system that directs one or more manufacturing instruments to produce one or more implant components from a starting material, such as a raw material or starting blank material. Implant design data, implant manufacturing data, or implant data, optionally with user-defined parameters, may be inputted or transferred by a user and/or by electronic transfer into a software-directed computer system that performs a series of operations to transform the data and optional parameters into one or more surgical procedure specifications. Implant design data, implant manufacturing data, implant data, or surgical procedure data, optionally with user-defined parameters, may be inputted or transferred by a user and/or by electronic transfer into a software-directed computer system that directs one or more automated surgical instruments, for example, a robot, to perform one or more surgical steps. In certain embodiments, one or more of these actions can be performed as steps in a single process by one or more software-directed computer systems.

A computer operating according to a software program can be used to assess a combination of user-selected and/or weighted parameters and then reports to a user the output values or ranges for those parameters. For example, in certain embodiments, the following parameters can be optimized for each patient implant: tibial cut height (preferably minimize); position of joint line (preferably preserve for natural kinematics); and thickness of femoral cut (preferably minimize).

Optimization of multiple parameters may result in conflicting constraints; for example, optimizing one parameter causes an undesired deviation to one or all of the parameters. In cases where not all constraints can be achieved at the same time, parameters can be assigned a priority or weight in the software program depending on the user's desired design goals, for example, minimization of bone loss, or retention of existing joint line to preserve kinematics, or combination to accommodate both parameters in overall design.

In any automated process or process step performed by the computer system, constraints pertaining to a specific implant model, to a group of patients or to the individual patient may be taken into account. For example, the maximum implant thickness or allowable positions of implant anchors may depend on the type of implant. The minimum implant thickness can depend on the patient's bone quality.

In certain embodiments, the final implant includes one or more bone cuts. The cut planes for these bone cuts can be automatically determined by the computer system, for example using anatomical landmarks. In certain embodiments, a computer program can aid in the determination of bone cuts that are optimized to preserve the maximum amount of bone for each individual patient based on a series of two-dimensional images or a three-dimensional representation of the articular anatomy and geometry and the desired limb alignment and/or desired deformity correction. Optionally, the cut planes can be adjusted by the operator.

The computer system can also construct the implant surfaces. Surfaces may be composed of different elements. In certain embodiments, elements of the surfaces conform to the patient's anatomy. In these situations, the computer system can build a surface using the patient's anatomical model, for example by constructing a surface that is identical with or mostly parallel to the patient's anatomical surface. In certain embodiments, the computer system uses geometric elements such as arcs or planes to construct a surface. Transitions between surfaces can be smoothed using tapers or fillets. Additionally, the computer system may take into account constraints such as minimum or maximum thickness or length or curvature of parts or aspects of the implant when constructing the surfaces.

In another embodiment, the computer system can automatically or semiautomatically add other features to the implant design. For example, the computer system can add pegs or anchors or other attachment mechanisms. The system can place the features using anatomical landmarks. Constraints can be used to restrict the placement of the features. Examples of constraints for placement of pegs are the distance between pegs and from the pegs to the edge of the implant, the height of the pegs that results from their position on the implant, and forcing the pegs to be located on the center line. Optionally, the system can allow the user to fine-tune the peg placement, with or without enforcing the constraints.

### 4.3 Libraries

As described herein, implants of various sizes, shapes, curvatures and thicknesses with various types and locations and orientations and number of bone cuts can be designed and manufactured. The implant designs and/or implant components can be catalogued and stored to create a library. The library can be a virtual library of implants, or components, or elements that can be combined and/or altered to create a final implant. The library can include a catalogue of physical implant components. In certain embodiments, physical implant components can be identified and selected using the library. The library can include previously-generated implant components having one or more patient-specific aspects, and/or components with standard or blank aspects that can be altered to be patient-specific. Accordingly, implants and/or implant aspects can be selected from the library. **FIGS. 7-6A - 7-6K** show implant components with exemplary aspects that can be included in such a library.

A virtual or physical implant component can be selected from the library based on similarity to prior or baseline parameter optimizations, such as one or more of (1) deformity correction and limb alignment (2) maximum preservation of bone, cartilage, or ligaments, (3) preservation and/or optimization of other features of the patient's biology, such as trochlea and trochlear shape, (4) restoration and/or optimization of joint kinematics, and (5) restoration or optimization of joint-line location and/or joint gap width. Accordingly, one or more implant aspects, such as (a) implant shape, external and internal, (b) implant size, and/or (c) implant thickness, can be determined precisely and/or determined within a range from the library selection. Then, the selected implant component can be designed or engineered further to include one or more patient specific aspects. For example, a joint can be assessed in a particular subject and a pre-existing implant design having the closest shape and size and performance characteristics can be selected from the library for further manipulation (e.g., shaping) and manufacturing prior to implantation. For a library including physical implant components, the selected physical component can be altered to include a patient-specific aspect by adding material (e.g., laser sintering) and/or subtracting material (e.g., machining).

Accordingly, an implant component can include one or more aspects designed patient-specifically and one or more aspects generated from one or more library sources. For example, in designing an implant for a total knee replacement comprising a femoral component and a tibial component, one component can include one or more patient-specific aspects and the other component can be selected from a library. Table 3 includes an exemplary list of possible combinations.

**TABLE 3: Illustrative Combinations of Patient-Specific and Library-Derived Components**

| Implant component(s) | Implant component(s) having a patient-specific aspect | Implant component(s) having a library derived aspect |
|---|---|---|
| Femoral, Tibial | Femoral and Tibial | Femoral and Tibial |
| Femoral, Tibial | Femoral | Femoral and Tibial |
| Femoral, Tibial | Tibial | Femoral and Tibial |
| Femoral, Tibial | Femoral and Tibial | Femoral |
| Femoral, Tibial | Femoral and Tibial | Tibial |
| Femoral, Tibial | Femoral and Tibial | none |

### 5. Designing or selecting aspects of knee implant components

The following subsections describe aspects of certain embodiments of models, implant designs, implants, and implant components related to a knee replacement. While the sections particularly describe embodiments of knee implants, it is understood that the teachings are applicable to other embodiments including, but not limited to, shoulder implants and hip implants.

### 5.1 Femoral implant component

A traditional total knee implant used in a primary total knee arthroplasty ("TKA") typically includes: an outer, joint-facing surface (i.e., inferior surface) having a standard topography; an inner, bone-facing surface (i.e., superior surface) that includes five standard bone cuts; and a standard implant thickness between the joint-facing surface and the bone-facing surface. **FIG. 8** shows the five standard bone cuts that are resectioned from a subject's bone to fit a traditional total knee implant.. Specifically, **FIG. 8** shows a coronal view of a patient's femoral bone **800.** The bone cuts typically performed with a traditional total knee implant include a horizontal cut **810,** an anterior cut **820,** a posterior cut **830** along each femoral condyle, an anterior chamfer cut **840,** and a posterior chamfer cut **850.** The anterior and posterior cuts **820, 830** typically are placed in a substantially coronal plane. With a traditional implant, these five standard bone cuts are made to approximately negatively-match the standard five-faceted shape on the inner, bone-facing surface of a traditional implant. In other words, the patient's bone is cut to the fit the shape of the traditional implant.

Dissimilarly, in various embodiments described herein, one or more features of an implant component and/or implant procedure are designed and/or selected to provide a patient-adapted implant component. For example, in certain embodiments, one or more features of an implant component and/or implant procedure are designed and/or selected preoperatively, based on patient-specific data, to substantially match (e.g., substantially negatively-match and/or substantially positively-match) one or more of the patient's biological structures or a predetermined percentage thereof. For example, in certain embodiments, a femoral implant component can include an outer, joint-facing surface (i.e., inferior surface) having a sagittal or j-curve on one or both condyles that, at least in part, positively-matches the corresponding bone or cartilage curvature on the patient's uncut femur. This patient-specific implant component feature can be preoperatively selected and/or designed based on the patient's joint dimensions as seen, for example, on a series of two-dimensional images or a three-dimensional representation generated, for example, from a CT scan or MRI scan.

### 5.1.1 Size

In certain embodiments, the minimum thickness, the maximum thickness, the thickness across the entire component, and/or one or more other aspects of a femoral implant component, can be designed to match or resemble the patient's dimensions, optimized dimensions, and/or standard dimensions. Standard dimensions may be used, for example, for engaging one or more standard dimensions on an opposing component.

In certain embodiments, the minimum implant thickness is less than 9 mm, less than 8 mm, less than 7 mm, less than 6 mm, and/or less than 5 mm. In certain embodiments, this minimum implant thickness can allow for a subsequent knee implant using a primary implant.

### 5.1.2 Joint-facing surface

In certain aspects, the joint-facing surface of a femoral implant component includes one or more patient-specific dimensions, for example, that positively or negatively-match the patient's biological structure or that are engineered to provide an optimized fit based on parameters derived from patient-specific data.

The joint-facing surface of an implant component can include a bearing surface that contacts one or more other surfaces in the joint during proper joint function. In a total knee implant, the bearing surfaces can include the medial and lateral condyles on the joint-facing surface of the femoral component and the corresponding surface on the tibial component that contacts the medial and lateral condyles of the femoral component during proper joint function. Bearing surfaces also can include the trochlear area of a femoral implant component and the corresponding surface of a patella or patella implant component. In certain embodiments, the femoral implant component can be designed and/or selected to include a joint-facing surface that substantially negatively-matches one or more dimensions of an opposing surface, such as a tibial surface or a patellar surface, of the patient's biological structure or of another implant component, such as a tibial implant component or a patellar implant component.

One or more of the bearing surfaces on a femoral component can be of standard design, for example, available in 6 or 7 different shapes, with a single radius or multiple radii in one dimension or more than one dimension. Alternatively, a bearing surface can be standardized in one or more dimensions and patient-adapted in one or more dimensions. Constant and variable radii can be selected in one dimension or multiple dimensions. Some of the radii can be, for example patient adapted. For example, in a knee implant different radii can be selected on a medial and a lateral condylc. Moreover, portions of one condyle can be patient-specific, while portions of the other condyle or the entire other condyle can be standard in shape.

### 5.1.3 Femoral condyles - sagittal and coronal curvatures

The medial and lateral femoral condyle surfaces are the primary load bearing surfaces of a femoral implant component that engage the tibia or a tibial implant component in a knee joint. Accordingly, the design of these surfaces, and the design for how they engage the opposing surface(s) on a corresponding tibial component, can affect various design parameters described above, including implant wear and kinematics, particularly the proper motion of the implant at the joint. **FIGS. 8-1A** and **8-1 B** show the load bearing surfaces of a femoral implant component in a coronal view **(****FIG. 8-1A****)** and in a sagittal view **(****FIG. 8-1B****).** As indicated by the figures, the load bearing surface on each condyle has a coronal curvature **1210** and a sagittal curvature **1220.** In certain embodiments, any one or more of the coronal curvature of the medial condyle, the sagittal curvature of the medial condyle, the coronal curvature of the lateral condyle, and/or the sagittal curvature of the lateral condyle can include, at least in part, patient-specific radii. The remaining curvatures with non-patient-specific radii can include radii that are engineered or optimized with respect to any of the parameters described above and/or radii that are standard, for example, as selected from a family of curvatures. For example, a medial condyle can be partially patient adapted with regard to sagittal curvature and have a standard curvature in the coronal plane, while a lateral condyle can have a standard curvature in both coronal and sagittal planes.

In preferred embodiments, the femoral implant component is designed to include one or both condylar, bearing surfaces having a sagittal curvature with, at least in part, patient-specific radii and a coronal curvature with a standard curvature. For example, the coronal curvature can be selected by choosing from a family of standard curvatures the one standard curvature that is most similar to the external radii of the patient's uncut femoral condyle. Alternatively, the curvature can be selected by choosing from a family of standard curvatures a standard curvature with larger radii in order to achieve a less constraining biomechanical situation, or with smaller radii in order to achieve a more constraining biomechanical situation during knee motion.

The coronal radius of a typical human femoral condyle can range from 20 to 30 mm. In certain embodiments, the coronal radius of one or both condyles on a femoral implant component can be greater than 20 mm, greater than 30 mm, between 20 and 40 mm, or between 30 and 40 mm. **FIGS. 8-2A** and **8-2B** show cross-sections from a coronal view of two femoral condyle sections of a femoral component. As shown, the component cross-sections have the same maximum thickness, but the component in **FIG. 8-2A** has a larger coronal radius than the component in **FIG. 8-2B****.** As can be seen from the figures, where the maximum thickness is the same for the two components, the component with the larger coronal radius allows for more material at the edge of the component, and therefore can be less likely to fail in this area of the femoral implant component.

In certain embodiments, the sagittal or j-curve of the femoral component can be designed to be tilted to allow for thicker material on the corresponding tibial implant, as shown in **FIG. 8-2C****.** Since the AP slope of the tibial cut in certain embodiments is anatomic, the patient's J-curve can be tilted by that same anatomic slope in order to achieve a thicker poly anteriorly. For example, the patient's J-curve can be tilted by the same anatomic slope in both the medial and lateral condyles of a femoral implant component. Alternatively, the patient's J-curve can be tilted to the anatomic slope in just the medial condyle and lateral condyle curve can remain at a certain angle. Alternatively, the patient's J-curve can be tilted to the anatomic slope in just the lateral condyle and the medial condyle curve can remain at a certain angle. In certain embodiments, some material can be removed from the posterior aspect of one or both condyles to allow for rotation.

**FIGS. 8**-**3A** - **8-3F** include additional information regarding designing coronal curves and/or sagittal or j- curves for certain embodiments of patient-specific femoral implant components. In certain embodiments, the medial condyle can be engineered to be 5mm lateral, which can help lateralize the patella. In certain embodiment, intercondylar width can be patient specific. Alternatively or in addition, a minimum intercondylar width, such as 40 mm, can be used if the patient-specific width is smaller than the minimum.

### 5.1.4 Cut planning and placement

In traditional knee implant systems, the anterior or trochlear bone cut is located substantially in the coronal plane and is parallel to the two posterior condylar cuts, as shown in **FIG. 9A****.** **FIGS. 9**-**1A** - **9-1C** show femoral implant components with three exemplary bone cuts. In certain embodiments, the bone cuts are rotated or oriented based on a certain flexion angle of the knee. An example of a flexed-fit cut design is described in Example 2, below. Any number of cut planes can be included in an implant device designed with flexed-fit cuts. For example, two, three, four, five, six, seven, eight, nine or more cut planes can be included in a flexed-fit design. One or more of the cuts can be curvilinear or the entire bone-facing surface can be curvilinear. The cuts can be oriented at any rotation, for example, at 5, greater than 5, 10, greater than 10, 15, greater than 15, 20, greater than 20, 25, or greater than 25 degrees flexion from the perpendicular to the sagittal femoral axis.

### 5.1.4.1 Anterior condylar facets

In certain embodiments, the anterior or trochlear implant facet is substantially not parallel to the coronal plane, as exemplified by the "1 cut" dashed line in **FIG. 9B****.** For example, the anterior or trochlear cut can be parallel to a tangent through the two peak areas of the trochlea, as shown in **FIG. 9B****.** By placing the implant facet at an angle relative to the patient's coronal plane, for example, parallel to a tangent of the medial and lateral trochlear peak areas, a substantial amount of bone can be preserved. In certain embodiments, two trochlear bone cuts can be placed for an implant with two trochlear facets, as exemplified by the "2 cut" solid line in FIG. 9B. For example, one of the two cuts can be substantially parallel to the medial trochlear facet and the other can be substantially parallel to the lateral trochlear facet. This can further enhance the degree of bone preservation.

### 5.1.4.2 Posterior condylar facets

In traditional knee implant systems, the medial and lateral posterior condyles are cut in the same plane as each other, substantially parallel to each other, and substantially parallel to the anterior cut. However, in certain embodiments, the implant can have posterior condylar facets that are not parallel and/or not in the same plane as each other. Alternatively or additionally, the implant can have posterior condylar facets that are substantially non-parallel with each other. Alternatively, or additionally, the implant can have one or more posterior condylar facets that are substantially non-parallel with the anterior cut.

In certain preferred embodiments, the posterior condylar cut for the medial side can be perpendicular to the long axis of the medial condyle. The posterior condylar cut for the lateral side can be perpendicular to the long axis of the lateral condyle.

In certain embodiments, the anterior and posterior cuts can be substantially non-parallel to the coronal plane in the superoinferior orientation, as shown in **FIG. 10.**

### 5.1.4.3 Distal facets

In certain embodiments, the medial and lateral sides of a distal facet of a femoral implant component can be cut in the same plane as each other and/or substantially parallel to each other. However, in certain embodiments, the implant can have a distal facet with portions that are not parallel and/or not in the same plane as each other. Alternatively or additionally, the implant can have a distal facet that includes portions at separate heights.

### 5.1.4.4 Chamfer facets

Traditional implant systems include one anterior and one posterior chamfer facet. However, in certain embodiments, additional chamfer facets can be included. The additional chamfer cuts can be substantially tangent to the articular surface. For example, one or more additional anterior chamfer cuts can be included and/or one or more additional posterior chamfer cuts can be included. By increasing the number of chamfer facets on the implant and placing the cuts in close proximity to the tangent of the articular surface, additional bone can be preserved. In certain embodiments, the cut plane for one or more of the anterior chamfer cuts can be defined by the extent of the trochlear gap in the patient's joint. Specifically, one or more of the anterior cut planes can be designed so that there is no exposed surface on the bone-facing side of the implant component at the trochlear gap.

### 5.1.4.5 Cut strategies

Computer software can be used that calculates the closest location possible of the cuts relative to the articular surface, so that all intersects of adjoining cuts are just within the bone, rather than outside the articular surface. The software can move the cuts progressively closer to the articular surface. When all intersects of the cuts reach the endosteal bone level or the subchondral bone level, the maximum exterior placement of cuts is achieved and, with that, the maximum amount of bone preservation.

In certain embodiments, the bone cuts for an implant can be optimized based on an engineered condyle curvature. Specifically, a model or a mathematical formula can be used to engineer an optimized or corrected condyle shape. For example, the lateral condyle is often found to be deformed or hypoplastic for patient's in need of knee replacement, so an optimized lateral condyle can include additional radius and/or material added to the outer, condylar, joint-facing surface of the implant on the lateral condyle as compared to the patient's native, uncut condyle. Hypoplastic lateral condyles may be present in 20% of patients that require knee replacement.

The model or mathematical formula used to engineer condyle curvature can be based on one or more patient-specific dimensions. In certain embodiments, the lateral condyle can be engineered to have a curvature relative to the patient's medial condyle curvature. For example, the lateral J-curve can be engineered to have a smaller radius, for example, a 5%, 10%, 15%, 20%, 10-15%, and/or 0-20% smaller radius than the patient's medial J-curve.

The increase in radius and/or material on the outside of the condyle can be used to design a material savings on the inside of the corresponding section of the implant component, thus maintaining a minimum material / implant thickness. In this way, by adding radius and/or material to the external contour of the implant condyle, a minimum material thickness of the implant can be achieved externally. This allows for less material on the inner, bone-facing surface of the implant and, accordingly, less bone to be cut from the corresponding condyle of the patient. This approach also can be used to correct condyle shape abnormalities, such as lateral condyle abnormalities, such as hypoplasia, using patient-specific implants.

### 5.1.5 Pegs and cement pockets

Femoral implant components of certain embodiments also can include other features that are patient-specific and/or optimized according to one or more of the parameters discussed above.

The design of the component attachment pegs also may include features that are patient-specific and/or optimized according to one or more of the parameters discussed above. For example, the attachment pegs may be flexed relative to the biomechanical or anatomical axes.

The design of the component bone cement pocket or pockets also may include features that are patient-specific and/or optimized according to one or more of the parameters discussed above. **FIGS. 11A** and **11B** show bone cement pockets in a component of some embodiments **(****FIG. 11A****)** and in a traditional component **(****FIG. 11B****).** As shown in **FIG. 11A****,** each section or facet of the bone-facing surface of the component can have an independent cement pocket. One or more of the cement pockets can be offset from the periphery by 2 mm or more. Each pocket can have a maximum depth of less than 0.9 mm, for example, 0.5 mm or less.

### 5.1.6 Patella facets of femoral implant component

In traditional total knee replacement systems, a single patellar facet is used that is typically substantially parallel to the coronal plane. Patellar revision can be very challenging and bone preservation is preferred in the patella. In certain embodiments, two or more patellar facets can used on the implant. The patellar facets can be patient-specific, i.e., designed to match the patient's normal patellar tracking in the trochlear groove. Alternatively, the patellar facets can be optimized, i.e., to enhance kinematics between component surfaces. A method for designing a patient-specific implant to optimize tracking of the patella along the trochlear groove of a femoral implant component is described below in Example 4. Specifically, the exemplary implant design in Example 4 uses a patient-specific sagittal curvature and an engineered coronal curvature to allow the patella component to track properly in the trochlear groove. In certain other embodiments, the coronal curvature additionally can be patient-specific. In certain embodiments, the coronal curvature is patient-specific and the sagittal curvature is standard or engineered.

Patellar facets are placed via two or more patellar bone cuts. The two or more patellar facets can be substantially tangent or parallel to the medial and lateral patellar facet. They optionally also can be substantially tangent or parallel to the superior and inferior patellar facet, in particular, when more than two implant facets or bone cuts are used. In certain embodiments, the patellar facets include one or more curvilinear surfaces. In preferred embodiments, the trochlear groove is slightly larger than the corresponding engaging surface of the patella. In certain embodiments, the trochlear groove of the femoral implant is moved laterally relative to the patient's trochlear groove.

### 5.2 Patella implant component

Certain embodiments include a patella implant component having a patient-specific shape and size with a dome-shaped or prolate-shaped topography, 2-4 mm lateralizcd. A patella component designed in this way can be used to address poor ML and/or AP fit of traditional designs and/or restore the patient's normal patella topography. In addition or alternatively, the thickness of the patella implant can be less than about 11 mm, less than about 10 mm, less than 9 mm, less than about 8.5 mm, about 8 mm, less than about 8 mm, about 7 mm, and/or less than 7 mm.

Certain embodiments are directed to restoring the patient's original patella thickness, which can help to preserve bone and restore patella-femoral ("P-F") kinematics, for example, by restoring the patient's P-F joint-line. Accordingly, in certain embodiments, the thickness of the patella implant can be matched to be substantially the same as the thickness of the patient's patella.

In certain embodiments, one or more aspects of a patella implant are designed to optimally engage an engineered trochlear groove of a femoral implant component. For example, certain embodiments of an implant may include a patella implant component as described in **FIGS. 11-1A** and **11-1B****.**

Certain embodiments include a patella implant that is non-spheroid in shape, such as prolate in shape (i.e., an elongated shape, like a football or lemon), as shown in **FIG. 11-2****.** For example, the top-side of the patella implant can be lemon shaped such that it has a differing medial-lateral versus vertical radius. This design can allow for a reduced thickness of the leading edges of the implant during flexion/extension.

### 5.3 Tibia implant component

Certain embodiments include tibial implant components having one or more patient-specific or engineered aspects. For example, tibial components can include different medial and lateral cut heights. The lateral tibial plateau may be cut at 1 or 2 mm higher than the medial tibial plateau. The lateral tibial plateau facet of the implant can be 1 or 2 mm higher than the medial tibial plateau facet thereby moving the lateral tibial plateau bone cut 1 or 2 mm higher resulting in more bone preservation. **FIGS. 12A** and **12B** show tibial cuts and unicompartmental medial and lateral components with and without a polyethylene layer having different heights relative to the tibial plateau.

In certain embodiments, the medial tibial plateau facet can be oriented at an angle different than the lateral tibial plateau facet. Typically, each of the medial and the lateral tibial plateau facets is at an angle that is patient-specific, for example, similar to the original slope or slopes of the medial and lateral tibial plateaus, for example, in the sagittal plane. This is applicable to implants that use two unicompartmental tibia components, one medial and one lateral. It can also be applicable to implant systems that use single component tibia components, for example PCL retaining, posterior stabilized or ACL and PCL retaining components. The slope preferably is between 0 and 7 degrees, but other embodiments with other slope angles outside that range can be used. The tibial slope can vary across one or both tibial facets from anterior to posterior. For example, a lesser slope, e.g. 0-1 degrees, can be used anteriorly, and a greater slope can be used posteriorly, for example, 4-5 degrees. Variable slopes across at least one of a medial or a lateral tibial facet can be accomplished, for example, with use of burrs (for example guided by a robot) or with use of two or more bone cuts on at least one of the tibial facets. In certain embodiments, two separate slopes are used medially and laterally.

In certain embodiments, the medial and tibial plateau components of the tibia are cut at a different angle. Optionally, the medial and the lateral tibia also can be cut at a different distance relative to the tibial plateau. In this setting, the two horizontal plane tibial cuts medially and laterally can have different slopes and/or can be accompanied by one or two vertical cuts, typically placed medial to tibial plateau components.

The medial tibial plateau component can have a flat, convex, concave, or dished surface and/or it can have a thickness different than the lateral tibial plateau component. The lateral tibial plateau component can have a flat, convex, concave, or dished surface and/or it can have a thickness different than the medial tibial plateau component. The different thickness can be achieved using a different material thickness, for example, metal thickness or polyethylene thickness on either side. In certain embodiments, the lateral and medial surfaces are selected or designed to closely resemble the patient's anatomy prior to developing the arthritic state.

Certain embodiments of tibial trays can have the following features, although other embodiments are possible: modular insert system (polymer); cast cobalt chrome; standard blanks (cobalt portion and/or modular inert) can be made in advance, then shaped patient-specific to order; thickness based on size (saves bone, optimizes strength); allowance for 1-piece or 2-piece insert systems; and/or different medial and lateral fins.

In certain embodiments, the tibial tray is designed or cut from a blank so that the tray periphery matches the edge of the cut tibial bone, for example, the patient-matched peripheral geometry achieves >70%, >80%, >90%, or >95% cortical coverage. In certain embodiments, the tray periphery is designed to have the same shape, but be slightly smaller, than the cortical area.

The patient-specific tibial implants of certain embodiments allow for design flexibility. For example, inserts can be designed to compliment an associated condyle of femoral device, and can vary in dimensions to optimize design, for example, one or more of height, shape, curvature (preferably flat to concave), and location of curvature to accommodate natural or engineered wear pattern.

### 5.3.1 Bone cuts for a tibial implant component

In the knee, a tibial cut can be selected so that it is, for example, 90 degrees perpendicular to the tibial mechanical axis or to the tibial anatomical axis. The cut can be referenced, for example, by finding the intersect with the lowest medial or lateral point on the plateau.

The slope for tibial cuts typically is between 0 and 7 or 0 and 8 degrees in the sagittal plane. Rarely, a surgeon may elect to cut the tibia at a steeper slope. The slope can be selected or designed into a patient-specific cutting jig using a preoperative imaging test. The slope can be similar to the patient's preoperative slope on at least one of a medial or one of a lateral side. The medial and lateral tibia can be cut with different slopes. The slope also can be different from the patient's preoperative slope on at least one of a medial or one of a lateral side.

The tibial cut height can differ medially and laterally, as shown in **FIGS. 12A** and **12B****.** In some patients, the uncut lateral tibia can be at a different height, for example, higher or lower, than the uncut medial tibia. In this instance, the medial and lateral tibial cuts can be placed at a constant distance from the uncut medial and the uncut lateral tibial plateau, resulting in different cut heights medially or laterally. Alternatively, they can be cut at different distances relative to the uncut medial and lateral tibial plateau, resulting in the same cut height on the remaining tibia. Alternatively, in this setting, the resultant cut height on the remaining tibia can be elected to be different medially and laterally.

In certain embodiments, a patient-specific proximal tibia cut (and the corresponding bone-facing surface of the tibial component) is designed by: (1) finding the tibial axis perpendicular plane ("TAPP"); (2) lowering the TAPP 2 mm below the lowest point of the medial tibial plateau; (3) sloping the lowered TAPP 5° posteriorly (no additional slope is required on the proximal surface of the insert); (4) fixing the component stem posterior slope at 5°; and (5) using the tibial anatomic axis derived from Cobb for tibial implant rotational alignment. As shown in **FIG. 12C****,** cut depths below 2mm, such as 3 mm or 4 mm, may be designed, for example, if the bone includes an abnormality and a lower cut addresses the abnormality.

In certain embodiments, a patient-specific proximal tibia cut (and the corresponding bone-facing surface of the tibial component) uses the preceding design except for determining the A-P slope of the cut. In certain embodiments, a patient-specific A-P slope is used if the patient's anatomic slope is between 0° to 7°, a slope of 7° is used if the patient's anatomic slope is between 7° and 10°, and a slope of 10° is used if the patient's anatomic slope is greater than 10°.

In certain embodiments, a patient-specific A-P slope is used if the patient's anatomic slope is between 0° to 7° and a slope of 7° is used if the patient's anatomic slope is anything over 7°

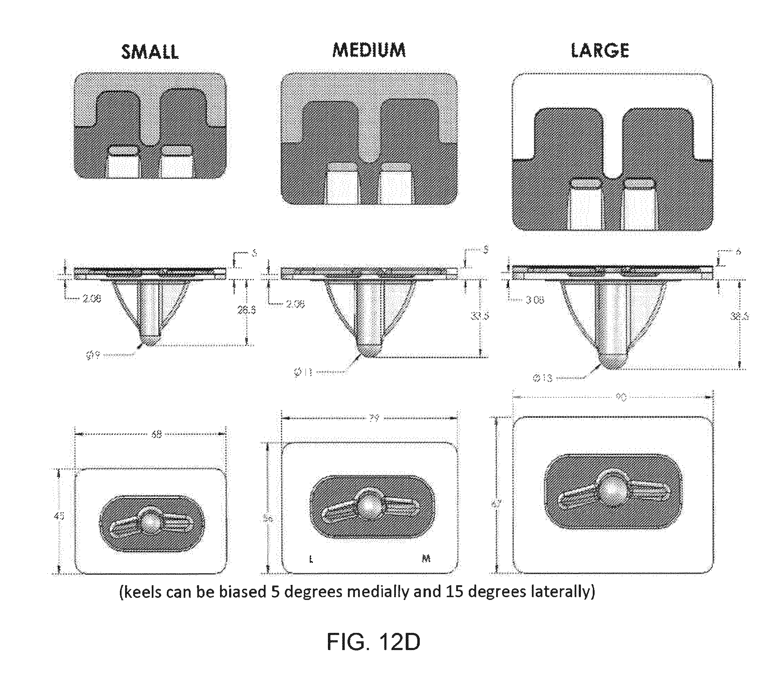

In certain embodiments, the axial profile of the tibial implant can be designed to match the axial profile of the patient's cut tibia. **FIG. 12D** includes additional considerations for tibial implant designs. Any of the tibial implant components described above can be derived from a blank that is cut to include one or more patient-specific aspects.

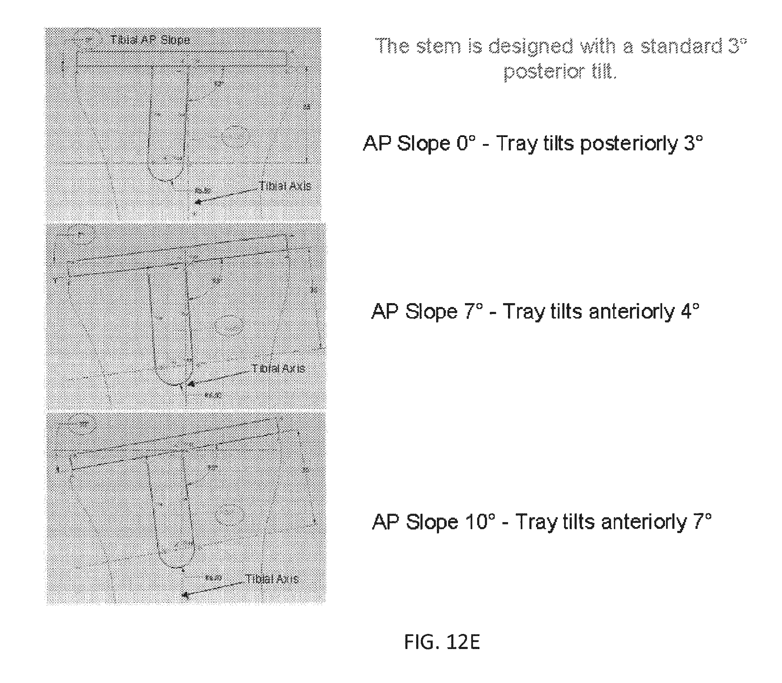

A patient specific peg alignment (e.g., either aligned to the patient's mechanical axis or aligned to another axis) can be combined with a patient-specific A-P cut plane. For example, in certain embodiments the peg can be aligned in relation to the patient's sagittal mechanical axis, for example, at a predetermined angle relative to the patient's mechanical axis. **FIG. 12E** shows exemplary A-P and peg angles.

### 5.3.2 Joint-facing surface

The joint-facing surface of a tibial implant component is largely a bearing surface. Like the femoral implant bearing surface described above, a bearing surface on a tibial implant (e.g., a groove or depression in the tibial surface that receives contact from a femoral component condyle) can be of standard design, for example, available in 6 or 7 different shapes, with a single radius or multiple radii in one dimension or more than one dimension. Alternatively, a bearing surface can be standardized in one or more dimensions and patient-adapted in one or more dimensions. Constant and variable radii can be selected in one dimension or multiple dimensions. Some of the radii can be patient adapted.

Each of the two contact areas of the polyethylene insert of the tibial implant component that engage the femoral medial and lateral condyle surfaces can be any shape, for example, convex, flat, or concave, and can have any radii. In certain embodiments, any one or more of the curvatures of the medial or lateral contact areas can include a patient-specific radii. Specifically, one or more of the coronal curvature of the medial contact area, the sagittal curvature of the medial contact area, the coronal curvature of the lateral contact area, and/or the sagittal curvature of the lateral contact area can include, at least in part, patient-specific radii. In preferred embodiments, the tibial implant component is designed to include one or both medial and lateral bearing surfaces having a sagittal curvature with, at least in part, patient-specific radii and a standard coronal curvature. Having, at least in part, patient adapted sagittal radii, in turn, can help achieve normal kinematics with full range of motion. The coronal curvature can be selected, for example, by choosing from a family of standard curvatures the one standard curvature that is most similar to the external radii of the patient's uncut femoral condyle.

In preferred embodiments, the tibial contact area has a standard convex coronal radius that is larger, for example slightly larger, for example, between 0 and 1 mm, between 0 and 2 mm, between 0 and 4 mm, between 1 and 2 mm, and/or between 2 and 4 mm larger, than the coronal radius on the corresponding femoral component. By using a standard coronal radius on a femoral condyle with a negatively-matching standard coronal radius or slightly larger on a tibial insert, the wear characteristics of the tibial implant, in this example the polyethylene insert, can be optimized. This approach also has some manufacturing benefits.

For example, a set of different-sized tools can be produced wherein each tool corresponds to one of the pre-selected standard coronal curvatures. The corresponding tool then can be used in the manufacture of a polyethylene insert of the tibial implant component, for example, to create a curvature in the polyethylene insert. **FIG. 13A** shows six exemplary tool tips **1310** and a polyethylene insert **1320** in cross-section in the coronal view. The size of the selected tool can be used to generate a polyethylene insert having the desired coronal curvature. **FIG. 13A** shows an exemplary polyethylene insert having two different coronal curvatures created by two different tool tips. The action of the selected tool on the polyethylene insert, for example, a sweeping arc motion by the tool at a fixed point above the insert, can be used to manufacture a standard or patient specific sagittal curvature. **FIG. 13B** shows a sagittal view of two exemplary tools **1330, 1340** sweeping from different distances into a polyethylene insert **1350** of a tibial implant component to create different sagittal curvatures in the polyethylene insert **1360.**

In certain embodiments, one or both of the tibial contact areas is a concave groove having an increasing or decreasing radius along its sagittal axis, for example, a groove with a decreasing radius from anterior to posterior.

In certain embodiments, the shape of the concave groove on the lateral and/or medial sides of the joint-facing surface of the tibial implant is positively-matched by a convex shape on the opposing side surface of the implant, as shown in **FIG. 14A****.** This can allow the thickness of the component to remain constant, even though the surfaces are not flat, and thereby can reduce wear of the material, for example, plastic material such as polyethylene. The constant material thickness also \helps to minimize implant thickness to achieve a certain mechanical strength. In addition, as shown in **FIG. 14A****,** any corresponding pieces of the component, such as a metal tray, also can include a matching groove to engage the curved surface of the plastic material. Two exemplary concavity dimensions are shown in **FIG. 14B****.** As shown in the figure, the concavities or scallops have depths of 1.0 and 0.7 mm, based on a coronal geometry of R42.4 mm. At a 1.0 mm depth, the footprint width is 18.3 mm. At a 0.70 mm depth, the footprint width is 15.3 mm

### EXAMPLES

Example 1 illustrates a process for designing a patient-specific total knee implant. Example 2 describes methods for designing and preparing bone cuts for a patient-specific femoral implant component. Example 3 illustrates a femoral component of a total knee replacement having non-traditional cuts on its inner, bone-facing surface. Example 4 illustrates a patient-specific implant design for an implant having a femoral component and a patella component. Example 5 illustrates a set of jigs for guiding patient-specific bone cuts in a femur-first technique. Example 6 illustrates a set of jigs for guiding patient-specific bone cuts in a tibia-first technique. Example 7 illustrates a tibial implant design and cut technique. Example 8 illustrates a tibial tray and insert design and related jig and cutting designs. Example 9 illustrates a finite element analysis ("FEA") test conducted on a femoral implant component. Example 10 illustrates a device component with an enhanced articular surface. Example 12 illustrates a design for a tibial implant component. Example 13 illustrates an implant and implant design having straight and curvilinear bone cuts. Example 14 illustrates an implant and implant design having resurfacing and one or no bone cuts.

### Example 1: Exemplary design process for certain patient-specific total knee implants

This example describes an exemplary process for designing a patient-specific total knee implant. The steps described in this design process can be performed in any order and can be performed more than once in a particular design process. For example, the steps can be reiterated and refined a second, third, or more times, before, during, or after performing other steps or sets of steps in the design process. While this process specifically describes steps for designing a patient-specific total knee implant, it can be adapted to design other embodiments, for example, patient-specific implants for shoulders and hips.

### 1.1 Methods

The exemplary design process shown in **FIG. Ex 1-1** includes four general steps and, optionally, can include a fifth general step. Each general step includes various specific steps. The general steps are identified as (1)-(5) in the figure. These steps can be performed virtually, for example, by using one or more computers that have or can receive patient-specific data and appropriate software or instructions for performing such steps.

In general step (1), limb alignment and deformity corrections are determined, to the extent that either is needed for a specific patient's situation. In general step (2), the patient's tibial and femoral dimensions are determined, based on data collected from the patient.

In general step (3), bone preservation is maximized by virtually designing each cut on the femur and tibia. This general step can include one or more of the steps of (i) simulating cuts on one or both articular sides, (ii) applying optimized cuts across both sides, (iii) allowing for non-co-planar and/or non-parallel femoral cuts, and (iv) maintaining and/or determining minimal material thickness. The minimal material thickness for the implant design can be an established threshold, for example, as previously determined by a finite element analysis ("FEA") of the implant's standard characteristics and features. Alternatively, the minimal material thickness can be determined for the specific implant, for example, as determined by an FEA of the implant's standard and patient-specific characteristics and features. This step dictates for a surgeon the design of bone resection to perform in the surgical theater and it also dictates the design of the bone-facing surface(s) of the implant or implants, which can substantially negatively-match the resectioned bone surfaces.

In general step (4), a corrected, normal and/or optimized articular geometry on the femur and tibia is recreated virtually. For the femur, this general step includes one or both of the steps of (i) selecting a standard sagittal profile or designing a patient-specific sagittal profile, and (ii) selecting a standard coronal profile or designing a patient-specific coronal profile. One or both of the sagittal and coronal profiles optionally can have different medial and lateral dimensions. For the tibia, this general step includes one or both of the steps of (iii) selecting a standard anterior-posterior slope or designing a patient-specific anterior-posterior slope, either of which optionally can vary from medial to lateral sides, and (iv) selecting a standard poly-articular surface or designing a patient-specific poly-articular surface. The patient specific poly-articular surface can be designed, for example, to simulate the normal or optimized three-dimensional geometry of the patient's tibial articular surface. This step contributes to the design on the outer joint-facing or articular surface(s) of the implant or implants.

In optional general step (5), the virtual implant model is assessed and can be adapted to achieve patient-specific normal or optimized kinematics. For example, the outer joint-facing or articular surface(s) of the implant or implants can be assessed and adapted to improve kinematics. This general step includes one or more of more of the steps of (i) virtually simulating biomotion of the model, (ii) adapting the implant design to achieve truly normal kinematics, and (iii) adapting the implant design to avoid potential impingement.

### 1.2 Results and discussion

The exemplary design process described above yields both a surgical resection design for altering articular surfaces of bones during surgery and a design for an implant that specifically fits the patient, for example, following the designed bone resectioning. Specifically, the designed implant, which can be manufactured or machined to specifications using known techniques, includes one or more surfaces that negatively-match the resectioned bone surface. The implant also can include other design features that are patient-specific, such as minimal implant thickness, articular geometry, and kinematic design. This design process can be applied to various joint implants and to various types of joint implants. For example, this design process can be applied to a total knee, cruciate retaining, posterior stabilized, and/or ACL/PCL retaining implants.

The exemplary patient-specific design process described above, including the resulting patient-specific implants and patient-specific bone resectioning methods, offers several advantages over traditional primary and revision implants and processes. For example, it allows for one or more pre-primary implants such that a subsequent knee replacement or improvement can take the form of a primary knee surgery. Specifically, because minimal bone is resectioned, a subsequent procedure can be performed with a traditional, primary, off-the-shelf primary knee implant. This offers a significant advantage for younger patients who may require in their lifetime more than a single revision to their knee implant. In fact, the exemplary patient-specific design process described above may allow for two or more pre-primary implants or procedures before such bone is lost that a traditional, primary implant is required.

The advantageous minimal bone resectioning, and therefore minimal bone loss, with this process arises from the fact that the bone-facing surfaces of the implants are designed patient-specifically. The patient-specific design of this surface allows for non-traditional, faceted bone cuts that are patient-specific and optimized using any number of cuts to conserve bone for the patient. With traditional implants, bone cuts are standardized and do not account for patient attributes such as, without limitation, size or weight of the patient, size of the joint, and size, shape and/or severity of defects in the joint.

Another advantage is that the patient-specific design process described above can restore a patient's native, normal kinematics, for example, by reducing or eliminating the patient's mid-flexion instability, by reducing or eliminating "tight" closure, by improving or extending flexion, by improving or restoring cosmetic appearance, and/or by creating or improving "normal" or "expected" sensations in the patient's knee. The patient-specific design for a tibial implant allows for an engineered surface that replicates the patient's normal anatomy yet also allows for low contact stress on the tibia.

For surgeons and medical professionals, the patient-specific design process described above provides a simplified surgical technique. The designed bone cuts and designed fit of the manufactured or machined implants eliminate the complications that arise in the surgical setting with traditional, misfitting implants.

As noted above, the design procedure can include manufacturing or machining the patient-specific implants in accordance with the specifications determined by the design steps described above. Manufacturing can include using a designed mold to form the patient-implant. Machining can include altering a blank form to conform to the specifications determined by the design steps described above. For example, using the steps described above, the femoral implant component can be manufactured from a designed mold and the tibial implant component, including each of a tibial tray and insert, can be customized from standard blanks.

### Example 2: Methods for designing and performing bone cuts for a patient-specific implant component

This example describes two exemplary methods for designing and performing bone cuts for two different patient specific femoral implant components.

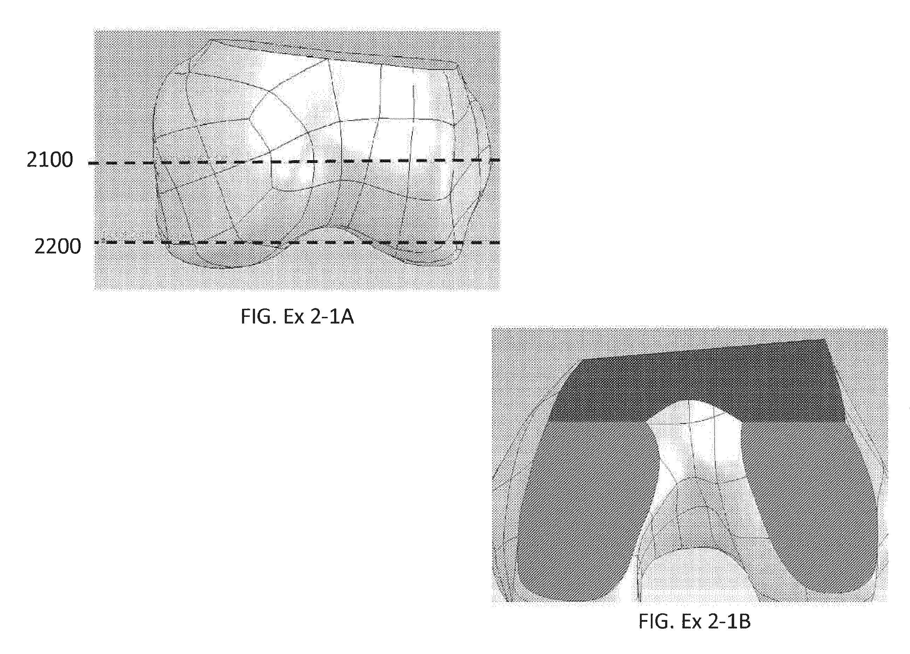

In both methods, a model of a patient's distal femur is created based on patient-specific data collected from one or more two- or three-dimensional images. As shown in **FIG. Ex 2-1A,** the epicondylar axis **2100** is determined for the patient's femur. Five bone cut planes and cut angles are created using the epicondylar axis **2100.** Specifically, four of the five cut planes - the distal cut, posterior cut, posterior chamfer cut, and anterior chamfer cut - are designed to be parallel with the epicondylar axis **2100. FIG. Ex 2-1A** shows the distal cut plane **2200** parallel to the epicondylar axis **2100.** The anterior cut plane is designed to be oblique to the plane of the epicondylar axis **2100,** which can minimize the amount of bone resectioned on the lateral side of the cut. FIG. **2-1B** shows an example of an anterior oblique cut plane.

For each of the five cut planes, an optimized maximum cut depth tangent to the bone surface at the angle of each cut plane also is designed. The optimized maximum cut depths are shown in **FIGS. Ex 2-2A - Ex 2-2E.** Specifically, in this example, the maximum cut depth is 6 mm for the distal cut plane **(FIG. Ex 2-2A),** the anterior chamfer cut plane **(FIG. Ex 2-2B),** the posterior chamfer cut **(FIG. Ex 2-2C),** and the posterior cut plane **(FIG. Ex 2-2D).** The maximum cut depth is 5 mm for the anterior cut plane **(FIG. Ex 2-2D).**

The optimized number of cut planes, angles of cut planes, and depths of cut planes can be determined independently for each of the medial and lateral condyles. For example, **FIGS. Ex 2**-**2A** - **Ex 2-2E** show optimized cut planes based on the medial condyle. However, **FIGS. Ex 2-3A** and **Ex 2-3B** show cut planes for the lateral condyle posterior chamfer and lateral condyle posterior cut planes that are independently optimized based on patient-specific data for the lateral condyle. This type of independent optimization between condyles can result in a number of cut planes, angles of cut planes, and/or depths of cut planes that differ between condyles.

The two bone cut design methods differ in how the five cut planes are oriented about the epicondylar axis. In the first design, shown in **FIG. 2-4A****,** a distal cut plane is designed perpendicular to the sagittal femoral axis **2400.** In the second design, referred to as a "flexed" or "flex-fit" design" and shown in **FIG. Ex 2-4B,** the distal cut plane is rotated 15 degrees in flexion from the perpendicular to the sagittal femoral axis. Additional cut planes are shifted accordingly for each design method, as shown in **FIGS. Ex 2-5A and Ex 2-5B.**

**FIGS. Ex 2-6A** and **Ex 2-6B** show the completed cut femur models for each cut design. For each design, the maximum resection depth for each cut plane was 6 mm. The "flex-fit" design can provide more posterior coverage in high flexion. However, it also may require more anterior bone resectioning to achieve sufficient coverage and may require particular attention during actual bone cutting to avoid any incomplete bone removal at the trochlear notch 2600. In certain embodiments of a bone cut design method, the anterior and cut planes diverge from the component peg axis by five degrees each, as shown in **FIG. Ex 2-7A.** With a traditional femoral implant component, the posterior and anterior cut planes diverge 2 degrees and 7 degrees, respectively, from the peg axis. Moreover, in certain embodiments, the peg can be designed to have various dimensions. For example, the design in **FIG. Ex 2-7B** includes a peg diameter of 7 mm tapering to about 6.5 mm, a length of 14 mm with a rounded tip, and a base with a 1 mm fillet **2700.**

The resulting femoral implant component design for the first design method is shown in **FIGS. Ex 2-8A** and **Ex 2-8B.** In addition to optimized cut planes described above, this design also includes a peripheral margin 0.5 mm from the edge of cut bone. The design also includes engineered coronal curvatures on the condyles. The resulting femoral implant component designs for the first and second design methods are shown side-by-side in **FIGS. Ex 2-9A** and **Ex 2-9B.** The sagittal view of the figures shows the difference in anterior and posterior coverage for the two component designs.

As mentioned above, the optimization of bone cuts can result in a cut design that has any number of cut planes, angles of cut planes, and depths of cut planes. The desired optimization parameters can include, for example, one or more of: (a) deformity correction and limb alignment (b) maximum preservation of bone, cartilage, or ligaments, and (c) restoration of joint kinematics. Additional parameters that may be included in the design process can include one or more of (d) implant shape, external and internal, (e) implant size, (f) implant thickness, (g) joint line location, and (h) location and preservation of particular features of the patient's biological structure, such as trochlea and trochlear shape.

### Example 3: Design of a femoral component of a total knee replacement with a bone-facing surface that optimizes bone preservation

This example describes an exemplary design of femoral components for a total knee replacement implant. In particular, the exemplary design and implant includes a femoral component having seven cuts on the inner, bone-facing surface.

### 3.1 Methods

A femoral implant component (PCL-retaining) is designed with seven bone cuts for a femur-first technique. The design is depicted in **FIG. Ex 3-1** as a virtual model. The design includes seven cuts on the inner, bone-facing surface. The seven cuts include a distal femoral cut that is perpendicular to the sagittal femoral axis, and an anterior cut that is not oblique. The corresponding bone cut angles are shown in FIG. **Ex 3-2A** and in **FIG. Ex 3-2B.** Specifically, anterior cuts are at 25 degrees, 57 degrees, and 85 degrees from the distal femoral cut, as shown in **FIG. Ex 3-2A.** Posterior cuts are at 25 degrees, 57 degrees, and 87 degrees of the distal femoral cut, as shown in **FIG. Ex 3-2B.** The femoral implant component also includes on the bone-facing surface cement cutouts that are 0.5 mm deep and offset from the outer edge by 2 mm, and a peg protruding from the each of the lateral and medial distal bone cut section on the inner surface of the component. The pegs are 7 mm in diameter, 17 mm long and are tapered by 0.5 degrees as they extend from the component. **FIG. Ex 3-3** shows the cement pocket and peg features.

### 3.2 Results and Discussion

In a traditional femoral implant component, the bone-facing surface consists of five standard cuts. However, the femoral component in this example includes seven cuts on the bone-facing surface. The additional cuts allow for a greater implant thickness at the intersection of the cuts and, therefore, less bone removal than is required by a traditional femoral implant component. The outer, articulating surface of the component can have patient-specific aspects and/or standard aspects.

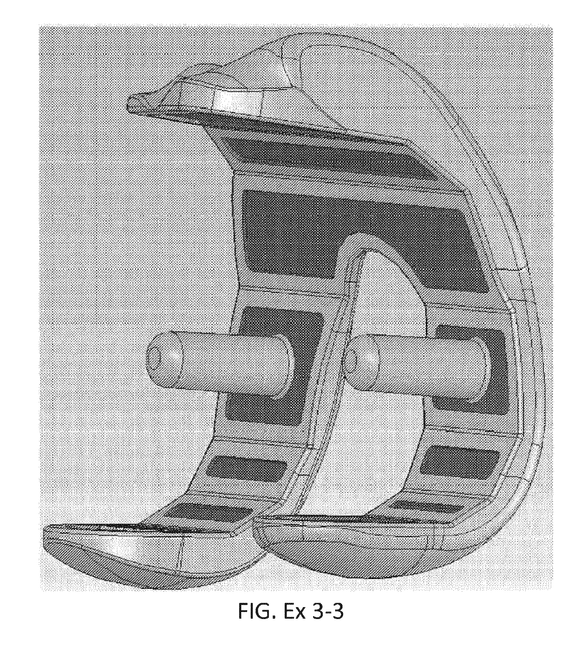

**FIG. Ex 3-4A** and **FIG. 3-4B** show models of bone cuts with corresponding bone volumes for a model having five bone cuts to the femoral articular surface **(FIG. Ex 3-4A)** and for a model having seven bone cuts to the femoral articular surface **(FIG. Ex 3-4B).** As shown, the model having five bone cuts corresponds to a volume of 103,034 mm³, while the model having seven bone cuts corresponds to a volume of 104,220 mm³ of bone.

By way of comparison, **FIG. Ex 3-5A** and **FIG. 3-5B** show virtual models of bone cuts with corresponding bone volumes for a model having five, not flexed bone cuts to the femoral articular surface **(FIG. Ex 3-5A)** and for a model having five, flexed bone cuts to the femoral articular surface **(FIG. Ex 3-5B).** As shown, the model having five, not flexed bone cuts corresponds to a volume of 109,472 mm³, while the model having five, flexed bone cuts corresponds to a volume of 105,760 mm³.

**FIG. Ex 3-6A - Ex 3-6D** show outlines of a traditional femoral component (in hatched lines) overlaid with, in **FIG. Ex 3-6A**, the model having seven bone cuts to the femoral articular surface; in **FIG. Ex 3-6B**, the model having five, bone cuts to the femoral articular surface; in **FIG. Ex 3-6C,** the model having five, not flexed bone cuts to the femoral articular surface; and in **FIG. Ex 3-6D,** the model having five, flexed bone cuts to the femoral articular surface. As shown in each of these figures, the designed bone cuts save substantial bone as compared to those required by a traditional implant component.

In summary, the exemplary component designs described in this example can save bone as compared to a traditional implant component and thereby allow the implant to be pre-primary. Alignment of cuts may also be patient specific, for example, symmetric or asymmetric, parallel or non-parallel, aligned perpendicular to the sagittal plane or not perpendicular, varied from medial to lateral condyle, etc. Design of cuts may also be "flexed" (i.e., rotated or offset relative to the biomechanical or anatomical axes). Design of attachment pegs may also be flexed relative to the biomechanical or anatomical axes.

### Example 4: A patient-specific engineered trochlea design

This example describes a patient-specific trochlea design that is optimized for proper kinematics of the patella-femoral ("PF") joint.

### 4.1 Method

**FIG. Ex 4-1A - Ex 4-1E** show an exemplary design of a knee implant, including a femoral component and a patella component, with a material cutaway region highlighted in red in certain figures. The placement of the patella and material removal was as follows: As shown in **FIG. Ex 4-1A,** the flat bone-bearing surface of the patella **4100,** was made parallel to the epicondylar axis **4110** in the coronal view. As shown in **FIG. Ex 4-1B,** the center plane of the patella implant was made collinear with the epicondylar axis **4120.** This allows for general positioning at the peak area of the trochlea. As shown in **FIG. Ex 4-1C,** in this position the medial-lateral center of the trochlea is identified **4130,** and the patella implant component is brought down so the lowest points are coincident **4140.** As shown in **FIG. Ex 4-1D,** the patella profile is swept along the sagittal curve of the trochlear region **4150.**

### 4.2 Results and discussion

This exemplary implant design uses a patient-specific sagittal curvature and an engineered coronal curvature to allow the patella component to track properly in the trochlear groove. This exemplary implant design for the femoral component and a patella component can allow various advantages including a reduction of lateral overstuffing of the P-F joint and a post-operative patella tracking that is normal or close to the patient's pre-operative and/or pre-disease state. In certain embodiments, the lateral peak can be retained, which may minimize dislocation events. In certain embodiments, the patella implant bone-bearing surface can be or appear to be approximately parallel to the osteochondral junction of the native patella.

### Example 5: Bone cuts using a femur-first jig set

This example describes methods and devices for performing a series of bone cuts to receive a patient-specific implant. Specifically, a set of jigs is designed in connection with the design of a patient-specific implant component. The designed jigs guide the surgeon in performing one or more patient- specific cuts to the bone so that those cut bone surface(s) negatively-match the patient-specific bone cuts of the implant component. The set of jigs described in this example are designed for a femur-first cut technique.

In a first step, shown in **FIGS. Ex 5-1A** and **Ex 5-1B,** a first femur jig is used to establish peg holes and pin placements for a subsequent jig used for a distal cut. In this example, the first jig is designed to circumvent 3 mm of cartilage thickness. In a second step, shown in **FIGS. Ex 5-2A** and **Ex 5-2B,** the distal cut is performed with a second femur jig. In this example, the second jig is patient-specific. However, in certain embodiments that apply a traditional distal cut, a standard jig can be used. In a third step, as shown in **FIGS. Ex 5-3A,** the anterior cut, the posterior cut, and the chamfer cuts are performed with a third femur jig. In this example, the jig includes slots that are 1.5 mm wide to allow for a saw blade thickness (i.e., no metal guides). For implant component designs having six or more inner, bone-facing surfaces, for example, having one or two additional chamfer cuts, the additional cuts can be performed using one or more additional jigs, for example, as shown in **FIG. Ex 5-3B.** In this example, the additional jig is designed to accommodate two steep additional chamfer cuts.

Next, the tibia is cut using one or more jigs designed to make patient-specific cuts to the tibia. An exemplary tibial jig is depicted in **FIGS. Ex 5-4** and **Ex 5-5.** A tibial alignment pin **5400** is used to help properly orient the jig. The portion **5410** of the jig inserted between the femur and tibia can have a variable thickness. In certain embodiments, the tibial jig can be designed to accommodate for composite thickness from the distal cut femur **5420.** Alternatively or additionally, a balancing chip **5600** can be used to address differences in the distance between the tibia and femur surfaces. For example, in certain embodiments a tibia jig may be designed to rest on 2 mm of cartilage, while a balancing chip is designed to rest on the distal cut femur.

A balancing chip is shown in **FIG. Ex 5-6.** If a varus deformity of the knee is observed, virtual realignment can be addressed by including added thickness to the balancing chip in the area that would produce a leg in neutral alignment **5610.** For a grossly mal-aligned contra-lateral leg, correction can be per a surgeon's order. The balancing chip can include a feature **5620** to attach it to the tibia jig, and thereby allow for accurate distal placement of the tibial cut while at the same time accommodating for composite thickness. An exemplary balancing chip attached to a tibia jig is shown in **FIGS. Ex 5**-**7A** and **Ex 5-7B.** To facilitate attachment, the balancing chip handle **5700** matches the tibial slope designed into the tibial cut and tibial implant. Preferably, the balancing chip is designed to enter into the joint easily.

### Example 6: Bone cuts using a tibial-first jig set

This example describes methods and devices for performing a series of bone cuts to receive a patient-specific implant. Specifically, a set of jigs is designed in connection with the design of a patient-specific implant component. The designed jigs guide the surgeon in performing one or more patient- specific cuts to the bone so that those cut bone surface(s) negatively-match the patient-specific bone cuts of the implant component. The set of jigs described in this example are designed for cuts to a femoral implant component in a tibia-first cut technique.

In a first step, shown in **FIG. Ex 6-1,** a first jig is used to establish placement and alignment of femoral implant peg holes. In the example, the placement is flexed 5 degrees with respect to the sagittal femoral axis. In a second step, shown in **FIG. Ex 6-2,** a second jig is used to establish placement pins for the distal cut jig. The second jig can have different thicknesses **6200** to accommodate composite thickness from the cut tibial surface. In a third step, as shown in **FIG. Ex 6-3,** a distal cut jig is positioned based on the placement established by the previous jig. The distal cut jig can be patient specific or standard. Lastly, as shown in **FIG. Ex 6-4,** remaining cuts are performed with a chamfer cut jig. In the example, the anterior cut is not oblique.

### Example 7: Tibial implant design and bone cuts

This example illustrates tibial implant components and related designs, as described on **FIGS. Ex 7-1A - Ex 7-3C.** This example also describes methods and devices for performing a series of tibial bone cuts to receive a tibial implant component, as described and shown on **FIGS. Ex 7-4A - Ex 7-5.**

### Example 8: Tibial tray and insert designs

This example illustrates designs and implant components for tibial trays and inserts, as described on **FIGS. Ex 8-1A - Ex 8-3E.**

### Example 9: Finite element analysis

This example illustrates an exemplary finite element analysis ("FEA") that can be conducted on a device component of some embodiments as one parameter in the optimization of patient-specific aspects of the implant. Specifically, this example describes FEA conducted on three variations of a femoral implant component.

### 9.1 Methods

This analysis investigates the effect of interference fit and loading scenarios on three different large knee femoral implant component geometries: (a) a component with six bone cuts and a perpendicular distal bone cut ("Perp 6-Cuts"); (b) a component with five bone cuts and a perpendicular distal bone cut ("Perp 5-Cuts"); and (c) a component with six bone cuts and flexed bone cuts ("Flexed 6-Cuts"), as shown in **FIG. Ex 9-1A.** The three knee femoral implant component geometries tested represent implants for the largest expected anatomy, as shown compared to a traditional large implant in **FIGS. Ex 9-1B1, Ex 9-1B2,** and **Ex 9-1B3.** Target results included identification of maximum principle stresses and displacements. For a general reference on conducting FEA on knee implant components, see "Initial fixation of a femoral knee component: an in vitro and finite element study," Int. J. Experimental and Computational Biomechanics, Vol 1, No. 1, 2009.

**FIG. Ex 9-1C** shows set-up information for the testing. For initial runs of the three variations, the models of the femur were setup with 0.35 degrees interference fit angles on the Anterior Shield (A, FIG. Ex 9-1), upper most medial Condyle (B, FIG. Ex 9-2), and upper most lateral condyle (C, FIG. Ex 9-2) surfaces. This angle was set by running iterative analyses until a Max Principal Stress of roughly 240 MPa (the fatigue endurance limit of CoCr) was achieved. Secondary analysis runs were performed with no interference fit on the three Femoral Implant geometries.

All contact surfaces between the implant and femur (D, FIG. Ex 9-3) were set up as frictional (0.5 coefficient of friction based on the general reference described above), and the surfaces between the implant and condyle support plates (E, FIG. 9-3) were frictionless.

For all cases the top face of the femur (F, FIG. Ex 9-4) was fully fixed. The bottom faces of the condyle support plates **(G** and **H,** **FIG. 9-5****)** were either fixed in all directions or, when the load was applied, allowed to move along the femoral axis only (Z direction shown on visible coordinate system).

Loads of 1601 N (360 lbs.) to the lateral condyle support plate and 2402 N (540 lbs.) to the medial condyle support plate were applied in the direction of the Femoral Axis (Z axis shown, **FIG. Ex 9-6).** A balance was struck to align model performance with the different contact areas and results. The overall mesh is shown in **FIG. Ex 9-7.** The mesh of the implant component was refined for best results in the high stress areas **(FIG. Ex 9-8).**

### 9.2 Results and Discussion

The three different large knee femoral implant component geometries that were assessed were sized to correspond to large anatomical knees. The results for Interference No Load, Interference Plus Load, and No Interference Plus Load are shown in **Table Ex 9-1** below. The corresponding high stress locations (identical for all three models) are shown in **FIGS. Ex 9-9, Ex 9-10,** and **Ex 9-11.** These data can be used in the design of patient-specific implant components, for example, to identify a minimum component thickness for areas of high stress. As shown in the table, there was a 24% reduction in stress with 6 cuts compared to five cuts 221 MPa versus 292 MPa, interference plus no load).

**Table Ex 9-1**

| | Perp-s-Cuts | | | | Perp-s-Cuts | | | | Flexed-s-Cuts | | |
|---|---|---|---|---|---|---|---|---|---|---|---|
| | Interference | Inter. + Load | No Inter. + Load | | Interference | Inter. +Load | No Inter. + Load | | Interference | Inter. +Load | No Inter. +Load |
| **Max Principal Stress [MPa]** | 245.0 | 221.0 | 38.1 | | 211.3 | 323.0 | 1205 | | 261.4 | 214.0 | 83.5 |
| | Detectors (mm) | | | | Detectors (mm) | | | | Detectors (mm) | | |
| **Lateral Condyle** | 0.11 | 0.11 | 0 | | 0.10 | 0.10 | 0 | | 0.11 | 0.11 | 0 |
| **Medial Condyle** | 0.08 | 0.08 | 0 | | 0.07 | 0.08 | 0 | | 0.07 | 0.07 | 0 |
| **Anterior Shield** | 0.18 | 0.18 | 0.05 | | 0.17 | 0.18 | 0.06 | | 0.20 | 0.21 | 0.05 |

### Example 10: A femoral component device with enhanced articular surface

This example illustrates an exemplary device component with an enhanced articular surface. **FIG. Ex 10-1A** is a front schematic view of engaging portions of a knee implant **10. FIG. Ex 10-1B** is a cross-sectional schematic view in the coronal plane of a femoral component **20** of the implant **10** of **FIG. Ex 10-1A.** With reference to **FIG. Ex 10-1A** and **FIG. Ex 10-1B,** this exemplary embodiment of a patient-specific implant **10** includes a femoral component **20** and a tibial tray component **30,** and it is designed based on patient-specific data. An inner, bone-facing surface **40** of the femoral component **20** conforms to the corresponding surface of the femoral condyle. Alternatively, it can conform to one or more optimized bone cuts on the femoral condyle. However, the outer, articular surface **50** of the component **20** is enhanced to incorporate a smooth surface having a nearly constant radius in the coronal plane. The corresponding articular surface **70** of the tibial tray **30** has a surface contour in the coronal plane that is matched to the outer articular surface **50.** In this embodiment, the articular surface **70** has a radius that is five times the radius of outer articular surface **50.** In certain embodiments, the articular surface **50** of the component **20** incorporates a sagittal curvature that positively-matches the patient's existing or healthy sagittal radius.

**FIG. Ex 10-2A - Ex 10-2D** show cross-sectional schematic views in the coronal plane of respective alternate embodiments of a femoral component.

The design of implant **10** has several advantages. First, the design of articular surface **50** allows the thickness of femoral component to be better controlled as desired. For example, referring to **FIG. Ex 10-2A,** if a curve of an articular surface **80** of a femoral component **90** is too large, the thickness of the femoral component may be too thick along a centerline **100** of the implant, thereby requiring an excessive amount of bone to be removed when the implant is placed on the femoral condyle. On the other hand, referring to **FIG. Ex 10-2B,** if the same curve **80** is applied to a device having an appropriate centerline thickness **110,** the margins or sidewalls **120** and **130** of the device may be too thin to provide proper structural support. Similarly, referring to **FIG. Ex 10-2C,** if the curve of the outer articular surface **120** of a femoral component **130** is too flat, the device does not exhibit the tapering from a centerline **140** to the margins or sidewalls **150** and **160** of the device and may not function well.

Referring again to **FIG. Ex 10-1A** and **FIG. Ex 10-1B,** a second advantage of the implant **10** over certain other embodiments of patient-specific devices is that the smooth articular surface **50** is thought to provide better kinematics than a true representation of the surface of the patient's femoral condyle may provide.

For example, referring also to **FIG. Ex 10-2D,** one method of making patient specific implants is to use a simple offset, in which a femoral component **170** is designed using a standard offset from each point of the modeled surface of the patient's femoral condyle. Using such a design, the thickness of the device remains essentially constant, and an outer surface **180** essentially positively-matches or conforms to the underlying inner femoral-facing surface **190,** as well as the modeled surface of the femoral condyle on which it is based. While this provides a truly patient-matched outer surface, it is not necessarily optimal for the kinematics of the resulting implant, due to, for example, rough areas that may produce higher, more localized loading of the implant. By using a smooth surface with an essentially pre-determined shape, the loading of the implant can be better managed and distributed, thereby reducing the wear on the tibial tray component **30.**

The third advantage, which is also related to the loading and overall kinematics of the implant, is in the negative-matching of the tibial articular surface **70** to the femoral articular surface **50** in the coronal plane. By providing a radius that is predetermined, for example, five times the radius of the femoral articular surface **50** at its centerline in the present embodiment, the loading of the articular surfaces can be further distributed. Thus, the overall function and movement of the implant is improved, as is the wear on the tibial tray, which is polyethylene in this embodiment. While the present embodiment uses a ratio of five times the radius of the outer surface at its centerline (note that the radius of the outer surface may be slightly different at other locations of the outer surface **50** away from the centerline), other embodiments are possible, including an outer tibial surface that, in the coronal plane, is based on other ratios of curvature, other curvatures, other functions or combinations of curves and/or functions at various points. Additionally, while the embodiments shown in **FIG. Ex 10-2A - FIG. Ex 10-2D** are not considered to be optimal designs generally, they are embodiments that can be generated using automated systems and may have preferable characteristics in some instances.

### Example 11: Tibial implant component design

This example illustrates a design for a tibial implant component, as described more fully on **FIGS. Ex 11-1 - Ex 11-7C.**

The features of the tibial resection designed in conjunction with the implant component of this example include: perpendicular to tibial axis; single cut based on medial posterior slope; and bone cut 2-3 mm below lowest area of medial tibial plateau.

The features of the tibial implant component design of this example include: tray maximizes coverage and extend to cortical margins whenever possible; medial compartment coverage is maximized; no overhang on medial compartment; avoid internal rotation of tibial components to avoid patellar dislocation; and avoid excessive external rotation to avoid overhang laterally and impingement on the popliteus tendon.

### Example 12: Implant and implant design with curvilinear bone cuts

This example illustrates an exemplary implant, implant design, and method for designing an implant having both straight and curvilinear bone cuts. Specifically, a femoral implant is designed to include 3 mm curvilinear cut depths and corresponding implant thicknesses along the distal portion of each condyle. The cut depth and implant thickness along each condyle is designed independently of the other condyle. In addition, jigs for performing the curvilinear cuts to the articular bone surface are described.

Using a computer model generated from patient-specific data, posterior and anterior cut lines are created in the model, as shown in **FIGS. Ex 12-1A** and **12-1B.** To design the curvilinear cut line on the medial condyle, a medial split line is identified on the condyle, as shown in **FIG. Ex 12-2A,** and then a 3 mm deep cut line is generated to follow the split line, as shown in **FIG. Ex 12-2B.** The resulting virtual curvilinear cut is shown in **FIG Ex 12-2C.** The same steps are performed independently for the lateral condyle, as shown in **FIGS. Ex 12-3A - 12-3C.**

The resulting cut model, as shown in **FIG. Ex 12-4A** can be used to engineer the bone-facing surface of the corresponding patient-specific implant, as shown in **FIGS. Ex 12-4B** and **12-4C.** Specifically, the inner, bone facing surface of the implant is designed and engineered to substantially negatively-match the cut surface on the model. Optionally, and as shown in the figures, the outer, joint-facing surface of the implant also can be designed and engineered to include one or more patient-specific aspects.

The resulting cut model also can be used to design one or more cutting jigs that are fitted to the bone to guide the bone cutting procedure. For example, **FIG. Ex 12-5A** shows a model of a bone after being resected using a jig that allows sagittal cutting of the bone along the J-curve specific to a patient's particular anatomy. **FIGS. Ex 12-5B** and **12-5C** show an alternative set of jigs that can be used with a router-type saw. Specifically, a router-type bit can fit into the central channel of the jig shown in **FIG. Ex 12-5B** to cut along the channel to a specific depth, for example, 3 mm. Then, as shown in **FIG. Ex 12-5C,** a second jig having two channels that circumvent the channel of the first jig can be applied. The router-type bit can fit into these two channels to cut medial and lateral to the first channel to the same depth, for example, 3 mm.

**FIG. Ex 12-6A** shows a model of the prepared bone following jig-guided bone cuts. **FIG. Ex 12-6B** shows the model of **FIG. Ex 12-6A** with a two-piece patient-specific implant designed with an inner bone-facing surface that substantially negatively-matches the cut bone surface.

### Example 13: Implant and implant design with resurfacing

Example 13 illustrates an implant and implant design having a resurfaced portion and a bone cut portion and an implant and implant design having a resurfaced surface with no bone cuts.

Using a patient-specific computer model generated from patient-specific data, a femoral implant is designed to include a single, posterior cut on its inner, bone-facing surface, as shown in **FIGS. Ex 13A-4E** above and in **FIGS. Ex 13-1A** and **14-1B.** The remaining portions of the inner, bone-facing surface of the implant are designed to substantially negatively-match the articular surface of the bone that it engages. Optionally, the outer, joint-facing surface of the implant also can be designed and engineered to include one or more patient-specific aspects. As shown in the figures, the patient-specific implant with a single bone cut is prepared as two pieces or components, which allows for fitting the curving anterior portion of the implant **1390** around the anterior portion **1392** of the femur.

The femoral implant design shown in **FIGS. Ex 13-2A** and **Ex 13-2B** and the corresponding implant shown in **FIG. Ex 13-2C** also use a two-piece or two-component design, in part to allow for fitting the curving anterior portion of the implant **1390** around the anterior portion **1392** of the femur. Specifically, using a patient-specific computer model generated from patient-specific data, a femoral implant was designed to include no bone cuts on its inner, bone-facing surface. Instead, the inner, bone-facing surface of the implant was designed to substantially negatively-match the articular surface of the bone that it engages. Optionally, the outer, joint-facing surface of the implant also can be designed and engineered to include one or more patient-specific aspects.

### INCORPORATION BY REFERENCE

The entire disclosure of each of the publications, patent documents, and other references referred to herein is incorporated herein by reference in its entirety for all purposes to the same extent as if each individual source were individually denoted as being incorporated by reference.

### EQUIVALENTS

The invention may be embodied in other specific forms without departing from the spirit or essential characteristics thereof. The foregoing embodiments are therefore to be considered in all respects illustrative rather than limiting. The scope of the invention is thus indicated by the appended items rather than by the foregoing description, and all changes that come within the meaning and range of equivalency of the items are intended to be embraced therein.

### DISCLOSED ITEMS

What is itemed is:
1. A patient-specific femoral implant for implantation on a portion of a femur of a patient's knee, comprising:
   a condylar portion having a bone- facing surface for abutting at least a portion of a condyle of the patient's knee and an articular surface generally opposite the bone-facing surface; the articular surface having a patient-specific curvature generally disposed in a first plane, the patient-specific curvature substantially replicating a corresponding curvature of at least a portion of the patient's condyle and being located approximately in the same location as the corresponding curvature of the patient's condyle when the bone-facing surface abuts the condyle; the articular surface having a constant curvature in a second plane that is generally transverse to the first plane.
2. The implant of item 1, wherein the corresponding curvature of the patient's condyle is a J-curve.
3. The implant of item 1, wherein the patient-specific curvature extends substantially the entire length of the articular surface.
4. The implant of item 10, wherein the patient-specific curvature extends a portion of the length of the articular surface.
5. The implant of item 1, wherein the patient-specific curvature extends along the majority of the length of a weight bearing area of the implant.
6. The implant of item 1, wherein the patient-specific curvature extends along a portion of the length of a weight bearing area of the implant.
7. The implant of item 1, wherein the first plane is a sagittal plane.
8. The implant of item 1, wherein the first plane is a coronal plane.
9. The implant of item 1, wherein the patient-specific curvature substantially matches a corresponding curvature of the patient's condyle.
10. The implant of item 1, wherein the patient-specific curvature approximates a corresponding curvature of the patient's condyle.
11. The implant of item 1 , wherein the patient-specific curvature is a smoothed curvature that eliminates or reduces at least some local maxima a curvature of the patient's condyle.
12. The implant of item 1, wherein the patient-specific curvature is a portion of a curve in substantially a coronal plane.
13. The implant of item 1, wherein the constant curvature extends substantially the entire length of the articular surface.
14. The implant of item 1, wherein the constant curvature extends a portion of the length of the articular surface.
15. The implant of item 1, wherein the constant curvature extends along a portion of the length of a weight bearing area of the implant.
16. The implant of item 1, wherein the constant curvature extends a portion of the length of the articular surface generally in the direction of the first plane.
17. The implant of item 1, wherein the constant curvature extends along the entire length of a weight bearing area of the implant generally in the direction of the first plane.
18. The implant of item 1, wherein the constant curvature extends along a portion of the length of a weight bearing area of the implant generally in the direction of the first plane.
19. The implant of item 1, wherein the second plane is a sagittal plane.
20. The implant of item 1, wherein the second plane is a coronal plane.
21. The implant of item 1, wherein the constant curvature approximates an average curvature of a corresponding curvature of the patient's condyle.
22. The implant of item 1, wherein the constant curvature is a standardized curvature.
23. A patient-specific femoral implant for implantation on a portion of a femur of a patient's knee, comprising:
   a first condylar portion having a first bone- facing surface for abutting at least a portion of a first condyle of the patient's knee and a first articular surface generally opposite the first bone-facing surface;
   a second condylar portion having a second bone- facing surface for abutting at least a portion of a second condyle of the patient's knee and a second articular surface generally opposite the second bone- facing surface;
   the first articular surface having a first patient-specific curvature generally disposed in a first plane, the first patient-specific curvature substantially replicating a curvature of the patient's first condyle and being located approximately in the same location as the curvature of the patient's first condyle when the first bone-facing surface abuts the condyle; and
   the second articular surface having a second patient-specific curvature generally disposed in a second plane, the second patient-specific curvature substantially replicating a curvature of the patient's second condyle and being located approximately in the same location as the curvature of the patient's second condyle when the second bone-facing surface abuts the second condyle.
24. The implant of item 23, wherein the first articular surface has a constant curvature in a plane that is generally transverse to the first plane.
25. The implant of item 24, wherein the second articular surface has a constant curvature in a plane that is generally transverse to the first plane
26. The implant of item 23, wherein the first and second patient specific curvatures replicate at least a portion of corresponding j-curves of the knee.
27. The implant of item 23, wherein the first and second planes are generally sagittal planes.
28. The implant of item 27, wherein the first and second planes are substantially coronal planes.
29. The implant of item 23, wherein the first plane is generally a sagittal plane.
30. The implant of item 29, wherein the second plane is generally a coronal plane.
31. The implant of item 23, wherein the first plane is substantially a coronal plane.
32. The implant of item 23, wherein the second plane is generally a sagittal plane.
33. The implant of item 23, wherein the first patient specific curvature extends for substantially the entire length of a weight-bearing portion of the first articular surface.
34. The implant of item 33, wherein the second patient-specific curvature extends for substantially the entire length of a weight-bearing portion of the second articular surface.
35. A femoral implant having at least one of a medial condyle and a lateral condyle with a condylar bone-facing surface and a condylar articular surface, the condylar articular surface comprising:
   (a) a condylar articular surface curvature in a first plane derived from patient-specific data and designed to match a corresponding surface curvature of the patient's anatomy, or a predetermined percentage thereof, along at least a weight-bearing portion of the condylar articular surface; and
   (b) a condylar articular surface curvature in a second plane that is engineered or selected to be constant along at least a weight-bearing portion of the condylar articular surface.
36. The femoral implant of item 35, wherein the first plane is a sagittal plane and the second plane is a coronal plane.
37. The femoral implant of item 35, wherein the first plane is a coronal plane and the second plane is a sagittal plane.
38. The femoral implant of item 35, further comprising one or more additional implant features or measurements derived from patient-specific data and adapted for the particular patient.
39. The femoral implant of item 35, wherein the one or more additional features or measurements includes one or planar facets on the bone-facing surface of the femoral implant being derived from patient-specific data and adapted to maximize bone preservation for the particular patient.
40. The femoral implant of item 35, wherein the one or more additional features or measurements includes a condylar width of the implant being derived from patient-specific data and adapted to substantially match a corresponding width of the patient's femoral condyle, or a predetermined percentage thereof.
41. The femoral implant of item 35 , wherein the one or more additional features or measurements includes a distance between medial and lateral condyles on the implant being derived from patient-specific data and adapted to substantially match a corresponding distance between the patient's medial and lateral femoral condyles, or a predetermined percentage thereof.
42. The femoral implant of item35, wherein the one or more additional features or measurements includes a thickness of the implant from the bone-facing surface to the articular surface being derived from patient-specific data and adapted to substantially match a corresponding thickness from a corresponding planned resection cut surface to a corresponding articular surface on the patient's femur, or a predetermined percentage thereof.
43. The femoral implant of item 35, wherein the one or more additional features or measurements includes a cross-sectional perimeter shape of the implant being derived from patient-specific data and adapted to substantially match a corresponding cross-sectional perimeter shape of the patient's femur, or a predetermined percentage thereof.
44. The femoral implant of item35, wherein the one or more additional features or measurements includes a volume of a portion of the implant derived from patient-specific data and adapted to substantially match a corresponding volume of a portion of the patient's femur, or a predetermined percentage thereof.

## Claims

1. An implant system for repairing a knee joint of a patient, comprising:
a femoral implant, wherein an articulating surface of a condylar portion of the femoral implant comprises a sagittal curvature derived from patient-specific image data of the knee joint of the patient; and
a tibial implant having a joint-facing surface, wherein at least a portion of the joint-facing surface of the tibial implant has a shape based on the joint-facing surface of the condylar portion of the femoral implant.

2. The implant system of claim 1, wherein a coronal curvature of the joint-facing surface of the condylar portion of the femoral implant is substantially constant at least a portion of the articulating surface.

3. The implant system of claim 1, wherein a coronal curvature of at least a portion of the articulating of the tibial implant is substantially constant.

4. The implant system of claim 1, wherein the femoral implant is configured to replace both medial and lateral femoral condyles of the knee joint of the patient, and comprises a corresponding medial condylar portion and a corresponding lateral condylar portion.

5. The implant system of claim 4, wherein:
(a) the tibial implant comprises a single insert or dual inserts having the joint-facing surface, wherein at least a portion of the joint-facing surface of the single insert or dual inserts has a shape based on the articulating surface of both medial and lateral condylar portions of the femoral implant; or
(b) the tibial implant comprises an all polyethylene implant having the joint-facing surface, wherein at least a portion of the joint-facing surface of the all polyethylene implant has a shape based on the articulating surface of both medial and lateral condylar portions of the femoral implant.

6. The implant system of claim 1, wherein the femoral implant is configured to replace a medial or a lateral femoral condyle of the knee joint of the patient, and comprises a corresponding medial condylar portion or a corresponding lateral condylar portion.

7. The implant system of claim 1, wherein the sagittal curvature of the articulating surface of the condylar portion of the femoral implant is adapted based on patient-specific measurements to improve a feature of the femoral implant relative to the knee joint of the patient, optionally wherein the feature of the femoral implant is a feature selected from the group consisting of an A-P dimension, an M-L dimension, an S-I dimension and any combination thereof.

8. The implant system of claim 1, further including one or more surgical tools created using the patient-specific image data.

9. A knee implant system for repairing a knee joint of a patient comprising:
a femoral implant component having one or more patient-adapted features and one or more standard features, wherein the one or more patient-adapted features comprise an M-L dimension of a joint-facing condylar portion of the femoral implant that matches a corresponding M-L dimension of a femoral condyle of the knee joint of the patient; and
a tibial implant component.

10. The knee implant system of claim 9, wherein the femoral implant comprises a medial condylar portion and a lateral condylar portion, wherein the one or more standard features include an intercondylar M-L dimension of the femoral implant that is designed to be of a standard length.

11. The knee implant system of claim 10, wherein the tibial implant component comprises a medial insert and a lateral insert, wherein the distance between the medial and lateral inserts is standard and configured to match the intercondylar M-L dimension of the femoral implant.

12. The knee implant system of claim 9, wherein the one or more patient-adapted features are derived from patient-specific data obtained from the patient, wherein the patient-specific data comprises one or more parameters of the patient's biological features selected from the group consisting of: one or more anatomical structures, anatomical axes, alignment, biomechanical axes, kinematics, soft tissue balance, soft tissue impingement of the knee joint of the patient, one or more bone properties of the patient, demographic data of the patient, and any combination thereof.

13. The knee implant system of claim 9, wherein the femoral implant comprises a bone-facing surface having two or more facet portions configured to abut a resected surface of a corresponding femoral condyle of the knee joint of the patient.

14. The knee implant system of claim 13, wherein the resected surface of the corresponding femoral condyle is prepared with a predetermined resectioning strategy selected or designed for the patient.

15. The knee implant system of claim 9, further comprising one or more surgical tools that facilitate the placement of the femoral implant in the patient's knee joint, optionally further comprising one or more surgical tools created for the patient, wherein the one or more surgical tools are created based on the predetermined resectioning strategy.
